# EUROPEAN PATENT APPLICATION

(11) **EP 4 825 857 A1**
(43) Date of publication of application: **21.10.2026**
(21) Application number: 24902877.0
(22) Date of filing: 12.12.2024
(51) Int. Cl.: C07D 471/04, C07D 487/04, A61K 31/519, A61P 1/00, A61P 11/00, A61P 11/06

(54) **TRICYCLIC FUSED HETEROCYCLIC PDE3/4 DUAL INHIBITOR, AND PREPARATION AND USE THEREFOR**

(30) Priority: 13.12.2023 CN 202311717507
(71) Applicant: Shijiazhuang Yiling Pharmaceutical Co., Ltd., Shijiazhuang, Hebei 050035 (CN)
(72) Inventor: JIA, Zhenhua, Shijiazhuang, Hebei 050035 (CN); WANG, Hongtao, Shijiazhuang, Hebei 050035 (CN); HOU, Yunlong, Shijiazhuang, Hebei 050035 (CN); QI, Hui, Shijiazhuang, Hebei 050035 (CN); MI, Yao, Shijiazhuang, Hebei 050035 (CN); HAO, Yuanyuan, Shijiazhuang, Hebei 050035 (CN); TONG, Yanan, Shijiazhuang, Hebei 050035 (CN); LI, Faguang, Shijiazhuang, Hebei 050035 (CN); ZHANG, Pengfei, Shijiazhuang, Hebei 050035 (CN); HAO, Meng´an, Shijiazhuang, Hebei 050035 (CN); WANG, Xiaohang, Shijiazhuang, Hebei 050035 (CN); XU, Duo, Shijiazhuang, Hebei 050035 (CN)
(74) Representative: Barker Brettell LLP
(86) International application number: PCT/CN2024/138683
(87) International publication number: WO 2025/124460

(57) **Abstract**

A tricyclic fused heterocyclic PDE3/4 dual inhibitor, a method for preparing the inhibitor, and use thereof are disclosed. Specifically, a compound of Formula I, or a pharmaceutically acceptable form thereof is provided, wherein each substituent is as defined in the specification. The compound or pharmaceutically acceptable form thereof may be used as a phosphodiesterase inhibitor, and exhibits high activity.

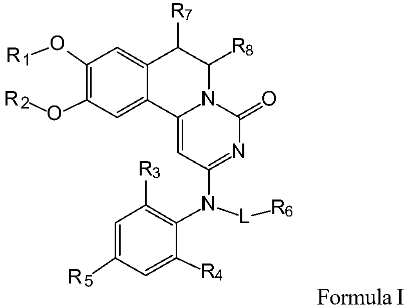

## Description

### TECHNICAL FIELD

The present disclosure relates to a tricyclic fused heterocyclic phosphodiesterase inhibitor, particularly a PDE3/4 dual inhibitor, and a method for preparation thereof and use thereof.

### BACKGROUND

Phosphodiesterases (PDEs) are an enzyme superfamily comprising at least 11 families and 22 isoforms, and are involved in intracellular and extracellular signal transduction and functional regulation. PDEs catalyze the ring-opening hydrolysis of the intracellular second messengers cyclic adenosine monophosphate (cAMP) and cyclic guanosine monophosphate (cGMP), yielding AMP and GMP, respectively.

The PDE3 family consists of two genes, PDE3A and PDE3B. In the respiratory system, PDE3 activity is mainly found in alveolar macrophages, endothelial cells, and platelets. PDE3 is involved in the regulation of various physiological processes *in vivo,* such as relaxation of vascular smooth muscle, inhibition of platelet aggregation, antithrombotic effects, cardiotonic effects, and inhibition of cell proliferation. However, excessive use of PDE3 inhibitors may lead to adverse effects such as hypotension and tachycardia, which greatly limit their clinical application.

PDE4 is an enzyme that specifically hydrolyzes cAMP. The PDE4 family consists of four isoforms, namely PDE4A, PDE4B, PDE4C, and PDE4D. Each isoform is encoded by a corresponding gene and differs in cellular distribution and function. PDE4 is mainly distributed in airway smooth muscle cells, inflammatory cells, and immune cells, and regulates the intracellular cAMP level. Most PDE4 inhibitors currently used in clinical practice are associated with certain adverse effects, such as gastrointestinal reactions such as nausea and vomiting, and even depression.

In view of the limitations associated with the use of PDE3 inhibitors or PDE4 inhibitors alone, as well as the adverse effects of PDE inhibitors, inhaled dual inhibitors of PDE3 and PDE4 appear to be a more attractive strategy for targeting key pathological features of chronic obstructive pulmonary disease (COPD) and asthma. Existing evidence has shown that inhaled PDE3/PDE4 dual inhibitors have a synergistic inhibitory effect, including synergistic anti-inflammatory and bronchodilatory effects.

CN100415743C discloses a pyrimido[6,1-a]isoquinolin-4-one derivative.

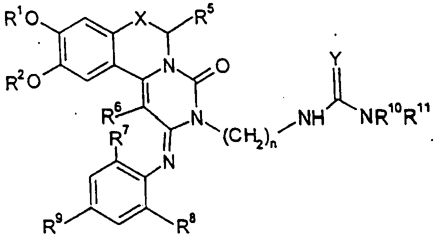

The compound represented by the general formula is useful as a PDE inhibitor for the treatment of respiratory diseases such as asthma. Compared with trequinsin, it has a longer duration of action and does not have the extremely bitter taste of trequinsin.

CN112368281A discloses a group of tricyclic compounds as PDE3/PDE4 dual inhibitors.

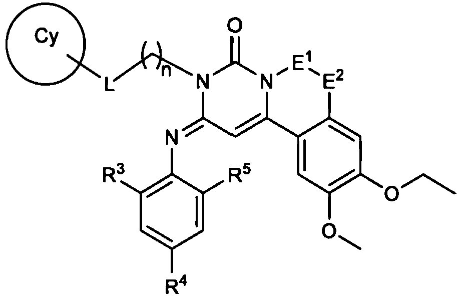

The compound represented by the general formula may be used for preparing a medicament for the treatment of PDE3/PDE4-related diseases, particularly COPD.

There remains a need in the art for novel PDE3/PDE4 inhibitors, particularly those having high activity and good druggability.

### SUMMARY OF INVENTION

An object of the present disclosure is to provide a novel compound as a PDE inhibitor.

Another object of the present disclosure is to provide a method for preparing the compound.

Another object of the present disclosure is to provide a use of the compound.

Another object of the present disclosure is to provide a pharmaceutical composition comprising the compound, and a use thereof.

Another object of the present disclosure is to provide an intermediate for preparing the compound.

Another object of the present disclosure is to provide a method for preparing the intermediate.

### <First Aspect>

The present disclosure provides a compound of Formula I, or a pharmaceutically acceptable form thereof, wherein the pharmaceutically acceptable form is selected from a pharmaceutically acceptable saltor co-crystal, a stereoisomer, a tautomer, a deuterate, a solvate, a chelate, a non-covalent complex, or a prodrug. 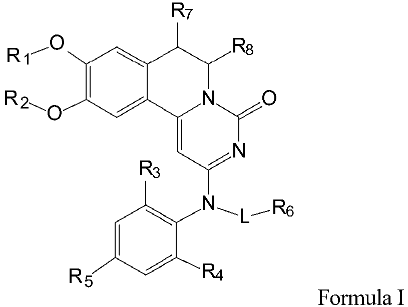 wherein:
R₁ and R₂ are each independently selected from H, a C₁₋₆ linear alkyl, a C₃₋₆ branched alkyl, or a C₃₋₆ cycloalkyl; each of the linear alkyl, branched alkyl, or cycloalkyl is optionally further substituted with 0 to 4 substituents selected from D, F, Cl, Br, I, OH, =O, NH₂, CN, COOH, a C₁₋₄ alkyl, or a C₁₋₄ alkoxyl;
R₃, R₄, and R₅ are each independently selected from H, halogen, CN, a C₁₋₆ alkoxy, a C₁₋₆ linear alkyl, a C₃₋₆ branched alkyl, or a C₃₋₆ cycloalkyl; each of the alkoxy, linear alkyl, branched alkyl, or cycloalkyl is optionally further substituted with 0 to 4 substituents selected from D, F, Cl, Br, I, OH, =O, NH₂, CN, COOH, a C₁₋₄ alkyl, or a C₁₋₄ alkoxy;
L is selected from 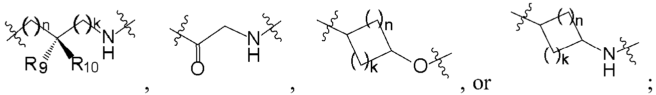 n is 0, 1, or 2; k is 0, 1, 2, or 3; the H in L is optionally further substituted with 0 to 4 substituents selected from F, Cl, Br, I, OH, =O, NH₂, CN, COOH, a C₁₋₄ alkyl, or a C₁₋₄ alkoxy; wherein R₉ and R₁₀ are each independently selected from H, a C₁₋₆ linear alkyl, a C₃₋₆ branched alkyl, a C₃₋₆ cycloalkyl, a C₃₋₆ heterocycloalkyl, a C₆₋₁₀ aryl, a C₃₋₁₀ heteroaryl, or COOCH₃, provided that R₉ and R₁₀ are not both H; the heteroaryl contains 1 to 3 heteroatoms selected from N, O, or S;
R₆ is selected from 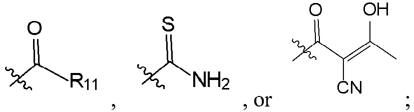 wherein R₁₁ is selected from amino, a C₁₋₆ alkoxy, a C₃₋₁₂ cycloalkyl, a C₆₋₁₀ aryl, or a C₅₋₁₀ heterocyclyl; wherein the heterocyclyl contains 1 to 3 heteroatoms selected from N, O, or S; each of the amino, alkoxy, cycloalkyl, aryl, and heterocyclyl is optionally substituted with 0 to 4 substituents selected from H, F, Cl, Br, I, OH, =O, NH₂, CN, COOH, CF₃, a C₁₋₄ alkyl, a C₁₋₄ alkoxy, or a C₃₋₆ cycloalkyl;
optionally, R₆ and L together form 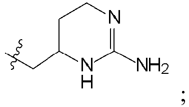
R₇ and R₈ are each independently selected from H, =O, halogen, NH₂, CN, a C₁₋₆ linear alkyl, a C₃₋₆ branched alkyl, a C₃₋₆ cycloalkyl, or

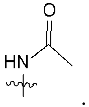

According to some specific embodiments of the present disclosure, in the compound of Formula I or a pharmaceutically acceptable form thereof, R₁ and R₂ are each independently selected from CH₃, CHF₂, CD₃ or a C₃₋₆ cycloalkyl.

According to some specific embodiments of the present disclosure, in the compound of Formula I or a pharmaceutically acceptable form thereof, R₁ and R₂ are each CD₃.

According to some specific embodiments of the present disclosure, in the compound of Formula I or a pharmaceutically acceptable form thereof, one of R₁ and R₂ is CH₃, and the other is CHF₂.

According to some specific embodiments of the present disclosure, in the compound of Formula I or a pharmaceutically acceptable form thereof, R₃, R₄, and R₅ are each independently selected from CH₃, i-Pr, OMe, CD₃, or halogen.

According to some specific embodiments of the present disclosure, in the compound of Formula I or a pharmaceutically acceptable form thereof, R₁ and R₂ are respectively the corresponding groups or values shown in any one of the compounds in Table 1.

According to some specific embodiments of the present disclosure, in the compound of Formula I or a pharmaceutically acceptable form thereof, R₅ is CH₃ which is optionally further substituted with 0 to 3 D atoms; and R₃ and R₄ are the same and are selected from CH₃, i-Pr, OMe, or halogen.

According to some specific embodiments of the present disclosure, in the compound of Formula I or a pharmaceutically acceptable form thereof, R₃, R₄, and R₅ are respectively the corresponding groups or values shown in any one of the compounds in Table 1.

According to some specific embodiments of the present disclosure, in the compound of Formula I or a pharmaceutically acceptable form thereof, L is 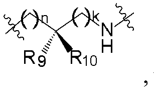 wherein n is 0 or 1, preferably 0. Preferably, k is 0, 1, or 2. Preferably, one of R₉ and R₁₀ is H, and the other is selected from H, a C₁₋₆ linear alkyl, a C₃₋₆ branched alkyl, a C₃₋₆ cycloalkyl, a C₃₋₆ heterocycloalkyl, a C₆₋₁₀ aryl, a C₅₋₁₀ heteroaryl, or COOCH₃; wherein the heteroaryl contains 1 or 2 N heteroatoms; and the H in L is optionally further substituted with 0 to 4 substituents selected from F, Cl, Br, I, OH, =O, NH₂, CN, COOH, a C₁₋₄ alkyl, or a C₁₋₄ alkoxy.

According to some specific embodiments of the present disclosure, in the compound of Formula I or a pharmaceutically acceptable form thereof, L is

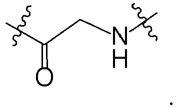

According to some specific embodiments of the present disclosure, in the compound of Formula I or a pharmaceutically acceptable form thereof, L is 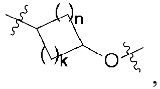 n is 0 or 1, and k is 1.

According to some specific embodiments of the present disclosure, in the compound of Formula I or a pharmaceutically acceptable form thereof, L is 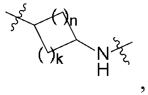 n is 0 or 1, and k is 1.

According to some specific embodiments of the present disclosure, in the compound of Formula I or a pharmaceutically acceptable form thereof, L is 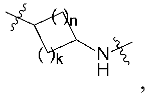 n is 2, and k is 2.

According to some specific embodiments of the present disclosure, in the compound of Formula I or a pharmaceutically acceptable form thereof, L is 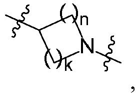 n is 1, and k is 1 or 2.

According to some specific embodiments of the present disclosure, in the compound of Formula I or a pharmaceutically acceptable form thereof, L is 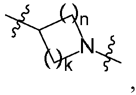 n is 2, and k is 2.

According to some specific embodiments of the present disclosure, in the compound of Formula I or a pharmaceutically acceptable form thereof, L is the corresponding group or value shown in any one of the compounds in Table 1.

According to some specific embodiments of the present disclosure, in the compound of Formula I or a pharmaceutically acceptable form thereof, R₆ is 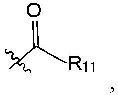 wherein R₁₁ is amino which is optionally further substituted with 0, 1, or 2 substituents selected from F, Cl, Br, I, OH, =O, NH₂, CN, COOH, CF₃, a C₁₋₄ alkyl, a C₁₋₄ alkoxy, or a C₃₋₆ cycloalkyl.

According to some specific embodiments of the present disclosure, in the compound of Formula I or a pharmaceutically acceptable form thereof, R₆ is 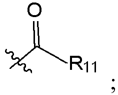 wherein R₁₁ is a C₆₋₁₀ aryl or a C₅₋₁₀ heterocyclyl; wherein the heterocyclyl contains 1, 2, or 3 N heteroatoms; each of the aryl and heterocyclyl is optionally further substituted with 0, 1, 2, or 3 substituents selected from H, F, Cl, Br, I, OH, =O, NH₂, CN, COOH, CF₃, a C₁₋₄ alkyl, a C₁₋₄ alkoxy, or a C₃₋₆ cycloalkyl.

According to some specific embodiments of the present disclosure, in the compound of Formula I or a pharmaceutically acceptable form thereof, R₆ is the corresponding group or value shown in any one of the compounds in Table 1.

According to some specific embodiments of the present disclosure, in the compound of Formula I or a pharmaceutically acceptable form thereof, R₇ is H or CH₃, and R₈ is H or F.

According to some specific embodiments of the present disclosure, in the compound of Formula I or a pharmaceutically acceptable form thereof, R₇ and R₈ are respectively the corresponding groups or values shown in any one of the compounds in Table 1.

According to some specific embodiments of the present disclosure, in the compound of Formula I or a pharmaceutically acceptable form thereof,
R₁ and R₂ are each independently selected from CH₃ or CD₃;
R₃, R₄, and R₅ are each CH₃;
L is 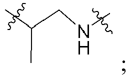
R₆ is 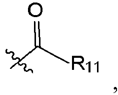 wherein R₁₁ is amino, methoxy, 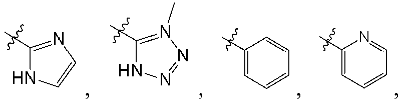 or 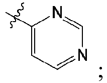 wherein the amino is optionally further substituted with 0 or 1 methyl or cyclopropyl; and the 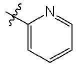 is optionally further substituted with 0 or 1 methyl or NH₂; and
R₇ and R₈ are each H.

According to some specific embodiments of the present disclosure, in the compound of Formula I or a pharmaceutically acceptable form thereof,
R₁ and R₂ are each CH₃;
R₃, R₄, and R₅ are each CH₃;
L is 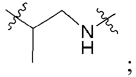
R₆ is 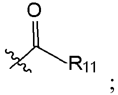 wherein R₁₁ is amino, 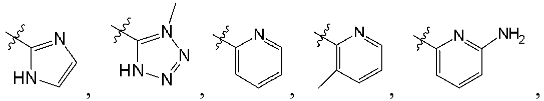 or 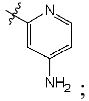
R₇ and R₈ are each H.

According to some specific embodiments of the present disclosure, in the compound of Formula I or a pharmaceutically acceptable form thereof, R₁, R₂, R₃, R₄, R₅, R₆, R₇, R₈, and L are respectively the corresponding groups or values shown in any one of the compounds in Table 1.

According to some specific embodiments of the present disclosure, the compound of Formula I or a pharmaceutically acceptable form thereof is one or more selected from the compounds set forth in Table 1.

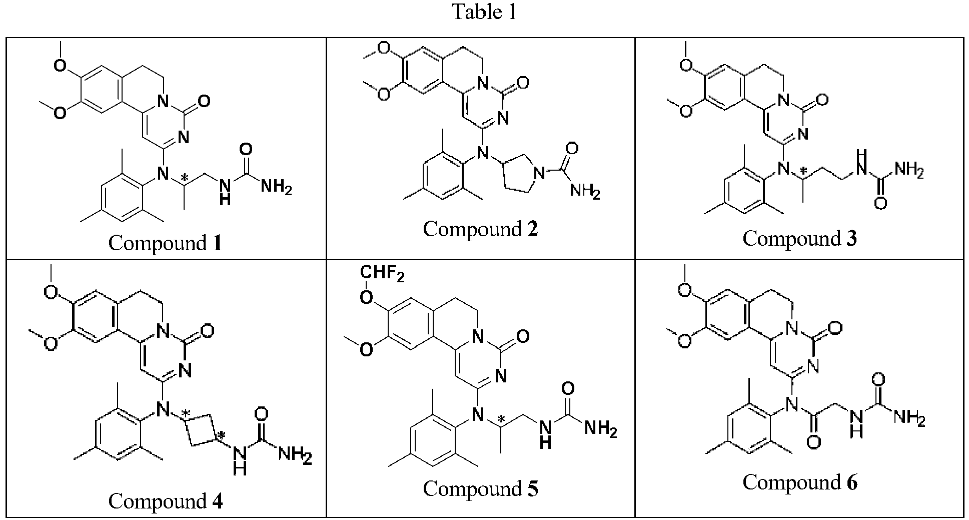

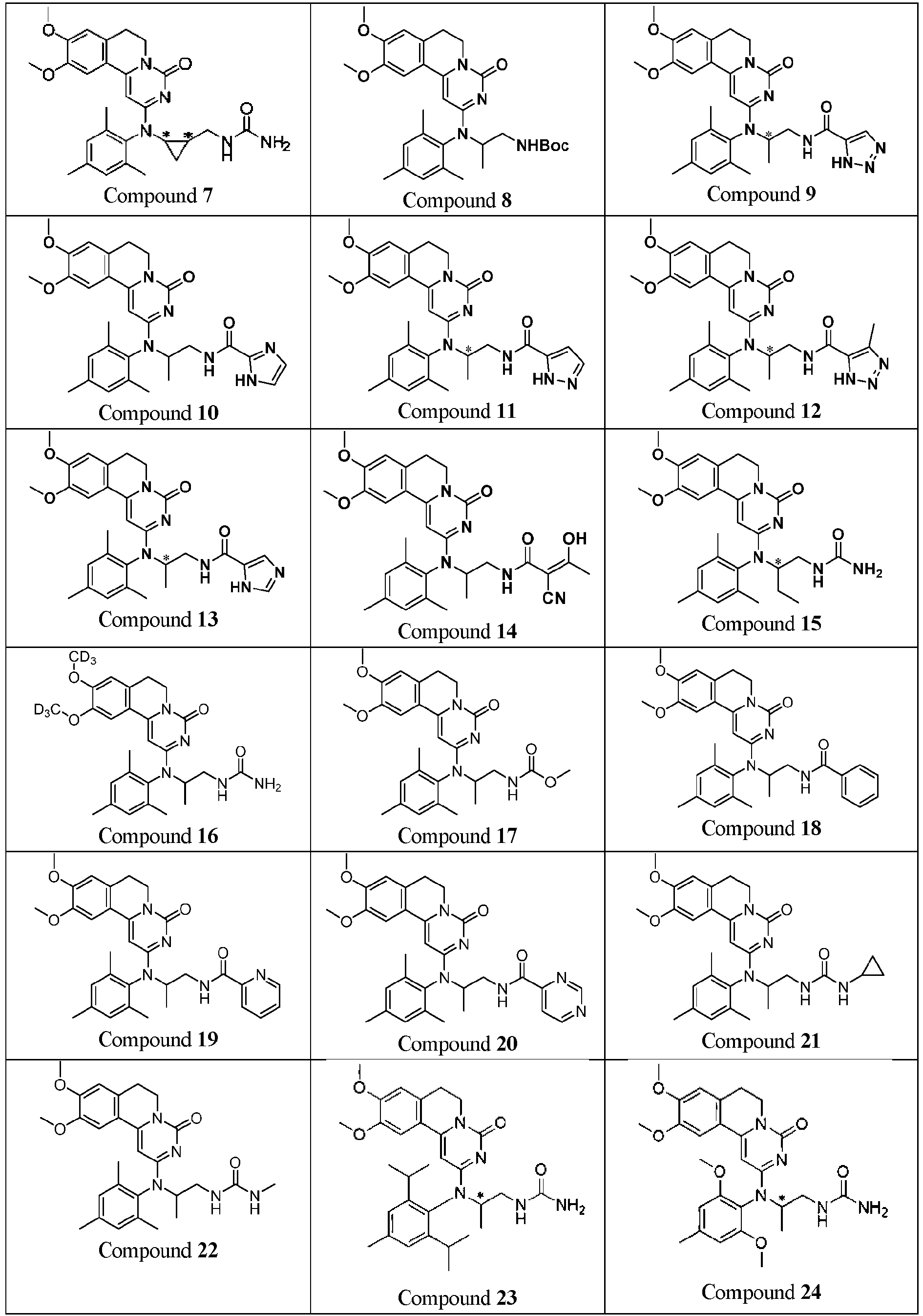

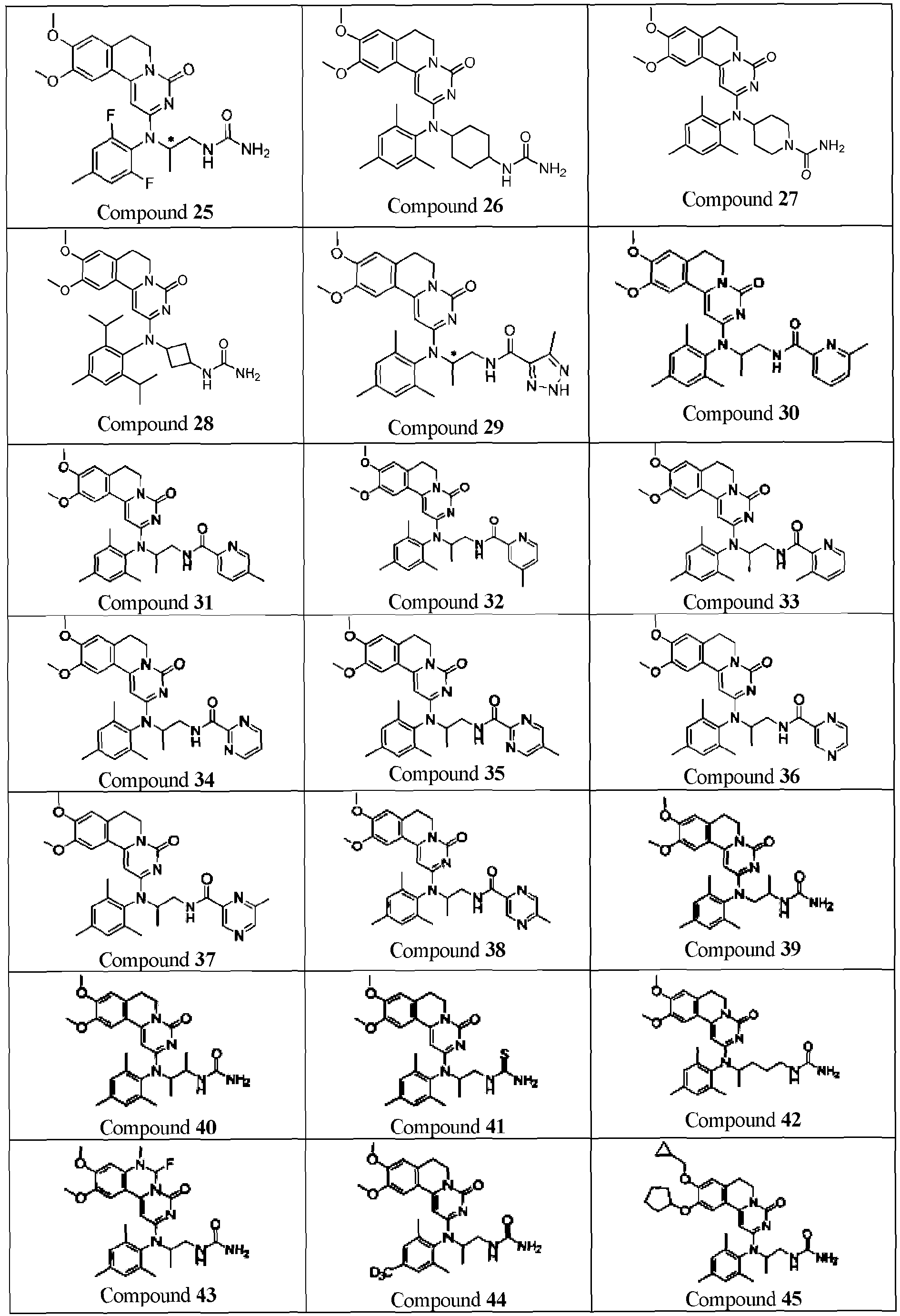

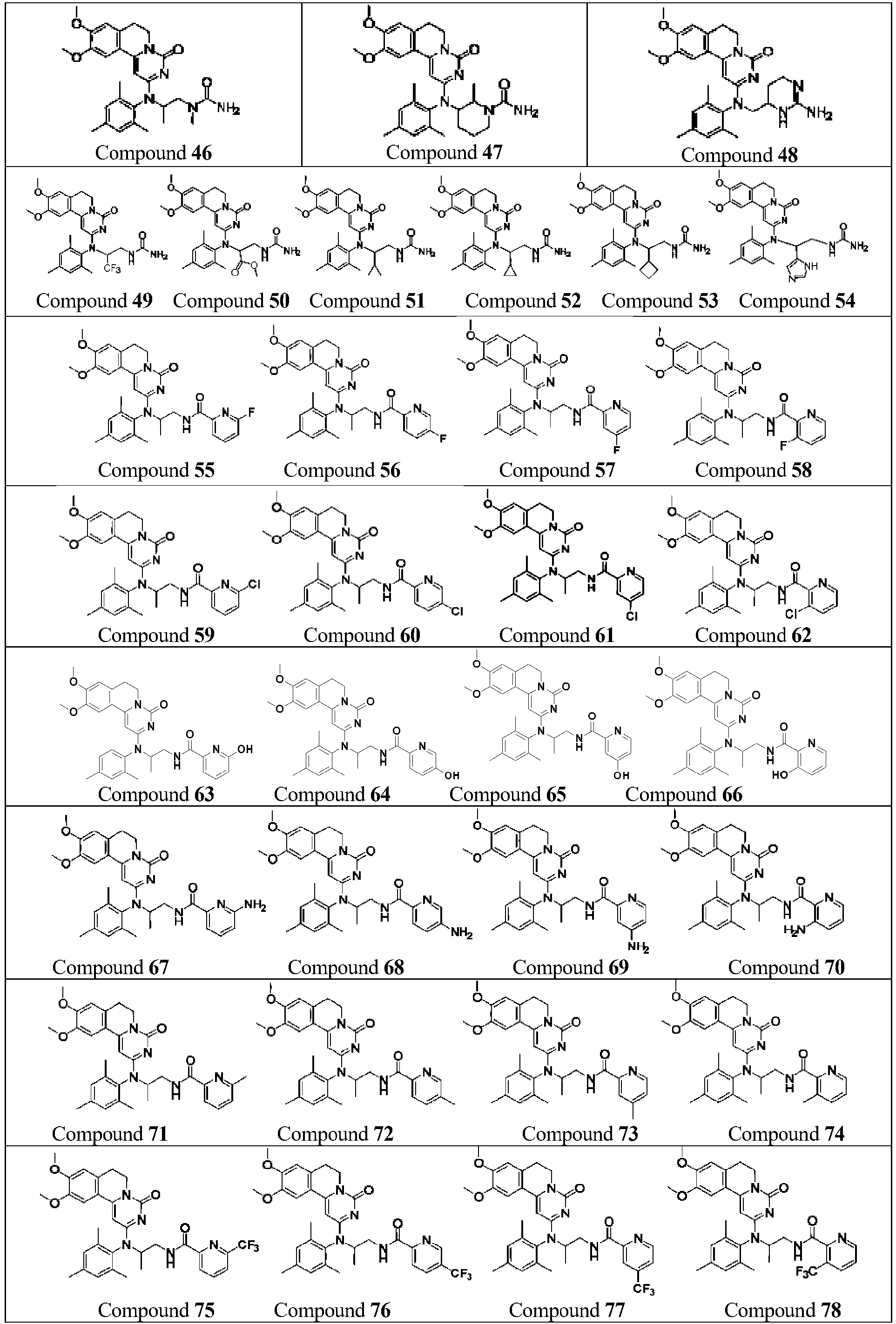

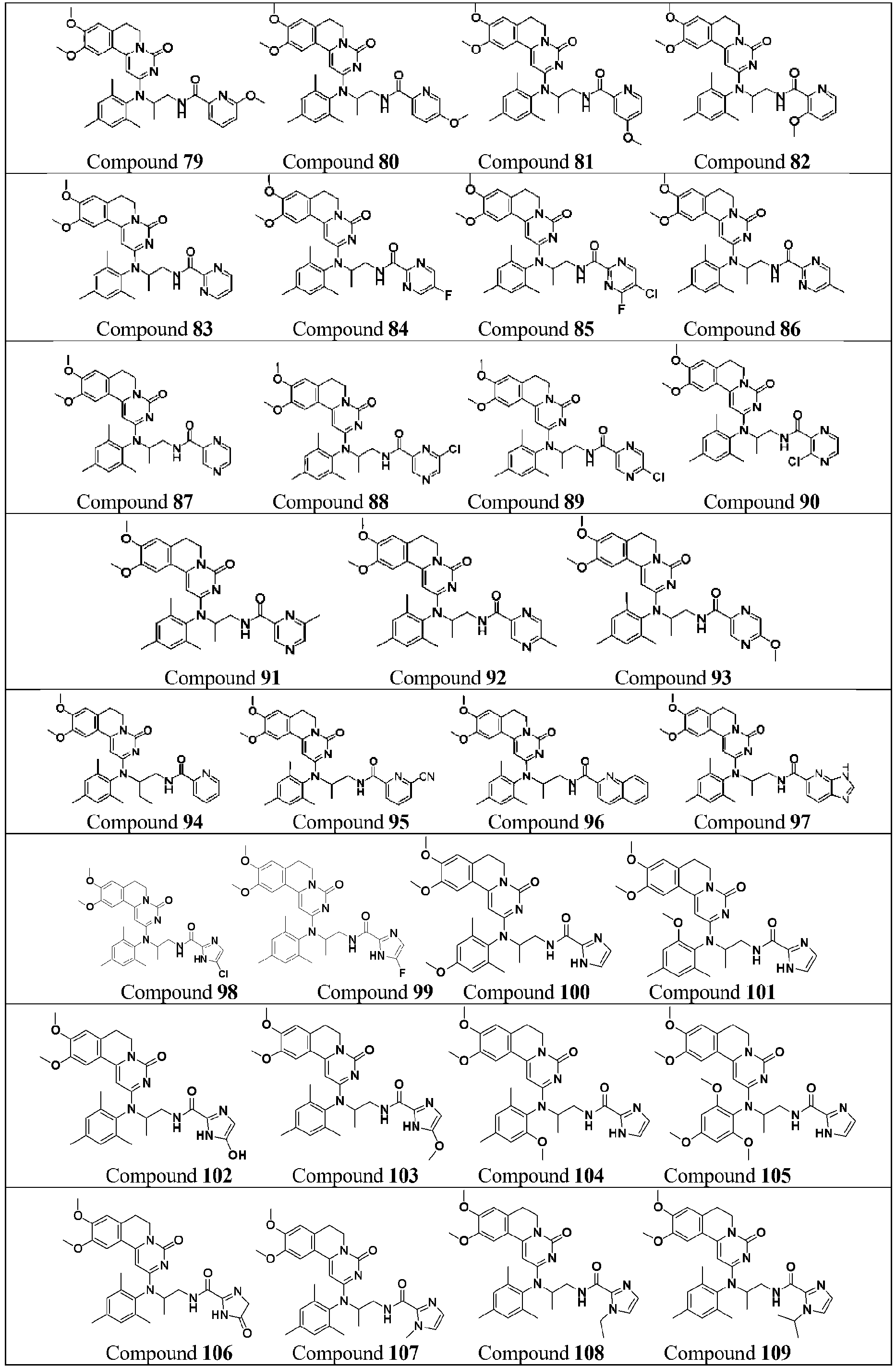

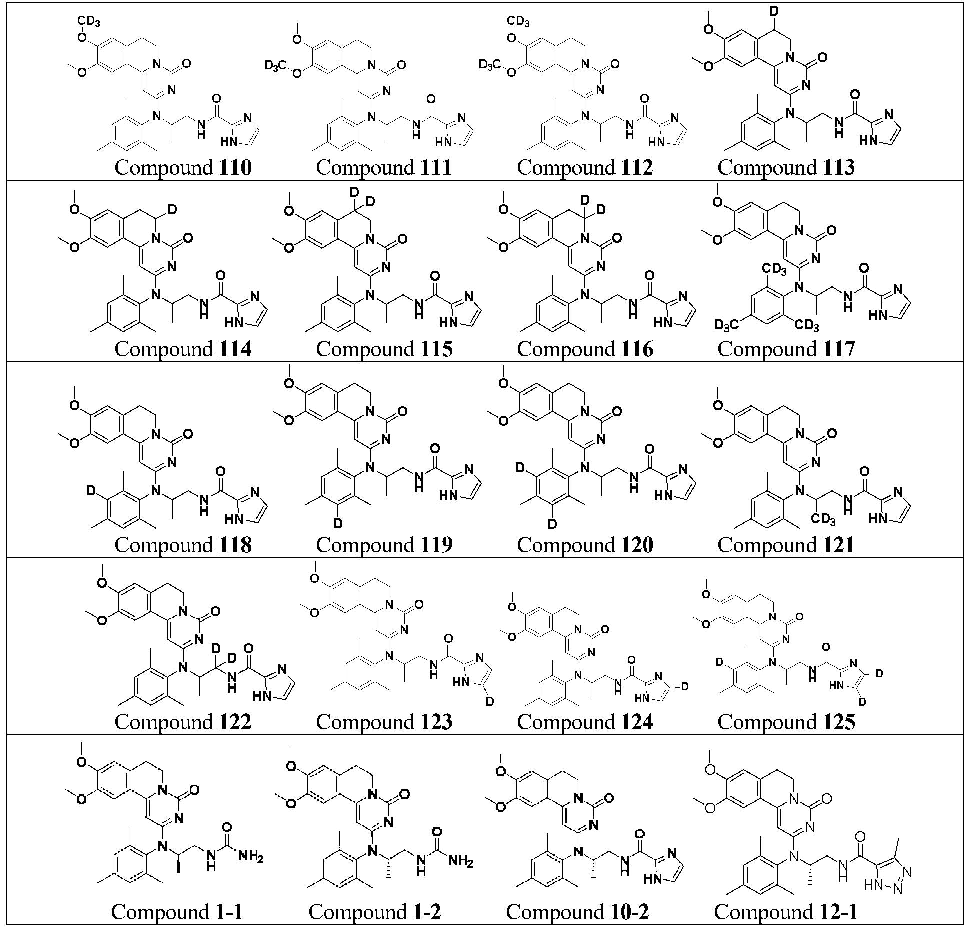

According to some specific embodiments of the present disclosure, in the compound of Formula I or a pharmaceutically acceptable form thereof, the pharmaceutically acceptable form is selected from a pharmaceutically acceptable salt or co-crystal, a stereoisomer, a tautomer, a deuterated form, a solvate, a chelate, a non-covalent complex, or a prodrug of any one of the compounds shown in Table 1.

### <Second Aspect>

The present disclosure further provides an intermediate compound, which has a structure represented by Formula II: 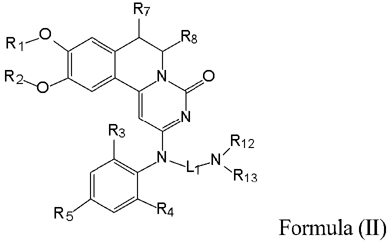
wherein R₁, R₂, R₃, R₄, R₅, R₇, and R₈ are as defined in the compound of Formula I or the pharmaceutically acceptable form thereof according to any embodiment of the <first aspect> of the present disclosure;
L₁ is selected from 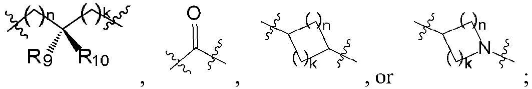 wherein R₉, R₁₀, n, and k are as defined in the compound of Formula I or the pharmaceutically acceptable form thereof according to any embodiment of the <first aspect> of the present disclosure; and R₁₂ and R₁₃ are each independently H, Boc, Cbz, SEM, Fmoc, Alloc, Pht, OTs, PMB, Bn, or Trt.

According to some specific embodiments of the present disclosure, the intermediate compound according to the present disclosure has the following structure: 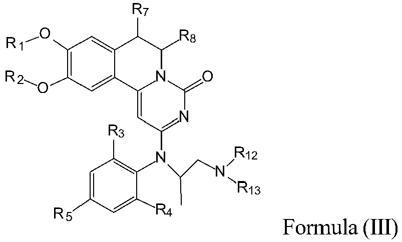 wherein one of R₁₂ and R₁₃ is H, and the other is Boc, Cbz, SEM, Fmoc, Alloc, Pht, OTs, PMB, Bn, or Trt.

### <Third Aspect>

The present disclosure further provides a method for preparing the compound or a pharmaceutically acceptable form thereof according to the <first aspect> of the present disclosure. The synthetic route for preparing the compound of Formula I or the pharmaceutically acceptable form thereof may be designed with reference to methods known in the art and based on the chemical structure of the compound of Formula I or the pharmaceutically acceptable form thereof.

According to some specific embodiments of the present disclosure, the method for preparing the compound or a pharmaceutically acceptable form thereof according to the <first aspect> of the present disclosure comprises:
modifying the carboxyl terminus of the intermediate compound of Formula II according to the <second aspect> of the present disclosure, thereby preparing a compound having the structure of Formula I.

According to some specific embodiments of the present disclosure, the method for preparing the compound or a pharmaceutically acceptable form thereof according to the <first aspect> of the present disclosure further comprises a step of preparing the intermediate compound according to the <second aspect> of the present disclosure.

According to some specific embodiments of the present disclosure, the method for preparing the compound or a pharmaceutically acceptable form thereof according to the <first aspect> of the present disclosure comprises the steps shown in any one of the reaction schemes of Examples 1 to 38.

According to some specific embodiments of the present disclosure, the method for preparing the compound or a pharmaceutically acceptable form thereof according to the <first aspect> of the present disclosure further comprises 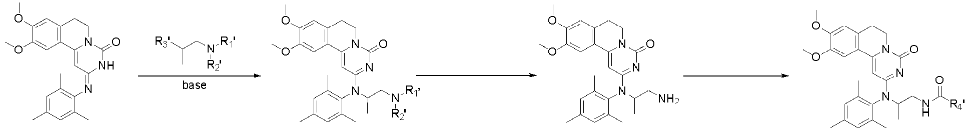
R₁', R₂'= H, Boc, Cbz, SEM, Fmoc, Alloc, Pht, OTs, PMB, Bn, Trt
R₃'=Cl, Br, I, OMs, OTs

### <Fourth Aspect>

The present disclosure further provides a pharmaceutical composition comprising the compound or a pharmaceutically acceptable form thereof (preferably a pharmaceutically acceptable salt thereof) according to the <first aspect> of the present disclosure, and a pharmaceutically acceptable carrier, excipient, and/or one or more additional therapeutic agents.

### <Fifth Aspect>

The present disclosure further provides use of the compound or a pharmaceutically acceptable form thereof (preferably a pharmaceutically acceptable salt thereof) according to the <first aspect> of the present disclosure, or the pharmaceutical composition according to the <fourth aspect> of the present disclosure, in the manufacture of a preparation for inhibiting a phosphodiesterase. Preferably, the phosphodiesterase comprises PDE3 and/or PDE4.

### <Sixth Aspect>

The present disclosure further provides use of the compound or a pharmaceutically acceptable form thereof (preferably a pharmaceutically acceptable salt thereof) according to the <first aspect> of the present disclosure, or the pharmaceutical composition according to the <fourth aspect> of the present disclosure, in the manufacture of a medicament for treating a phosphodiesterase-related disease.

The present disclosure further provides a method for treating a phosphodiesterase-related disease, comprising administering to a subject an effective amount of the compound or a pharmaceutically acceptable form thereof (preferably a pharmaceutically acceptable salt thereof) according to the <first aspect> of the present disclosure, or the pharmaceutical composition according to the <fourth aspect> of the present disclosure.

According to some specific embodiments of the present disclosure, the phosphodiesterase comprises PDE3 and/or PDE4.

According to some specific embodiments of the present disclosure, the phosphodiesterase-related disease comprises a respiratory disease, such as asthma.

According to some specific embodiments of the present disclosure, the subject is a mammal or a human, preferably a human.

The compound having the structure of Formula I or a pharmaceutically acceptable form thereof according to the present disclosure may be used as a phosphodiesterase inhibitor that exhibits potent inhibitory activity against phosphodiesterase, particularly PDE3 and/or PDE4, and therefore has practicability.

### Definition and Description

Unless otherwise indicated, the following terms and phrases used herein are intended to have the following meanings. A specific term or phrase should not be considered indefinite or unclear in the absence of a particular definition, but should be interpreted in its ordinary sense. When a trade name appears herein, it is intended to refer to its corresponding commercial product or an active ingredient thereof.

Unless otherwise specified, the absolute configuration of a stereogenic center is represented by a wedged solid bond ( 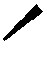 ) and a wedged dashed bond ( 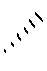 ), the relative configuration of a stereogenic center is indicated by a straight solid bond ( 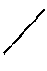 ) and a straight dashed bond ( 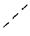 ). A wavy line ( 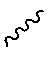 ) represents a wedged solid bond ( 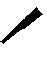 ) or a wedged dashed bond ( 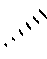 ). Or a wavy line ( 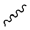 ) represents a straight solid bond ( 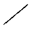 ) and a straight dashed bond ( 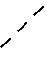 ).

The term "pharmaceutically acceptable" is for those compounds, materials, compositions, and/or dosage forms, is within the scope of reliable medical judgment, and is suitable for use in contact with human and animal tissues, with no excessive toxicity, irritation, allergic reaction or other problems or complications, commensurating with a reasonable benefit/risk ratio.

The term "pharmaceutically acceptable salt" or "pharmaceutically acceptable salt thereof" refers to a salt of the compound according to the present disclosure that is prepared by reacting the compound having a specific substituent according to the present disclosure with a relatively non-toxic acid or base. When the compound according to the present disclosure contains a relatively acidic functional group, a base addition salt can be obtained by bringing the neutral form of the compound into contact with a sufficient amount of a base in a pure solution or a suitable inert solvent. A pharmaceutically acceptable base addition salt includes a salt of sodium, potassium, calcium, ammonium, organic amine, or magnesium, or similar salts. When the compound according to the present disclosure contains a relatively basic functional group, an acid addition salt can be obtained by bringing the neutral form of the compound into contact with a sufficient amount of an acid in a pure solution or a suitable inert solvent. Examples of a pharmaceutically acceptable acid addition salt include an inorganic acid salt, wherein the inorganic acid includes, for example hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid, and the like; and an salt of an amino acid (such as arginine), and a salt of an organic acid such as glucuronic acid, and the like. Certain specific compounds according to the present disclosure contain both basic and acidic functional groups, and can be converted into either a base addition salt or an acid addition salt.

The pharmaceutically acceptable salt according to the present disclosure can be prepared from the parent compound that contains an acidic or basic moiety by a conventional chemistry method. Generally, such a salt can be prepared by reacting the free acid or base form of the compound with a stoichiometric amount of an appropriate base or acid in water or in an organic solvent or in a mixture thereof.

The term "co-crystal" means a crystalline material that comprises two or more distinct solids at room temperature, each having distinctive physical properties, such as structure, melting point, and heat of fusion.

The term "stereoisomer" (also called "optical isomer") refers to a stable isomer that has a vertical asymmetric plane resulting from at least one chiral factor (including a chiral center, a chiral axis, a chiral plane, etc.), thus being capable of rotating the plane polarized light. Since there are asymmetric centers and other chemical structures that may cause stereoisomerism in the compound according to the present disclosure, the present disclosure also includes these stereoisomers and mixtures thereof. Since the compound according to the present disclosure and the salt thereof may comprise asymmetric carbon atom(s), it may exist in the form of a single stereoisomer, a mixture of racemates, enantiomers, or diastereomers. Generally, these compounds may be prepared in the form of a racemic mixture. However, if necessary, such compounds may be prepared or isolated to obtain a pure stereoisomer, that is, a single enantiomer or diastereomer, or, a mixture with an enriched single stereoisomer (purity ≥98%, purity≥95%, ≥93%, ≥90%, ≥88%, ≥85%, or ≥80%). A single stereoisomer of the compound is synthesized from an optically active starting material comprising the required chiral center, or obtained by preparing a mixture of enantiomeric products followed by separation or resolution (for example, by converting a mixture of enantiomeric products into a mixture of diastereomers followed by separation or recrystallization, chromatography, or treatment with a chiral resolution reagent), or obtained by directly separating the enantiomers on a chiral chromatographic column. A starting compounds with specific stereochemistry are commercially available, or may be prepared in accordance with the methods described herein and then resolved by methods well known in the art. Unless otherwise indicated, all stereoisomeric forms of the compounds according to the present disclosure fall within the scope of the compounds according to the present disclosure.

The term "tautomer" (also called "tautomeric forms") refers to structural isomers with different energies that are capable of being converted into each other with a low energy barrier. If tautomerism is possible (such as in a solution), the tautomers may reach a chemical equilibrium. For example, proton tautomers (also called proton-transferred tautomers) include, but are not limited to, interconversion via proton migration, such as keto-enol isomerization, imine-enamine isomerization, amide-iminol isomerization, and the like. Unless otherwise indicated, all tautomeric forms of the compounds according to the present disclosure fall within the scope of the compounds according to the present disclosure.

Unless otherwise specified, the compounds represented by the structural formulae of the present disclosure may be present in the form of a purified single stereoisomer or tautomer, or in the form of a mixture comprising multiple stereoisomers or tautomers.

The term "solvate" refers to a substance formed by a compound or the pharmaceutically acceptable salt thereof according to the present disclosure and at least one solvent molecule binding through non-covalent intermolecular force. Common solvates include, but are not limited to, hydrates, ethanolates, acetonates, and the like.

The term "chelate" means a complex having a cyclic structure, which is obtained by chelation of two or more ligands to the same metal ion to form a chelated ring.

The term "non-covalent complex" means a complex formed by interaction between a compound and another molecule, wherein no covalent bond is formed between the compound and the molecule. For example, they may be complexed via van der Waals interaction, hydrogen bonding, or electrostatic interaction (also referred to as ionic bonding).

The term "prodrug" refers to a derivative compound capable of directly or indirectly providing the compound according to the present disclosure after administered to a patient. A particularly preferred derivative compound or prodrug is one capable of increasing the bioavailability of the compound according to the present disclosure after administered to a patient (for example, entering the blood more easily), or a compound capable of promoting the delivery of the parent compound to the site of action (for example, lymphatic system). Unless otherwise indicated, all prodrugs of the compounds according to the present disclosure fall within the scope of the present disclosure, and various prodrugs are well known in the art.

The term "each independently" means that at least two groups (or ring systems) having the same or similar value ranges and existing in the same structure may have the same or different meanings in a specific situation. For example, if substituent X and substituent Y are each independently hydrogen, halogen, hydroxy, cyano, alkyl, or aryl, when substituent X is hydrogen, substituent Y may be hydrogen or may be halogen, hydroxy, cyano, alkyl, or aryl; and similarly, when substituent Y is hydrogen, substituent X may be hydrogen, or may be halogen, hydroxy, cyano, alkyl, or aryl.

The terms "comprise," "comprises," "comprising," "include," "includes," and "including" are intended to be open-ended and non-limiting.

"Optional" or "optionally" means that the subsequent event or situation may, but not necessarily, occur, and the term includes the case in which the event or situation occurs and the case in which the event or situation does not occur.

The term "substituted" means one or more hydrogen atom(s) on a specific atom are substituted with a substituent, including deuterium and hydrogen variants, as long as the valence of the specific atom is normal and the compound after the substitution is stable. When the substituent is oxo (i.e. =O), it means two hydrogen atoms are substituted. An oxo substitution does not occur on an aromatic ring. The term "optionally substituted" means substituted or not substituted, unless otherwise specified, and the type and number of the substituent may be arbitrary as long as they are chemically possible.

When any variable (such as R) occurs more than once in the constitution or structure of a compound, the definition of the variable at each occurrence is independent. Thus, for example, if a group is substituted with 0-2 Rs, the group can be optionally substituted with up to two Rs, wherein the definition of each R is independent. Moreover, a combination of the substituent and/or the variant thereof is allowed only when the combination results in a stable compound.

Where a variable in a structural formula is absent, it means that the variable is not present. For example, where R in C-R is absent, the corresponding structure is actually C.

Where a variable linking two groups in a structural formula is a single bond or is absent, the two groups linked by the variable are directly connected. For example, where L in A-L-Z represents a bond or is absent, the corresponding structure is actually A-Z.

Where the atom in a substituent through which the substituent is linked to a group being substituted is not specified, the substituent may be linked through any atom thereof. For example, where phenyl is a substituent, it may be attached to the group being substituted through any carbon atom on the benzene ring.

Unless otherwise specified, the term "alkyl" refers to a linear or branched saturated hydrocarbon group, which may be mono-substituted (e.g. -CH₂F) or multi-substituted (e.g. -CF₃), and may be monovalent (e.g. methyl), divalent (e.g. methylene) or multivalent (e.g. methenyl). Examples of alkyl include methyl (Me), ethyl (Et), propyl (such as n-propyl and isopropyl), butyl (such as n-butyl, isobutyl, s-butyl, t-butyl), pentyl (such as n-pentyl, isopentyl, neopentyl) and the like.

Unless otherwise specified, the term "alkylene" refers to a divalent linear or branched saturated hydrocarbon group consisting solely of carbon atoms and hydrogen atoms, having no saturation, and linked to other moieties through two single bonds, including, but not limited to, methylene, 1,1-ethanediyl, 1,2-ethanediyl, and the like. For example, "C₁₋₃ alkylene" refers to a saturated divalent linear or branched hydrocarbon group containing 1 to 3 carbon atoms.

Unless otherwise specified, "cycloalkyl" includes any stable cyclic or polycyclic hydrocarbyl, and any carbon atom is saturated, which may be mono-substituted or multi-substituted, and may be monovalent, divalent, or multivalent. Examples of cycloalkyl include, but are not limited to, cyclopropyl, norbonanyl, [2.2.2]bicyclooctanyl, [4.4.0]bicyclodecanyl, and the like.

Unless otherwise specified, the term "alkoxy" refers to an alkyl group attached to the remainder of the molecule through an oxygen atom, wherein the alkyl group has the meaning described in the present disclosure. Unless otherwise specified, a C₁₋₅ alkoxy includes C1, C2, C3, C4 and C5 alkoxy. Examples of alkoxy include, but are not limited to, methoxy, ethoxy, n-propoxy, isopropoxy, n-butoxy, sec-butoxy, tert-butoxy, n-pentyloxy and S-pentoxy. The alkoxy may optionally be substituted with one or more substituents described herein.

Unless otherwise specified, the term "3- to 6-membered ring" refers to a saturated or unsaturated monocyclic ring with or without heteroatoms, wherein the ring has 3, 4, 5, or 6 atoms selected from C, O, S, and N. The 3- to 6-membered ring may be attached to the remainder of the molecule through any carbon atom, or through any ring nitrogen atom, if present.

Unless otherwise specified, the term "amine (amino)" means -NH₂, -NH(alkyl) or -N(alkyl)(alkyl).

Unless otherwise specified, the term "aromatic ring" refers to a polyunsaturated aromatic monocyclic hydrocarbon ring, which may be mono- or multi-substituted.

Unless otherwise specified, the term "4- to 6-membered heterocycloalkyl" refers to a saturated monovalent monocyclic hydrocarbon ring containing 3, 4 or 5 carbon atoms and one or more heteroatoms selected from O and NR^{a}, wherein R^{a} represents a hydrogen atom or a C₁₋₆ alkyl; the "4- to 6-membered heterocycloalkyl" may be attached to the rest of the molecule through any carbon atom, or through a nitrogen atom, if present.

Unless otherwise specified, the term "heteroaromatic ring" represents an aromatic ring containing 1 to 4 heteroatoms selected from one or more of N, O and S.

Unless otherwise specified, the term "heterocyclyl" refers to a saturated or partially saturated, monocyclic or polycyclic (for example, bicyclic such as fused cyclic, bridged cyclic or spiro cyclic) nonaromatic group that has ring atoms consisting of carbon atoms and at least one heteroatom selected from N, O and S, wherein the sulfur atom is optionally substituted to form S(=O), S(=O)₂ or S(=O)(=NR^{x}) wherein R^{x} is independently H or a C₁₋₄ alkyl. A heterocyclyl group may be attached to the remainder of the molecule through any ring atom, provided that the valence requirements are satisfied. For example, the term "3- to 8-membered heterocyclyl" as used herein refers to a heterocyclyl having 3 to 8 ring atoms. For example, a heterocyclyl may be oxiranyl, aziridinyl, azetidinyl, oxetanyl, tetrahydrofuryl, dioxolyl, pyrrolidinyl, pyrrolidinonyl, imidazolidinyl, pyrazolidinyl, tetrahydropyranyl, piperidinyl, piperazinyl, morpholinyl, thiomorpholinyl, dithianyl, or trithianyl.

Unless otherwise specified, the term "aryl" refers to a monocyclic or fused polycyclic, aromatic hydrocarbyl having a conjugated π-electron system. For example, the term "C₆₋₁₀ aryl" as used herein refers to an aryl having 6 to 10 carbon atoms. For example, an aryl may be phenyl, naphthyl, anthracyl, phenanthryl, acenaphthenyl, azulenyl, fluorenyl, indenyl, pyrenyl, etc.

Unless otherwise specified, the term "heteroaryl" refers to a monocyclic or fused polycyclic aromatic group having a conjugated π-electron system, the ring atoms of which consist of carbon atoms and at least one heteroatom selected from N, O and S. A heteroaryl group may be attached to the remainder of the molecule through any ring atom, provided that valence requirements are satisfied. For example, the term "5- to 10-membered heteroaryl" as used herein refers to a heteroaryl having 5 to 10 ring atoms. For example, a heteroaryl may be thienyl, furyl, pyrrolyl, oxazolyl, thiazolyl, imidazolyl, pyrazolyl, isoxazolyl, isothiazolyl, oxadiazolyl, triazolyl, thiadiazolyl, pyridinyl, pyridazinyl, pyrimidyl, pyrazinyl, triazinyl and benzo derivatives thereof, pyrrolopyridyl, pyrrolopyrazinyl, pyrazolopyridyl, imidazopyridyl, pyrrolopyrimidinyl, pyrazolopyrimidinyl, purinyl, etc.

Unless otherwise specified, the term "halogen" refers to fluorine (F), chlorine (Cl), bromine (Br), or iodine (I). The term "hydroxy" refers to -OH. The term "cyano" refers to -CN. The term "amino" refers to -NH₂.

Unless otherwise specified, the term "pharmaceutical composition" refers to a mixture of one or more of the compounds or pharmaceutically acceptable forms thereof according to the present disclosure with other chemical components, wherein the "other chemical components" means pharmaceutically acceptable carriers, excipients and/or one or more other therapeutic agents. A "carrier" refers to a material that does not cause significant irritation to an organism and does not abolish the biological activity and properties of the administered compound. An "excipient" refers to an inert substance added to a pharmaceutical composition to facilitate the administration of a compound. Non-limiting examples include calcium carbonate, calcium phosphate, sugar, starch, cellulose derivatives (including microcrystalline cellulose), gelatin, vegetable oils, polyethylene glycols, diluents, granulators, lubricants, binders, and disintegrants.

The compounds according to the present disclosure exhibit good inhibitory activity against PDE and have potential use in the treatment of diseases associated with PDE, particularly diseases associated with PDE3 and/or PDE4.

### DESCRIPTION OF THE DRAWINGS

FIG. 1 shows the X-ray diffraction pattern of single crystal of Compound 1-2 according to the present disclosure.
FIGs. 2A to 2G show the results of cell viability assays evaluating the effects of the compounds on bronchial smooth muscle cells.
FIG. 3 shows the TNF-α levels in cell supernatants measured in a pharmacodynamic study of the compounds in an LPS-induced inflammation model.
FIGs. 4A and 4B show the effects of Compound 10 according to the present disclosure on the airway resistance (RL, cmH₂O) and changes thereof in guinea pigs, respectively.
FIGs. 5A and 5B show the effects of Compound 1-2 according to the present disclosure on the airway resistance (RL, cmH₂O) and changes thereof in guinea pigs, respectively.

### DESCRIPTION OF EMBODIMENTS

The embodiments of the present disclosure will be described in detail below in connection with examples. It should be understood by those skilled in the art that the following examples are merely illustrative of the present invention and are not intended to limit the scope of the present invention. Unless otherwise indicated, examples for which no specific conditions are provided were carried out under conventional conditions or under conditions recommended by the manufacturers. Unless otherwise indicated, reagents and apparatus for which no manufacturer is specified are commercially available.

The structures of the compounds were determined by NMR or mass spectrometry. NMR was measured using a BRUKER 400M NMR spectrometer, with deuterated dimethyl sulfoxide (DMSO-*d*₆) or deuterated chloroform (CDCl₃) as a measurement solvent and tetramethylsilane (TMS) as an internal standard, and the chemical shifts (δ) were given in ppm (10⁻⁶). Mass spectra were obtained using a Waters ACQUITY Arc/ACQUITY QDa or Thermo U3000-ISQ EC LC-MS spectrometer.

HPLC analysis was performed using a Thermo U3000 high pressure liquid chromatograph. The high performance liquid preparative chromatography was carried out using a Hambone DAC-50 or Shimadzu LC-20AP preparative chromatograph.

Reactions were monitored using thin-layer chromatography or LC-MS chromatography. The following solvent systems were used in thin layer chromatography: a dichloromethane/methanol system, or a petroleum ether/ethyl acetate system, with the volume ratio of the solvents adjusted according to the polarity of the compound or by adding a small amount of triethylamine and the like. LC-MS was performed using a Waters ACQUITY Arc/ACQUITY QDa or Thermo U3000-ISQ EC LC-MS spectrometer.

200-300 mesh silica gel was generally used as the carrier in column chromatography. The eluent systems used included a dichloromethane/methanol system, or a petroleum ether/ethyl acetate system. The solvent volume ratios were adjusted according to the polarity of different compounds, or by adding a small amount of triethylamine and the like.

In the following examples and comparative examples, unless otherwise stated, the reaction temperature was room temperature (20°C to 30°C), and the solvents were dried and purified according to standard methods.

The comparative compounds are shown in Table 2.

**Table 2. Comparative Compounds**

| **Comparative Example** | **Compound Structure** | **Comparati ve Example** | **Compound Structure** |
|---|---|---|---|
| **Positive control** | 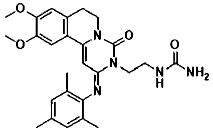 | D4 | |
| RPL-554 | | | |
| D1 | | D5 | |
| D2 | | D6 | 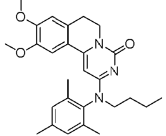 |
| D3 | | D7 | 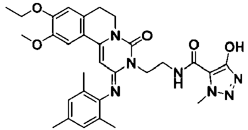 |

### Preparation of Comparative Compound D1:

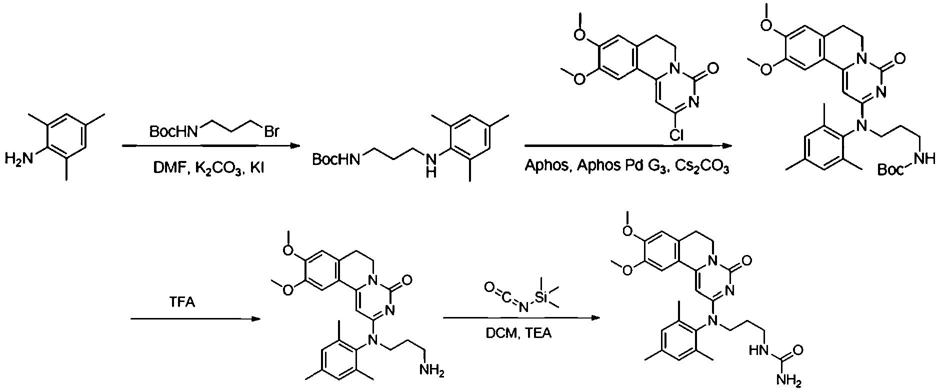

### Preparation steps:

**1.**

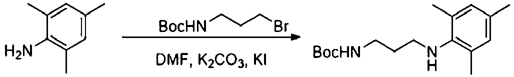

Under a nitrogen atmosphere at room temperature, K₂CO₃ (6.1 g, 44.3 mmol, 3 equiv.) and KI (2.5 g, 14.7 mmol, 1 equiv.) were added separately to a solution of 2,4,6-trimethylaniline (2.0 g, 14.7 mmol, 1 equiv.) and tert-butyl N-(3-bromopropyl)carbamate (14.1 g, 59.1 mmol, 4 equiv.) in DMF (20 mL, 258.4 mmol). A reaction was allowed to proceed at 80°C overnight. The reaction mixture was extracted with EA (3×50 ml). The organic phases were combined, washed with saturated sodium chloride solution (2×30 ml), and dried over anhydrous sodium sulfate. The resultant was filtered, and concentrated under reduced pressure. The resulting residue was purified by silica gel column chromatography using EA/PE (1/4) to give tert-butyl N-(3-((2,4,6-trimethylphenyl)amino)propyl)carbamate (2.2 g, 50.86% yield).

LCMS (ESI, m/z): [M+H] ⁺ = 293.2

**2.**

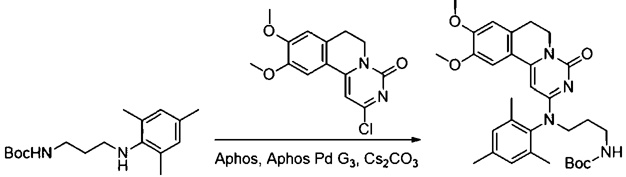

Under a nitrogen atmosphere at room temperature, Cs₂CO₃ (2.7 g, 8.2 mmol, 2 equiv.), APhos Pd G₃ (0.3 g, 0.4 mmol, 0.1 equiv.), and 4-(di-tert-butylphosphino)-N,N-dimethylaniline (0.1 g, 0.4 mmol, 0.1 equiv.) were added separately to a solution of tert-butyl N-(3-((2,4,6-trimethylphenyl)amino)propyl)carbamate (1.2 g, 4.1 mmol, 1 equiv.) and 2-chloro-9,10-dimethoxy-6,7-dihydropyrimido[4,3-a]isoquinolin-4-one (1.8 g, 6.1 mmol, 1.5 equiv.) in dioxane (20 mL). A reaction was allowed to proceed at 100°C under stirring overnight under a nitrogen atmosphere. The reaction mixture was extracted with EA (3× 50 mL). The organic phases were combined, washed with saturated sodium chloride solution (2× 30 mL), and dried over anhydrous sodium sulfate. The resulting mixture was filtered, and concentrated under reduced pressure. The resulting residue was purified by reverse-phase column chromatography under the following conditions: C18 column; mobile phase: water and acetonitrile; gradient: 10% to 90% over 30 min; UV: 254 nm. Tert-butyl N-[3-((9,10-dimethoxy-4-oxo-6H,7H-pyrimido[4,3-a]isoquinolin-2-yl)(2,4,6-trimethylphenyl)amino)propyl]carbamate was obtained (200 mg, 8.88% yield).

LCMS (ESI, m/z): [M+H] ⁺ = 549.4

**3.**

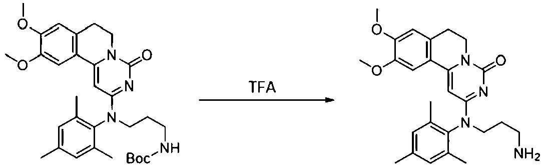

Under a nitrogen atmosphere at 0°C, TFA (5 mL) was added dropwise to a solution of tert-butyl N-[3-((9,10-dimethoxy-4-oxo-6H,7H-pyrimido[4,3-a]isoquinolin-2-yl)(2,4,6-trimethylphenyl)amino)propyl]carbamate (290 mg, 1 equiv.) in DCM (5 mL). The resulting residue was stirred for reaction at room temperature under a nitrogen atmosphere for 2 h. The reaction mixture was extracted with DCM (3×20 ml). The organic phases were combined, washed with saturated sodium chloride solution (2× 10 mL), and dried over anhydrous sodium sulfate. The resulting mixture was filtered, and concentrated under reduced pressure. 2-[(3-aminopropyl)(2,4,6-trimethylphenyl)amino]-9,10-dimethoxy-6H,7H-pyrimido[4,3-a]isoquinolin-4-one was obtained (200 mg, crude product).

LCMS (ESI, m/z): [M+H] ⁺ = 449.3

4.

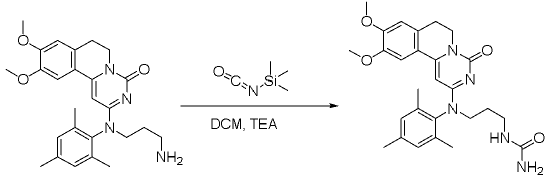

Under a nitrogen atmosphere at 0°C, trimethylsilyl isocyanate (128.4 mg, 1.1 mmol, 2.5 equiv.) was added dropwise to a solution of 2-[(3-aminopropyl)(2,4,6-trimethylphenyl)amino]-9,10-dimethoxy-6H,7H-pyrimido[4,3-a]isoquinolin-4-one (200 mg, 0.4 mmol, 1.0 equiv.) and TEA (135 mg, 1.3 mmol, 3 equiv.) in DCM (5 mL). The resulting mixture was stirred for reaction at 50°C under a nitrogen atmosphere for 2 days. The reaction mixture was concentrated under reduced pressure. The crude product was purified by HPLC under the following conditions: column: XBridge BEH Phenyl, 5 µm, 19 mm × 250 mm; mobile phase A: water (0.1% FA); mobile phase B: MeOH; flow rate: 25 mL/min; gradient: 43% B to 53% B over 10 min; wavelength: 254 nm/220 nm; RT1 (min): 9.6. 3-((9,10-dimethoxy-4-oxo-6H,7H-pyrimido[4,3-a]isoquinolin-2-yl)(2,4,6-trimethylphenyl)amino)propylurea was obtained (7.5 mg, 3.4% yield),.
LCMS (ESI, m/z): [M+H] ⁺ = 492.25
¹H NMR (400 MHz, Methanol-*d*₄) δ 8.22 (broad, 1H), 7.26 (s, 1H), 7.01 (s, 2H), 6.94 (s, 1H), 6.24 (s, 1H), 4.05 (t, *J =* 6.4 Hz, 2H), 3.91 (s, 6H), 3.61 (t, *J =* 7.8 Hz, 2H), 3.45 (t, *J* = 6.6 Hz, 2H), 3.01 - 2.89 (m, 2H), 2.29 (s, 3H), 2.24 (s, 6H), 1.88 (s, 2H).

### Preparation of Comparative Compound D2:

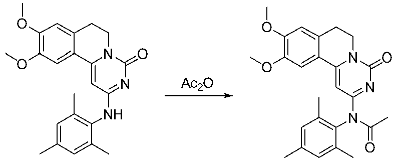

At room temperature, (2E)-9,10-dimethoxy-2-[(2,4,6-trimethylphenyl)amino]-3H,6H, 7H-pyrimido[4,3-a]isoquinolin-4-one (1.0 g, 2.55 mmol, 1.0 equiv.) and dichloromethane (20 mL) were added to a 50 mL flask and stirred. Acetic anhydride (0.29 g, 2.81 mmol, 1.1 equiv.) was added, and the reaction mixture was stirred at room temperature under a nitrogen atmosphere for 24 h. Upon completion of the reaction, the reaction system was cooled to 0°C, and water (10 mL) was added dropwise. The mixture was stirred for 1 h and extracted with dichloromethane three times. The organic phases were combined, dried, and concentrated by rotary evaporation. The resultant was purified by silica gel column chromatography to give N-(9,10-dimethoxy-4-oxo-6,7-dihydro-4H-pyrimido[6,1-a]isoquinolin-2-yl)-N-(2,4,6-trimethylphenyl)acetamide (0.11 g).
LCMS (ESI, m/z): [M+H] ⁺ =434.21
¹H NMR (400 MHz, CDCl₃) δ 7.72 (s, 1H), 7.18 (s, 1H), 6.94 (s, 2H), 6.74 (s, 1H), 4.19 (t, *J* = 6.4 Hz, 2H), 3.92 (s, 6H), 2.95 (t, *J =* 6.4 Hz, 2H), 2.31 (s, 3H), 2.12 (s, 6H), 2.05 (s, 3H).

### Preparation of Comparative Compound D3:

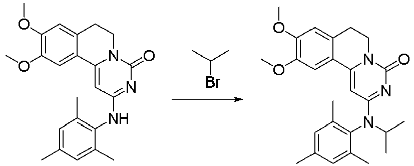

(2E)-9,10-Dimethoxy-2-[(2,4,6-trimethylphenyl)amino]-3H,6H,7H-pyrimido[4,3-a]isoquinolin-4-one (1.00 g, 2.554 mmol, 1.0 equiv.), 2-bromopropane (0.47 g, 3.831 mmol, 1.5 equiv.), Pd₂(dba)₃ (0.23 g, 0.255 mmol, 0.1 equiv.), and Cs₂CO₃ (2.50 g, 7.662 mmol, 3.0 equiv.) were dissolved in 1,4-dioxane (20 mL). The reaction mixture was heated to reflux for reaction with stirring under a nitrogen atmosphere for 12 h. After completion of the reaction, water (50 mL) was added, and the mixture was extracted with ethyl acetate (50 mL × 3), washed with saturated sodium chloride solution (50 mL × 1), dried over anhydrous sodium sulfate, filtered, and concentrated under reduced pressure to give 2-(isopropyl(2,4,6-trimethylphenyl)amino)-9,10-dimethoxy-6,7-dihydro-4H-pyrimido[6,1-a]isoquinolin-4-one (7.8 g, 71.43%).
LCMS (ESI, m/z): [M+H] ⁺ = 434.25
¹H NMR (400 MHz, Methanol-*d*₄) δ 7.08 (s, 2H), 6.89 (s, 1H), 6.58 (s, 1H), 5.35 (s, 1H), 4.82 - 4.74 (m, 1H), 4.06 (t, *J =* 6.4 Hz, 2H), 3.86 (s, 3H), 3.63 (s, 3H), 2.93 (t, *J* = 6.4 Hz, 2H), 2.34 (s, 3H), 2.14 (s, 3H), 1.25 (s, 3H), 1.23 (s, 3H).

### Preparation of Comparative Compound D4:

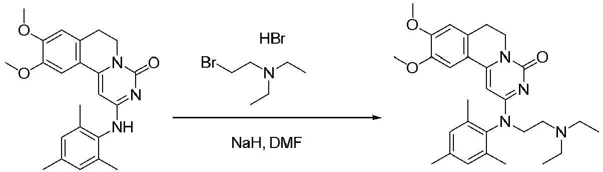

9,10-Dimethoxy-2-[(2,4,6-trimethylphenyl)amino]-6h,7h-pyrimido[4,3-a]isoquinolin-4-one (200 mg, 0.5 mmol, 1.0 equiv.) was dissolved in DMF (2 mL). NaH (40 mg, 1.0 mmol, 2.0 equiv., 60% purity) was added at 0°C, and the resulting mixture was stirred under a nitrogen atmosphere for 0.5 h. 2-(diethylamino)ethyl bromide hydrobromide (400 mg, 1.5 mmol, 3.0 equiv.) was added to the above mixture, and the reaction mixture was stirred at room temperature for 1 h. Water (10 mL) was added at room temperature to quench the reaction. The mixture was extracted with ethyl acetate (2 × 50 mL). The organic layer was washed with saturated brine (3 × 100 mL), dried over anhydrous sodium sulfate, filtered, and concentrated under reduced pressure. The resultant was purified by column chromatography (EA/PE = 1/2) to give 2-{[2-(diethylamino)ethyl](2,4,6-trimethylphenyl)amino}-9,10-dimethoxy-6h,7h-pyrimidine[4,3-a]isoquinolin-4-one (37.2 mg, 14% yield).
LCMS (ESI): [M+H] ⁺ = 491.25
¹H NMR (400 MHz, Methanol-*d*₄) δ 6.95 - 6.85 (m, 3H), 6.77 (s, 1H), 5.50 (s, 1H), 4.44 - 4.35 (m, 2H), 4.06 - 3.99 (m, 2H), 3.87 (s, 3H), 3.69 (s, 3H), 2.99 - 2.90 (m, 4H), 2.74 (q, *J =* 7.2 Hz, 4H), 2.28 (s, 3H), 2.05 (s, 6H), 1.16 (t, *J =* 7.2 Hz, 6H).

### Preparation of Comparative Compound D5:

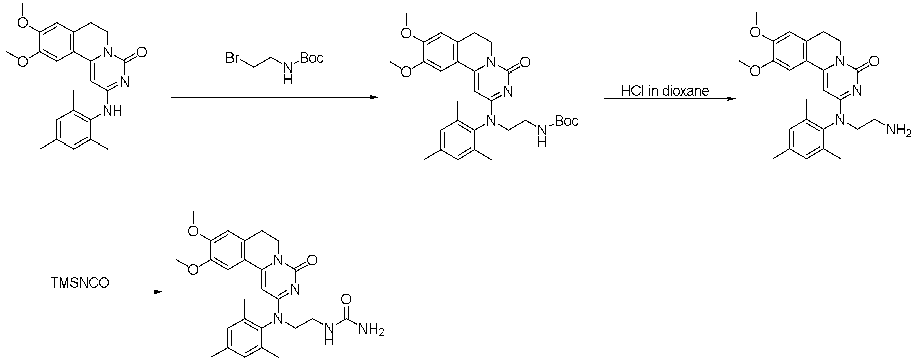

### Preparation steps:

**1.**

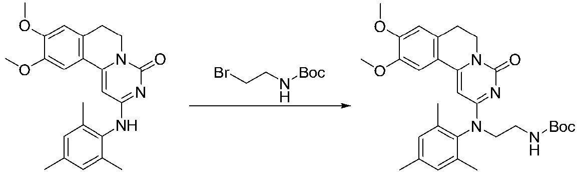

Under a nitrogen atmosphere at room temperature, 9,10-dimethoxy-2-[(2,4,6-trimethylphenyl)amino]-6H,7H-pyrimido[4,3-a]isoquinolin-4-one (500 mg, 1.2 mmol, 1.0 equiv.) and Cs₂CO₃ (1,248 mg, 3.8 mmol, 3.0 equiv.) were added to DMF (10 mL). And tert-butyl N-(2-bromoethyl)carbamate (715 mg, 3.1 mmol, 2.5 equiv.) and Pd₂(dba)₃ (116 mg, 0.1 mmol, 0.1 equiv.) were then added batchwise. The resulting residue was stirred at 100°C for a reaction under a nitrogen atmosphere overnight. Then the reaction mixture was extracted with ethyl acetate (3× 50 mL). The organic phases were combined, washed with saturated sodium chloride solution (2 × 30 mL), and dried over anhydrous sodium sulfate. The resulting mixture was filtered, and concentrated under reduced pressure. The resulting residue was purified by silica gel column chromatography eluting with dichloromethane/methanol (20/1) to give tert-butyl N-[2-((9,10-dimethoxy-4-oxo-6H,7H-pyrimido[4,3-a]isoquinolin-2-yl)(2,4,6-trimethylphenyl)amino)ethyl]carbamate (200 mg, 29.29%).

LCMS (ESI): [M+H] ⁺ = 535.2

**2.**

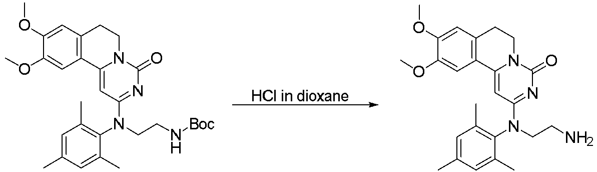

Under a nitrogen atmosphere at room temperature, tert-butyl N-[2-((9,10-dimethoxy-4-oxo-6H,7H-pyrimido[4,3-a]isoquinolin-2-yl)(2,4,6-trimethylphenyl)amino)ethyl]carbamate (180 mg, 0.3 mmol, 1.0 equiv.) was added to a HCl solution in dioxane (4 mL, 4 M), and the mixture was stirred for reaction for 1 h. The reaction mixture was basified to pH 8 with saturated aqueous sodium bicarbonate solution. Then the reaction mixture was extracted with ethyl acetate (3× 20 mL). The organic phases were combined, washed with saturated sodium chloride solution (2 × 10 mL), and dried over anhydrous sodium sulfate. The resulting mixture was filtered, and concentrated under reduced pressure to obtain 2-[(2-aminoethyl)(2,4,6-trimethylphenyl)amino]-9,10-dimethoxy-6H,7H-pyrimido[4,3-a]isoquinolin-4-one (160 mg, crude product).

LCMS (ESI): [M+H] ⁺ = 435.2

**3.**

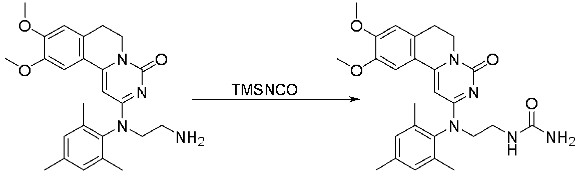

Under a nitrogen atmosphere at room temperature, 2-[(2-aminoethyl)(2,4,6-trimethylphenyl)amino]-9,10-dimethoxy-6H,7H-pyrimido[4,3-a]isoquinolin-4-one (100 mg, 0.2 mmol, 1.0 equiv) and TEA (69 mg, 0.6 mmol, 3.0 equiv) were added to DCM (5 mL), followed by dropwise addition of trimethylsilyl isocyanate (53 mg, 0.4 mmol, 2.0 equiv). The resulting mixture was stirred for reaction at room temperature under a nitrogen atmosphere for 1.5 h. The reaction mixture was extracted with dichloromethane (3× 20 mL). The organic phases were combined, washed with saturated brine (2 × 50 mL), and dried over anhydrous sodium sulfate. The resulting mixture was filtered, and concentrated under reduced pressure. The crude product was purified by HPLC (under the following conditions: column: YMC Triart C18 ExRs, 5 µm, 19 mm × 250 mm; mobile phase A: water (10 mmol/L NH₄HCO₃); mobile phase B: acetonitrile; flow rate: 25 mL/min; gradient: 32% B to 55% B in 10 min; wavelength: 254 nm/220 nm; RT1 (min): 9.17), to give 2-((9,10-dimethoxy-4-oxo-6H,7H-pyrimido[4,3-a]isoquinolin-2-yl)(2,4,6-trimethylphenyl)amino)ethylurea (26.4 mg, 24.02%).
LCMS (ESI): [M+H] ⁺ = 478.20
¹H NMR (400 MHz, Methanol-*d*₄) δ 7.11 (s, 2H), 6.94 (s , 1H), 6.70 (s, 1H), 5.51 (s, 1H), 4.15 - 4.08 (m, 2H), 3.99 - 3.92 (m, 2H), 3.89 (s, 3H), 3.67 (s, 3H), 3.44 (t, *J =* 6.4 Hz, 2H), 2.97 (t, *J* = 6.4 Hz, 2H), 2.36 (s, 3H), 2.18 (s, 6H).

### Preparation of Comparative Compound D6:

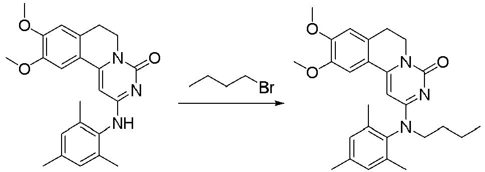

(2E)-9,10-Dimethoxy-2-[(2,4,6-trimethylphenyl)amino]-3H,6H,7H-pyrimido[4,3-a]isoquinolin-4-one (500 mg, 1.28 mmol, 1.0 equiv.), 1-bromobutane (263 mg, 1.92 mmol, 1.5 equiv.), Pd₂(dba)₃ (119 mg, 0.13 mmol, 0.1 equiv.), Cs₂CO₃ (1.25 g, 3.84 mmol, 3.0 equiv.), and Xantphos (150 mg, 26 mmol, 0.2 equiv.) were dissolved in 1,4-dioxane (10 mL). The mixture was heated under stirring to reflux for reaction under a nitrogen atmosphere for 12 h. After completion of the reaction, water (50 mL) was added, and the mixture was extracted with ethyl acetate (50 mL × 3), washed with saturated sodium chloride solution (50 mL × 1), dried over anhydrous sodium sulfate, filtered, and concentrated under reduced pressure. The resultant was purified by silica gel column chromatography to give 2-(butyl(2,4,6-trimethylphenyl)amino)-9,10-dimethoxy-6,7-dihydro-4H-pyrimido[6,1-a]isoquinolin-4-one (121 mg, 21.1%).

LCMS (ESI): [M+H] ⁺ = 448.26
¹H NMR (400 MHz, CDCl₃) δ 7.01 (s, 2H), 6.71 (s, 1H), 6.64 (s, 1H), 5.34 (s, 1H), 4.17 (t, *J =* 6.4 Hz, 2H), 3.93 - 3.85 (m, 5H), 3.73 (s, 3H), 2.90 (t, *J* = 6.4 Hz, 2H), 2.35 (s, 3H), 2.16 (s, 6H), 1.62 - 1.51 (m, 2H), 1.40 - 1.27 (m, 2H), 0.89 (t, *J =* 8.0 Hz).

### Example 1, Compound 1-1, and Compound 1-2

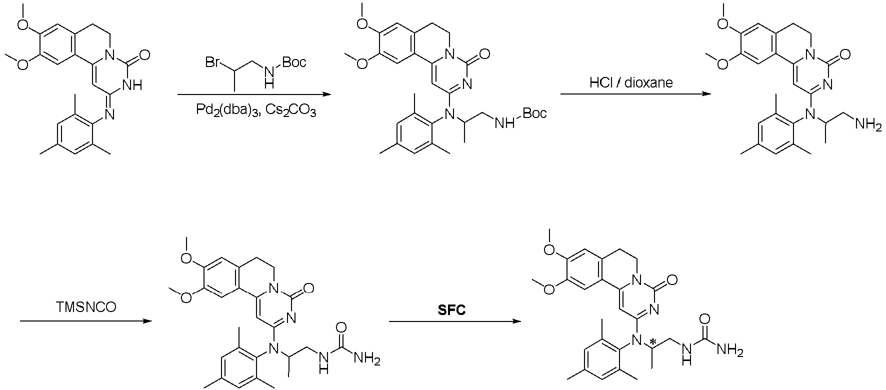

### Preparation steps:

**1.**

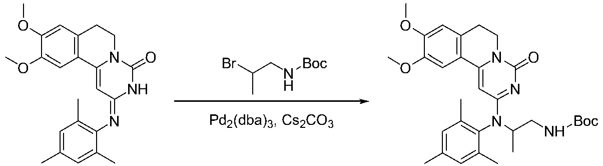

tert-Butyl N-(2-bromopropyl)carbamate (0.91 g, 3.8 mmol, 1.5 equiv), cesium carbonate (2.50 g, 7.662 mmol, 3.0 equiv), Pd₂(dba)₃ (0.23 g, 0.26 mmol, 0.1 equiv), and (E)-2-((2,4,6-trimethylphenyl)imino)-9,10-dimethoxy-2,3,6,7-tetrahydro-4H-pyrimido[6,1-a]isoquinolin-4-one (1 g, 2.54 mmol, 1.0 equiv) were dissolved in 1,4-dioxane (20 mL). The reaction mixture was stirred at 110°C overnight under a nitrogen atmosphere. The mixture was concentrated under reduced pressure, and the residue was dissolved in 10 mL of water. The resultant was extracted with ethyl acetate (3 × 10 mL). The organic layers were washed with 20 mL saturated aqueous sodium chloride solution, dried over anhydrous sodium sulfate, filtered, and concentrated under reduced pressure. The crude product was purified by reversed-phase column chromatography under the following conditions: column: C18; mobile phase A: water (10 mmol/L ammonium bicarbonate); mobile phase B: acetonitrile; flow rate: 60 mL/min; gradient: 30% B to 50% B over 20 min; wavelength: 254 nm, to give tert-butyl N-[(E)-2-(2-((2,4,6-trimethylphenyl)amino)-9,10-dimethoxy-4-oxo-6,7-dihydro-2H-pyrimido[6,1-a]isoquinolin-3(4H)-yl)propyl]carbamate (500 mg, 35.6% yield).

LCMS (ESI): [M+H] ⁺ = 549.06

**2.**

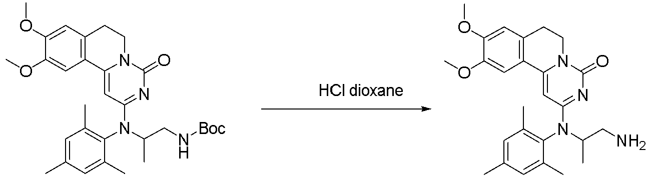

tert-Butyl N-[(E)-2-(2-((2,4,6-trimethylphenyl)amino)-9,10-dimethoxy-4-oxo-6,7-dihydro-2H-pyrimido[6,1-a]isoquinolin-3(4H)-yl)propyl]carbamate (500 mg, 0.91 mmol, 1.0 equiv) was dissolved in a HCL solution in 1,4-dioxane (4 M, 10 mL), and was stirred at room temperature for 1 h. The mixture was concentrated under reduced pressure, dissolved in 10 mL water, and extracted with ethyl acetate (3 × 10 mL). The organic layers were washed with 20 mL saturated aqueous sodium chloride solution, dried over anhydrous sodium sulfate, filtered, and concentrated under reduced pressure, to give 2-((1-aminopropan-2-yl)(2,4,6-trimethylphenyl)amino)-9,10-dimethoxy-6,7-dihydro-4H-pyrimido[6,1-a]isoquinolin-4-one (300 mg, 73.3% yield).

LCMS (ESI): [M+H] ⁺ = 449.16

**3.**

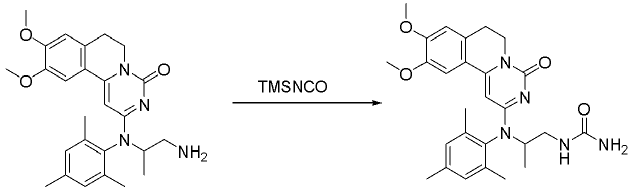

2-((1-Aminopropan-2-yl)(2,4,6-trimethylphenyl)amino)-9,10-dimethoxy-6,7-dihydro-4H-pyrimido[6,1-a]isoquinolin-4-one (200 mg, 0.45 mmol, 1.0 equiv), triethylamine (135 mg, 1.34 mmol, 3.0 equiv), and trimethylsilyl isocyanate (102.73 mg, 0.9 mmol, 2.0 equiv) were dissolved in 10 ml dichloromethane, followed by stirring at room temperature for 2 h. The mixture was concentrated under reduced pressure, dissolved in 10 mL water, and extracted with ethyl acetate (3 × 10 mL). The organic layers were washed with 20 mL saturated aqueous sodium chloride solution, dried over anhydrous Na₂SO₄, filtered, and concentrated under reduced pressure. The crude product was purified by reversed-phase column chromatography under the following conditions: column: Xselect CSH C18, 5 µm, 30 mm × 150 mm; mobile phase A: water (0.1% FA); mobile phase B: acetonitrile; flow rate: 60 mL/min; gradient: 28% B to 38% B over 8 min; wavelength: 254 nm/220 nm; RT1 (min): 5.85/7.65, to give 1-(2-((9,10-dimethoxy-4-oxo-6,7-dihydro-4H-pyrimido[6,1-a]isoquinolin-2-yl)(2,4,6-trimethylphenyl)amino)propyl)urea (100 mg, 45.6% yield).

LCMS (ESI): [M+H] ⁺ = 492.35

**4.**

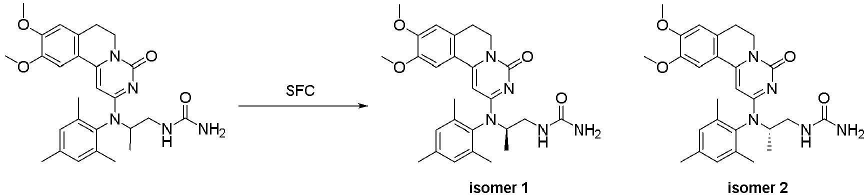

The racemate (100 mg) was separated by chiral HPLC under the following conditions: column: CHIRALPAK IE-3, 4.6 × 50 mm, 3 µm; mobile phase A: DCM-HPLC; mobile phase B: ETOH: DCM = 1:1; flow rate: 18 mL/min; gradient: isocratic 20; wavelength: 220 nm; RT1 (min): 17.4; RT2 (min): 21.8; sample solvent: MeOH-HPLC; injection volume: 0.45 mL; number of runs: 5, to give the products.
**Compound 1-1** (isomer 1, RT1 (min): 17.4, 13.8 mg, 22.14% yield)
LCMS (ESI): [M+H] ⁺ = 492.35
¹H NMR (400 MHz, Methanol-*d*₄) δ 7.14 - 7.11 (d, *J =* 10.1 Hz, 2H), 6.93 (s, 1H), 6.64 (s, 1H), 5.40 (s, 1H), 4.49 (broad, 1H), 4.20 - 4.04 (m, 2H), 3.89 (s, 3H), 3.66 (s, 3H), 2.97 (t, *J =* 6.5 Hz, 2H), 2.37 (s, 3H), 2.22 (s, 3H), 2.10 (s, 3H), 1.05 (s, 3H).
**Compound 1-2** (isomer 2, RT2 (min): 21.8, 13.0 mg, 22.0% yield)
LCMS (ESI): [M+H] ⁺ = 492.35
¹H NMR (400 MHz, Methanol-*d*₄) δ 7.12 (d, *J =* 10.1 Hz, 2H), 6.93 (s, 1H), 6.64 (s, 1H), 5.40 (s, 1H), 4.49 (broad, 1H), 4.20 - 4.04 (m, 2H), 3.89 (s, 3H), 3.66 (s, 3H), 2.97 (t, *J =* 6.5 Hz, 2H), 2.37 (s, 3H), 2.22 (s, 3H), 2.08 (s, 6H), 1.05 (s, 3H).

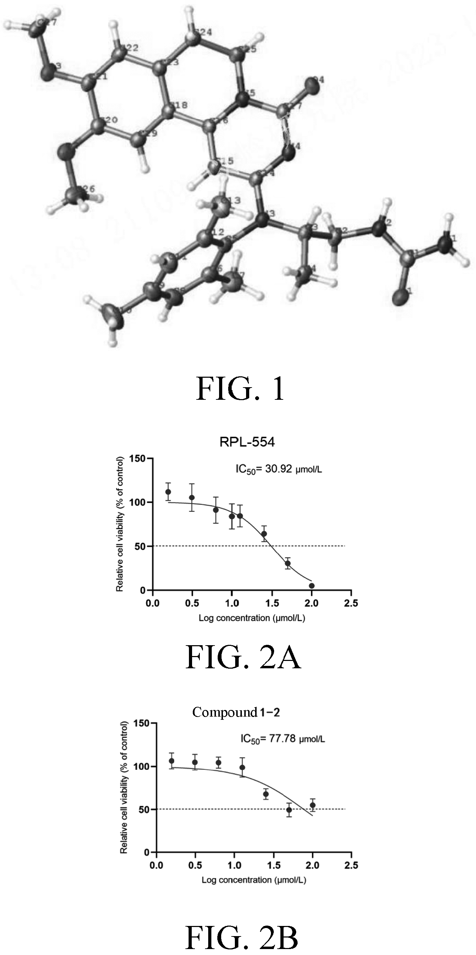

The X-ray diffraction pattern of single-crystal Compound 1-2 is shown in FIG. 1.

### Example 2, Compound 2

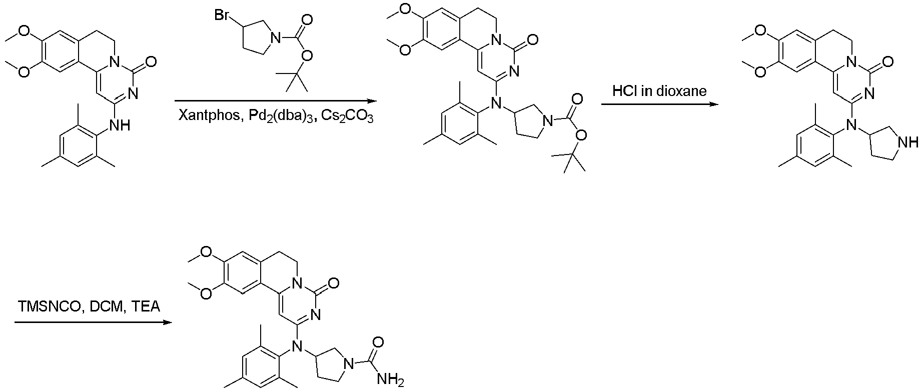

### Preparation steps:

**1.**

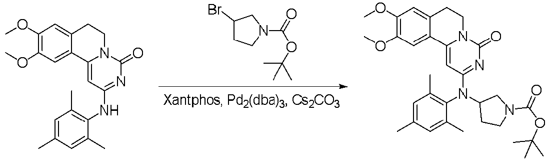

9,10-Dimethoxy-2-[(2,4,6-trimethylphenyl)amino]-6H,7H-pyrimido[4,3-a]isoquinolin-4-one (3.0 g, 7.7 mmol, 1.0 equiv) and tert-butyl 3-bromopyrrolidine-1-carboxylate (2.3 g, 9.2 mmol, 1.2 equiv) were dissolved in 1,4-dioxane (100 mL). Xantphos (0.9 g, 1.5 mmol, 0.2 equiv), cesium carbonate (5.0 g, 15.3 mmol, 2.0 equiv), and Pd₂(dba)₃ (0.7 g, 0.7 mmol, 0.1 equiv) were added thereto. The mixture was stirred at 100°C under a nitrogen atmosphere for 18 h, and then concentrated under reduced pressure. The residue was purified by reverse-phase chromatography under the following conditions: column, C18 silica gel column; mobile phase, acetonitrile, water, 0-100% gradient over 30 min; detector, UV 254 nm, to give tert-butyl 3-({9,10-dimethoxy-4-oxo-6H,7H-pyrimido[4,3-a]isoquinolin-2-yl}(2,4,6-trimethylphenyl)amino)pyrrolidine-1-carboxylate (1.4 g, 32.6% yield).

LCMS (ESI): [M+H] ⁺ = 561.3

**2.**

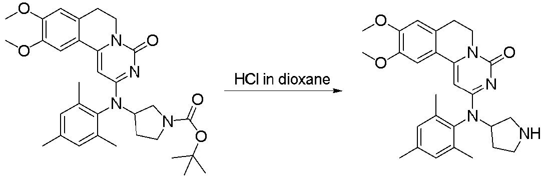

tert-Butyl 3-({9,10-dimethoxy-4-oxo-6H,7H-pyrimido[4,3-a]isoquinolin-2-yl}(2,4,6-trimethylphenyl)amino)pyrrolidine-1-carboxylate (300 mg, 0.5 mmol, 1.0 equiv) was mixed with a solution of hydrogen chloride in 1,4-dioxane (8 mL), and the resulting mixture was stirred at room temperature for 1 h. The resulting mixture was concentrated under reduced pressure to give 9,10-dimethoxy-2-[pyrrolidin-3-yl(2,4,6-trimethylphenyl)amino]-6H,7H-pyrimido[4,3-a]isoquinolin-4-one (300 mg, crude product). The crude product was used directly in the next step without further purification.

LCMS (ESI): [M+H] ⁺ = 461.2

**3.**

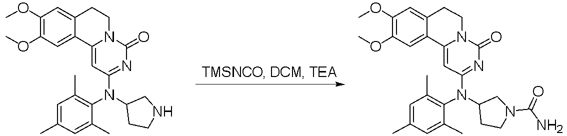

9,10-Dimethoxy-2-[pyrrolidin-3-yl(2,4,6-trimethylphenyl)amino]-6H,7H-pyrimido[4,3-a]isoquinolin-4-one (100 mg, 0.2 mmol, 1.0 equiv) was dissolved in dichloromethane (4 mL) at room temperature. Triethylamine (109 mg, 1.1 mmol, 5.0 equiv) and trimethylsilyl isocyanate (38 mg, 0.3 mmol, 1.5 equiv) were added thereto. The resulting mixture was stirred at room temperature for 2 h, and concentrated under reduced pressure. The crude product was purified by reversed-phase purification under the following conditions: column: YMC-Triart C8, 5.5 mmol/L; mobile phase A: water (10 mmol/L ammonium bicarbonate); mobile phase B: acetonitrile; flow rate: 60 mL/min; gradient: 32% to 50% B over 7 min; wavelength: 254 nm/220 nm; RT1 (min): 6.23, to give 3-({9,10-dimethoxy-4-oxo-6H,7H-pyrimido[4,3-a]isoquinolin-2-yl}(2,4,6-trimethylphenyl)amino)pyrrolidine-1-carboxamide (30.5 mg, 27.4% yield).
LCMS (ESI, m/z): [M+H] ⁺ = 504.25
¹H NMR (400 MHz, Methanol-d4) δ 7.13 (s, 2H), 6.92 (s, 1H), 6.64 (s, 1H), 5.41 (s, 1H), 5.08 - 4.99 (m, 1H), 4.19 - 4.03 (m, 3H), 3.88 (s, 3H), 3.66 (s, 3H), 3.58 - 3.52 (m, 1H), 3.29 (t, *J* = 7.3 Hz, 1H), 3.25 - 3.18 (m, 1H), 2.96 (t, *J* = 6.5 Hz, 2H), 2.38 (s, 3H), 2.29 - 2.22 (m, 1H), 2.17 - 2.15 (m, 6H), 1.77 (s, 1H).

### Example 3, Compound 3-1, and Compound 3-2

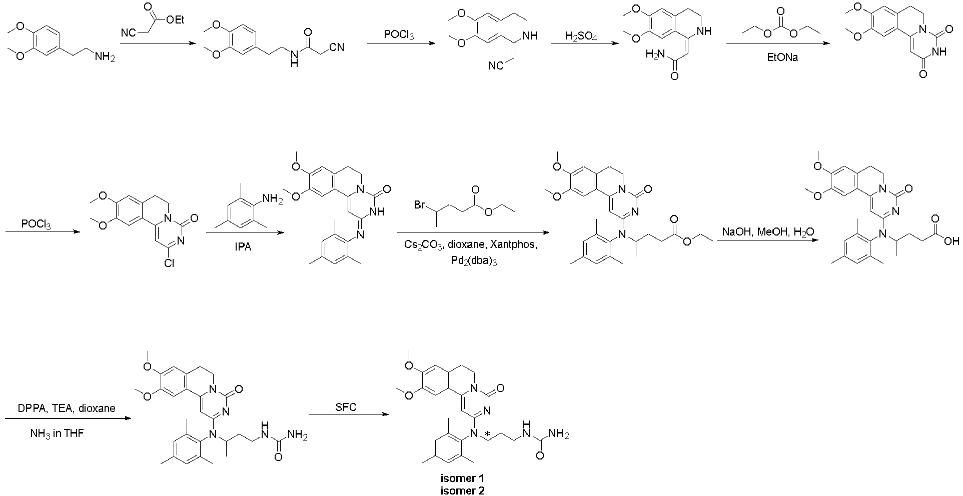

### Preparation steps:

**1.**

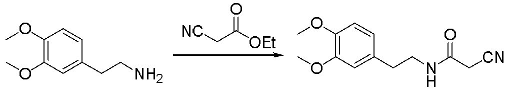

Dimethoxyphenethylamine (50.0 g, 275.8 mmol, 1.0 equiv) and ethyl cyanoacetate were sequentially added to a single-neck flask. The mixture was heated to undergo a reaction at 100°C for 16 h under a nitrogen atmosphere and then cooled to 70°C, and ethanol (80 mL) was added. The resultant was filtered. The filter cake was washed with ethanol (3 × 30 mL) and dried to remove residual ethanol, to give 2-cyano-N-[2-(3,4-dimethoxyphenyl)ethyl]acetamide (50.0 g, 73.0% yield).

LCMS (ESI, m/z): [M+H] ⁺ = 249.2

**2.**

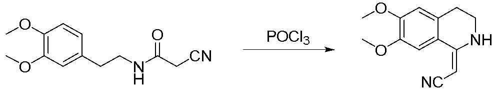

2-Cyano-N-[2-(3,4-dimethoxyphenyl)ethyl]acetamide (50.0 g, 201.3 mmol, 1.0 equiv) and phosphorus oxychloride (500 mL) were sequentially added to a single-neck flask. A reaction was allowed to proceed at 85 °C overnight under a nitrogen atmosphere. The solvent was removed by concentration, and water (300 mL) was added. The resultant was extracted with DCM (3 × 400 mL). The combined organic layers were dried over anhydrous sodium sulfate, and concentrated to give 2-[(1E)-6,7-dimethoxy-3,4-dihydro-2H-isoquinolin-1-ylidene]acetonitrile (45.0 g, 97.0% yield).

LCMS (ESI, m/z): [M+H] ⁺ = 231.2

**3.**

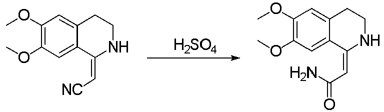

2-[(1E)-6,7-Dimethoxy-3,4-dihydro-2H-isoquinolin-1-ylidene]acetonitrile (45.0 g, 195.4 mmol, 1.0 equiv) and concentrated sulfuric acid (320 mL) were sequentially added to a single-neck flask. A reaction was allowed to proceed at room temperature for 3 h. The reaction mixture was slowly poured into ice water (1 L), and the pH was adjusted to 7 with 4 M sodium hydroxide at 0 °C. The mixture was extracted with EtOAc (3 × 600 mL), and the combined organic layers were dried over anhydrous sodium sulfate and concentrated to give 2-[(1E)-6,7-dimethoxy-3,4-dihydro-2H-isoquinolin-1-ylidene]acetamide (40.0 g, 82.4%).

LCMS (ESI, m/z): [M+H] ⁺ = 249.2

**4.**

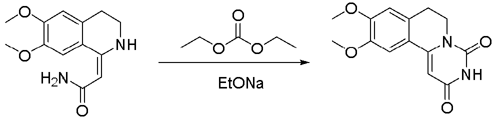

2-[(1E)-6,7-Dimethoxy-3,4-dihydro-2H-isoquinolin-1-ylidene]acetamide (40.0 g, 161.1 mmol, 1.0 equiv), ethanol (600 mL), and a solution of sodium ethoxide in ethanol (127 mL, 21%) were sequentially added to a single-neck flask. A reaction was allowed to proceed at 80°C for 0.5 h. Diethyl carbonate (57.1 g, 483.3 mmol, 3.0 equiv) was then added at the same temperature, and a reaction was allowed to proceed at 80°C overnight. After cooling to room temperature, water (500 mL) was added, and the mixture was adjusted to pH=7 with dilute hydrochloric acid. The resulting mixture was filtered. The filter cake was washed with ethanol (3 × 100 mL) and dried to remove residual ethanol, to give 9,10-dimethoxy-3H,6H,7H-pyrimido[4,3-a]isoquinolin-2,4-dione (40.0 g, 90.5%).

LCMS (ESI, m/z): [M+H] ⁺ = 275.2

**5.**

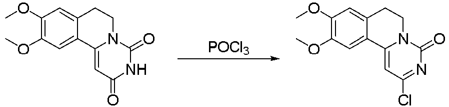

9,10-Dimethoxy-3H,6H,7H-pyrimido[4,3-a]isoquinolin-2,4-dione (40.0 g, 145.8 mmol, 1.0 equiv) and phosphorus oxychloride (240 mL) were sequentially added to a single-neck flask. A reaction was allowed to proceed at 100°C overnight under a nitrogen atmosphere. The solvent was removed by concentration, and water (500 mL) was added to dissolve the residue. The mixture was extracted with DCM (3 × 500 mL). The combined organic phases were dried over anhydrous sodium sulfate and concentrated to give 2-chloro-9,10-dimethoxy-6H,7H-pyrimido[4,3-a]isoquinolin-4-one (30.0 g, 70.2%).

LCMS (ESI, m/z): [M+H] ⁺ = 293.2

**6.**

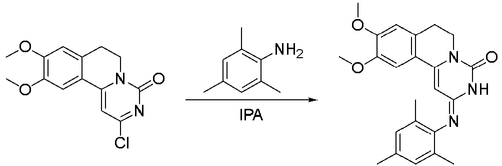

2-Chloro-9,10-dimethoxy-6H,7H-pyrimido[4,3-a]isoquinolin-4-one (2.0 g, 6.8 mmol, 1.0 equiv), isopropanol (50 mL), and trimethylaniline (4.6 g, 34.1 mmol, 5.0 equiv) were sequentially added to a single-neck flask. A reaction was allowed to proceed at 90°C overnight under a nitrogen atmosphere. The solvent was removed by concentration, and EA (20 mL) and saturated aqueous sodium bicarbonate solution (200 mL) were added. The resulting mixture was filtered. The filter cake was washed with EA (3 × 50 mL) and dried to remove residual EA, to give (2E)-9,10-dimethoxy-2-[(2,4,6-trimethylphenyl)imino]-3H,6H,7H-pyrimido[4,3-a]isoquinolin-4-one (2.0 g, 74.8%).

LCMS (ESI, m/z): [M+H] ⁺ = 392.2

**7.**

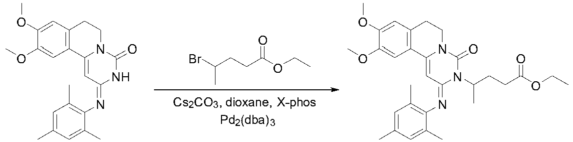

(2E)-9,10-Dimethoxy-2-[(2,4,6-trimethylphenyl)imino]-3H,6H, 7H-pyrimido[4,3-a]isoquinolin-4-one (2.0 g, 5.1 mmol, 1.0 equiv), ethyl 4-bromopentanoate (2.1 g, 10.2 mmol, 2.0 equiv), 1,4-dioxane (100 mL), cesium carbonate (5.0 g, 15.3 mmol, 3.0 equiv), Xantphos (0.6 g, 1.0 mmol, 0.2 equiv), and Pd₂(dba)₃ (0.5 g, 0.5 mmol, 0.1 equiv) were sequentially added to a single-neck flask. A reaction was allowed to proceed at 100°C for 12 h under a nitrogen atmosphere. The resultant was purified by normal-phase chromatography using DCM/MeOH (10:1) as the mobile phase to give ethyl 4-({9,10-dimethoxy-4-oxo-2-(2,4,6-trimethylphenyl)imino-6H,7H-pyrimido[4,3-a]isoquinolin-1-yl})pentanoate (500 mg, 18.8% yield).

LCMS (ESI, m/z): [M+H] ⁺ = 520.3

**8.**

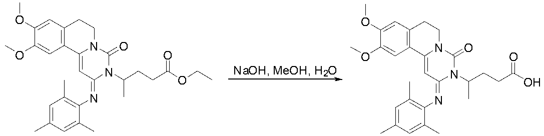

Ethyl 4-({9,10-dimethoxy-4-oxo-2-(2,4,6-trimethylphenyl)imino-6H,7H-pyrimido[4,3-a]isoquinolin-1-yl}pentanoate (500 mg, 1.0 mmol, 1.0 equiv), methanol (50 mL), and a solution of sodium hydroxide (77 mg, 2.0 mmol, 2.0 equiv) in water (10 mL) were sequentially added to a single-neck flask. A reaction was allowed to proceed at room temperature for 3 h. The solvent was removed by concentration, and water (20 mL) was added. The mixture was adjusted to pH=5 with dilute hydrochloric acid and extracted with EA (3 × 50 mL). The combined organic phases were dried over anhydrous sodium sulfate and concentrated to give 4-({9,10-dimethoxy-4-oxo-2-(2,4,6-trimethylphenyl)imino-6H,7H-pyrimido[4,3-a]isoquinolin-1-yl}pentanoic acid (450 mg, 95.1% yield).

LCMS (ESI, m/z): [M+H] ⁺ = 492.2

**9.**

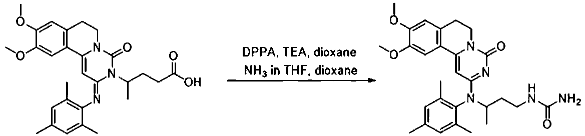

4-({9,10-Dimethoxy-4-oxo-2-(2,4,6-trimethylphenyl)imino-6H,7H-pyrimido[4,3-a]isoquinolin-1-yl}pentanoic acid (300 mg, 0.6 mmol, 1.0 equiv), 1,4-dioxane (100 mL), triethylamine (182 mg, 1.8 mmol, 3.0 equiv), and DPPA (336 mg, 1.2 mmol, 2.0 equiv) were sequentially added to a three-neck flask. A reaction was allowed to proceed at room temperature for 1 h under a nitrogen atmosphere, then heated to 100 °C for 1 h. After cooling to 80 °C, an ammonia solution in THF (1.4 M, 100 mL) was added, and a reaction was allowed to proceed at room temperature for 2 h. The solvent was removed by concentration, and the residue was purified by reversed-phase chromatography (column: X-Bridge BEH C18, 5 µm, 30 × 150 mm; mobile phase A: water (10 mmol/L NH₄HCO₃); mobile phase B: acetonitrile; flow rate: 60 mL/min; gradient: 39% B to 48% B over 9 min; wavelength: 254/220 nm; RT1 (min): 8), to give [3-({9,10-dimethoxy-4-oxo-6H,7H-pyrimido[4,3-a]isoquinolin-2-yl}(2,4,6-trimethylphenyl)amino)butyl]urea (94 mg, 30.46% yield).

LCMS (ESI, m/z): [M+H] ⁺ = 506.3

**10.**

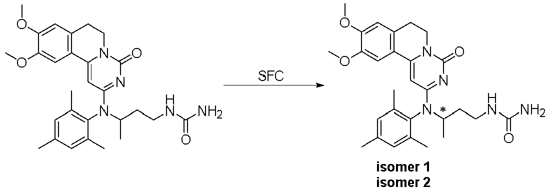

Crude [3-({9,10-dimethoxy-4-oxo-6H,7H-pyrimido[4,3-a]isoquinolin-2-yl}(2,4,6-trimethylphenyl)amino)butyl]urea (50 mg) was resolved by Chiral-HPLC (column: CHIRAL ART Cellulose-SB, 2 × 25 cm, 5 µm; mobile phase A: MtBE (0.1% DEA); mobile phase B: MeOH/DCM = 1:1-HPLC; Flow rate: 20 mL/min; gradient: isocratic; wavelength: 254 nm; RT1 (min): 5.3; RT2 (min): 7.2; Sample Solvent: MeOH/DCM = 1:1-HPLC; injection volume: 1 mL; Number Of Runs: 5) to give the products.
**Compound 3-1** (isomer 1, RT1 (min): 5.3, 17.6 mg, 34.78% yield)
LCMS (ESI, m/z): [M+H] ⁺ = 506.50
¹H NMR (400 MHz, Methanol-d4) δ 7.16 - 7.08 (m, 2H), 6.93 (s, 1H), 6.64 (s, 1H), 5.41 (s, 1H), 4.59 - 4.55 (m, 1H), 4.19 - 4.02 (m, 2H), 3.89 (s, 3H), 3.66 (s, 3H), 3.38 - 3.34 (m, 1H), 3.25 - 3.21 (m, 1H), 2.97 (t, J = 6.4 Hz, 2H), 2.38 (s, 3H), 2.34 - 2.30 (m, 1H), 2.23 (s, 3H), 2.08 (s, 3H), 1.80 - 1.76 (m, 1H), 0.99 (d, J = 6.9 Hz, 3H).
**Compound 3-2** (isomer 2, RT2 (min): 7.2, 17.8 mg, 34.78% yield)
LCMS (ESI, m/z): [M+H] ⁺ = 506.50
¹H NMR (400 MHz, Methanol-*d*₄) δ 7.16 - 7.08 (m, 2H), 6.93 (s, 1H), 6.64 (s, 1H), 5.41 (s, 1H), 4.59 - 4.45 (m, 1H), 4.19 - 4.02 (m, 2H), 3.89 (s, 3H), 3.66 (s, 3H), 3.38 - 3.34 (m, 1H), 3.25 - 3.21 (m, 1H), 2.97 (t, *J* = 6.4 Hz, 2H), 2.38 (s, 3H), 2.34 - 2.30 (m, 1H), 2.23 (s, 3H), 2.08 (s, 3H), 1.80 - 1.76 (m, 1H), 0.99 (d, *J* = 6.9 Hz, 3H).

### Example 4, Compound 4-1, and Compound 4-2

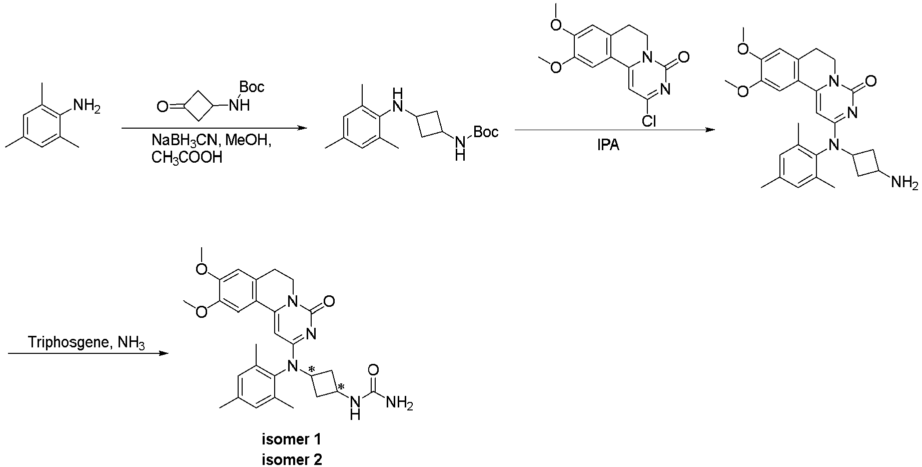

### Preparation steps:

**1.**

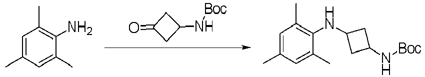

2,4,6-Trimethylaniline (3.0 g, 22.2 mmol, 1.0 equiv) was dissolved in MeOH (30 mL) at room temperature under air, followed by the addition of NaBH₃CN (1.0 g, 44.3 mmol, 2.0 equiv) and acetic acid (1 mL, 17.4 mmol, 0.7 equiv). The resulting mixture was stirred at room temperature for reaction overnight under a nitrogen atmosphere. Then the resulting mixture was concentrated under reduced pressure, and quenched with water at room temperature. The resulting mixture was extracted with ethyl acetate (2 × 100 mL). The combined organic layers were washed with saturated brine (3 × 200 mL), and dried over anhydrous sodium sulfate. The filtrate was concentrated under reduced pressure to give tert-butyl N-{3-[(2,4,6-trimethylphenyl)amino]cyclobutyl}carbamate (1.2 g, 16% yield).

LCMS (ESI): [M+H] + = 305.1

**2.**

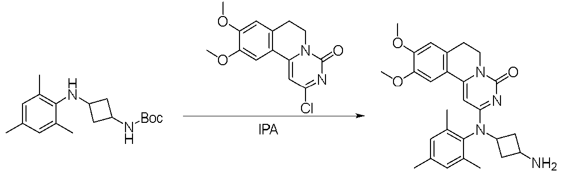

tert-Butyl N-{3-[(2,4,6-trimethylphenyl)amino]cyclobutyl}carbamate (1.2 g, 3.9 mmol, 1.0 equiv) and 2-chloro-9,10-dimethoxy-6H,7H-pyrimido[4,3-a]isoquinolin-4-one (1.3 g, 4.7 mmol, 1.2 equiv) were added to isopropanol (15 mL) at room temperature. The reaction mixture was heated to 100 °C and stirred for reaction overnight at 100 °C under a nitrogen atmosphere. Then the resulting mixture was concentrated under reduced pressure. The residue was purified by silica gel column chromatography eluting with petroleum ether/ethyl acetate (2/1) to give 2-[(3-aminocyclobutyl)(2,4,6-trimethylphenyl)amino]-9,10-dimethoxy-6H,7H-pyrimido[4,3-a]isoquinolin-4-one (150 mg, 6% yield).

LC-MS (ESI): [M+H] ⁺ = 461.2

**3.**

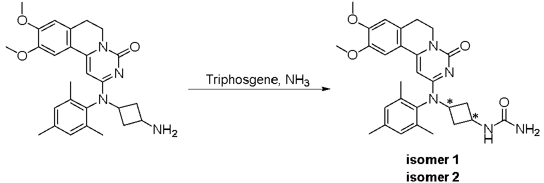

The N1-(2,4,6-Trimethylphenyl)cyclobutane-1,3-diamine (150 mg, 0.7 mmol, 1.0 equiv) was dissolved in DCM (1 mL) at room temperature under air, and triphosgene (193 mg, 0.6 mmol, 2.0 equiv) was added to the solution. The mixture was stirred at room temperature under a nitrogen atmosphere for 1 h. Ammonia in methanol (7 M, 15 mL) was then added to the above mixture at room temperature. The resulting mixture was stirred at room temperature overnight, and concentrated under reduced pressure. The residue was purified by reversed-phase flash chromatography under the following conditions: column, C18 silica gel; mobile phase, acetonitrile/water (10 mmol/L NH₄HCO₃), 30%-47% gradient over 7 min; detector, UV 254 nm. The crude product was further purified by preparative HPLC under the following conditions: column: CHIRAL ART Cellulose-SB, 2 × 25 cm, 5 µm; mobile phase A: MtBE (0.1% DEA)-HPLC; mobile phase B: MeOH: DCM = 1:1-HPLC; flow rate: 20 mL/min; gradient: isocratic; wavelength: 220 nm; RT1 (min): 6.5; RT2 (min): 9.4; Sample Solvent: injection volume: 0.7 mL; number of runs: 3, to give the products.
**Compound 4-1** (isomer 1, RT1 (min): 6.5, 16.3 mg, 9% yield)
LCMS (ESI): [M+H] ⁺ = 504.30
¹H NMR (400 MHz, Methanol-*d*₄) δ 7.20 (s, 1H), 7.05 (s, 2H), 6.93 (s, 1H), 6.11 (s, 1H), 4.56 - 4.47 (m, 1H), 4.28 - 4.15 (m, 1H), 4.03 (t, *J =* 6.2 Hz, 2H), 3.90 - 3.88 (m, 6H), 2.93 (t, *J =* 6.2 Hz, 2H), 2.78 - 2.66 (m, 2H), 2.32 (s, 3H), 2.23 (s, 6H), 1.88 - 1.73 (m, 2H).
**Compound 4-2** (isomer 2, RT2 (min): 9.4, 15.5 mg, 10% yield)
LCMS (ESI): [M+H] ⁺ = 504.40
¹H NMR (400 MHz, Methanol-*d*₄) δ 7.26 (s, 1H), 7.05 (s, 2H), 6.95 (s, 1H), 6.25 (s, 1H), 5.05 - 4.90 (m, 1H), 4.28 - 4.15 (m, 1H), 4.04 (t, *J =* 6.4 Hz, 2H), 3.92 (s, 6H), 2.95 (t, *J =* 6.4 Hz, 2H), 2.34 - 2.32 (m, 7H), 2.22 (s, 6H).

### Example 5, Compound 5-1, and Compound 5-2

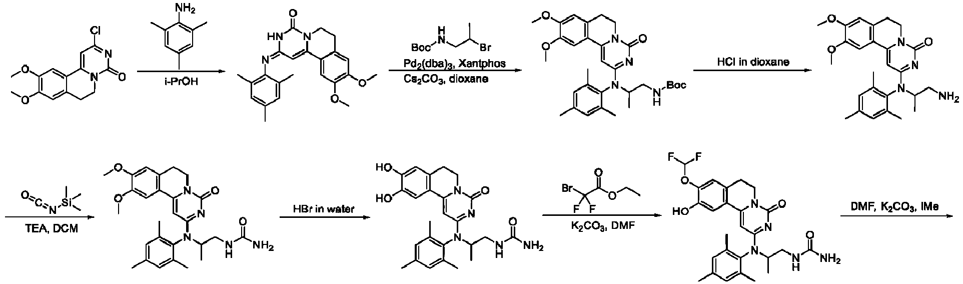

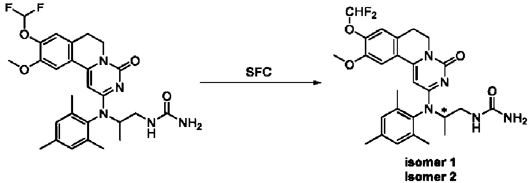

### Preparation steps:

1.

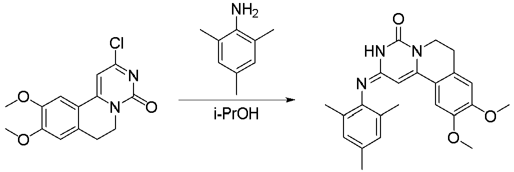

2-Chloro-9,10-dimethoxy-6h,7h-pyrimido[4,3-a]isoquinolin-4-one (200 mg, 0.7 mmol, 1.0 equiv) and 2,4,6-trimethylaniline (369 mg, 2.7 mmol, 4.0 equiv) were dissolved in isopropanol (4 mL). The mixture was stirred at 90 °C under a nitrogen atmosphere for 3 h. The resulting mixture was concentrated under reduced pressure, and purified by column chromatography with DCM/MeOH (10/1) to give (2E)-9,10-dimethoxy-2-[(2,4,6-trimethylphenyl)imino]-3H,6H,7H-pyrimido[4,3-a]isoquinolin-4-one (100 mg, 37.39% yield).

LCMS (ESI, m/z): [M+H] ⁺ = 392.1

2.

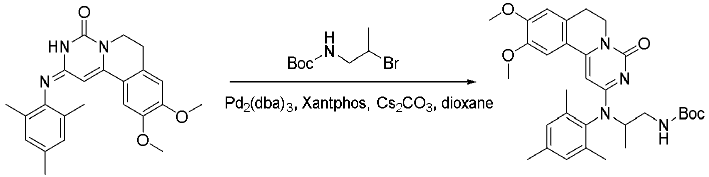

tert-Butyl N-(2-bromopropyl)carbamate (304 mg, 1.3 mmol, 1.0 equiv) and Cs₂CO₃ (1.0 g, 3.9 mmol, 3.0 equiv) were added to a solution of (2E)-9,10-dimethoxy-2-[(2,4,6-trimethylphenyl)imino]-3h,6H,7H-pyrimido[4,3-a]isoquinolin-4-one (500 mg, 1.3 mmol, 1.0 equiv) in 1,4-dioxane (10 mL), followed by addition of Pd₂(dba)₃ (117 mg, 0.1 mmol, 0.1 equiv) and Xantphos (74 mg, 0.1 mmol, 0.1 equiv) under nitrogen. The resulting mixture was stirred at 80°C under a nitrogen atmosphere for 6 h. The resulting mixture was concentrated under reduced pressure, and purified by reversed-phase column chromatography under the following conditions: column, C18 silica gel; mobile phase, water, MeCN (0.1% FA), 30%-70% gradient over 15 min; UV 254 nm, to give tert-butyl N-[2-({9,10-dimethoxy-4-oxo-6H,7H-pyrimido[4,3-a]isoquinolin-2-yl}(2,4,6-trimethylphenyl)amino)propyl]carbamate (400 mg, 57.08% yield).

LCMS (ESI, m/z): [M+H] ⁺ = 549.6

**3.**

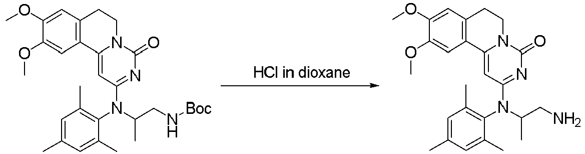

tert-Butyl N-[2-({9,10-dimethoxy-4-oxo-6H,7H-pyrimido[4,3-a]isoquinolin-2-yl}(2,4,6-trimethylphenyl)amino)propyl]carbamate (100 mg, 0.2 mmol, 1.0 equiv) and a HCl solution in 1,4-dioxane (4 M, 2 mL) were added to a 25 mL round-bottom flask. The reaction mixture was stirred at 25°C under a nitrogen atmosphere for 1 h. The resulting mixture was concentrated under reduced pressure, to give 2-[(1-aminopropan-2-yl)(2,4,6-trimethylphenyl)amino]-9,10-dimethoxy-6H,7H-pyrimido[4,3-a]isoquinolin-4-one (80 mg, crude product).

LCMS (ESI, m/z): [M+H] ⁺ = 449.2

**4.**

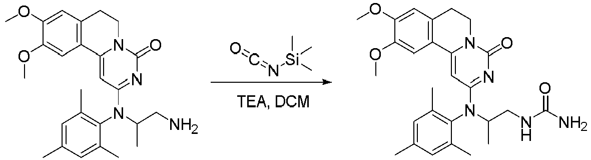

2-[(1-Aminopropan-2-yl)(2,4,6-trimethylphenyl)amino]-9,10-dimethoxy-6h,7h-pyrimido[4,3-a]isoquinolin-4-one (100 mg, 0.2 mmol, 1.0 equiv) and trimethylsilyl isocyanide (38 mg, 0.3 mmol, 1.5 equiv) were dissolved in DCM (5 mL) under stirring. TEA (60 mg, 0.6 mmol, 3.0 equiv) was added dropwise at 25°C under nitrogen. The reaction mixture was stirred at 25°C under a nitrogen atmosphere for 2 h. The mixture was then concentrated under reduced pressure, and purified by column chromatography under the following conditions: column, C18 silica gel; mobile phase, aqueous ACN (0.1% NH₃.H₂O), 25%-75% gradient over 20 min; UV 254 nm, to give 2-({9,10-dimethoxy-4-oxo-6H,7H-pyrimido[4,3-a]isoquinolin-2-yl}(2,4,6-trimethylphenyl)amino)propylurea (80 mg, 73.0% yield).

LCMS (ESI, m/z): [M+H] ⁺ = 492.2

**5.**

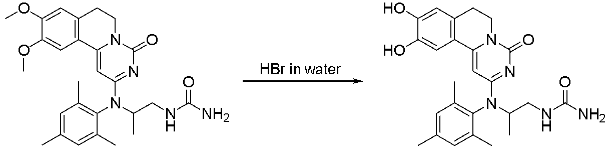

2-({9,10-Dimethoxy-4-oxo-6H,7H-pyrimido[4,3-a]isoquinolin-2-yl}(2,4,6-trimethylphenyl)amino)propylurea (100 mg, 0.2 mmol, 1.0 equiv) and aqueous hydrobromic acid (5 mL) were added to a 50 mL round-bottom flask. The resulting mixture was stirred at 120 °C under a nitrogen atmosphere for 3 h. The mixture was extracted with ethyl acetate (2 × 50 mL). The organic layer was washed with saturated brine (3 × 100 mL), dried over anhydrous sodium sulfate, filtered, and concentrated under reduced pressure, to give 2-({9,10-dihydroxy-4-oxo-6H,7H-pyrimido[4,3-a]isoquinolin-2-yl}(2,4,6-trimethylphenyl)amino)propylurea (80 mg, crude product).

LCMS (ESI, m/z): [M+H] ⁺ = 464.2

**6.**

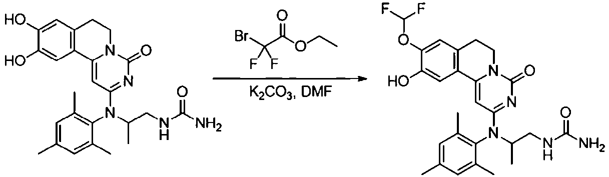

2-({9,10-Dihydroxy-4-oxo-6H,7H-pyrimido[4,3-a]isoquinolin-2-yl}(2,4,6-trimethylphenyl)amino)propylurea (100 mg, 0.2 mmol, 1.0 equiv) and K₂CO₃ (149 mg, 1.0 mmol, 5.0 equiv) were added to DMF (1.5 mL), followed by dropwise addition of ethyl 2-bromo-2,2-difluoroacetate (44 mg, 0.2 mmol, 1.0 equiv) at room temperature. The reaction mixture was stirred at 55°C under a nitrogen atmosphere for 1.5 h. The resulting mixture was concentrated under reduced pressure, and purified by reversed-phase column chromatography under the following conditions: column, C18 silica gel; mobile phase, water, ACN (0.1% FA), 20%-70% gradient over 20 min; UV, 254 nm, to give 2-{[9-(difluoromethoxy)-10-hydroxy-4-oxo-6H,7H-pyrimido[4,3-a]isoquinolin-2-yl](2,4,6-trimethylphenyl)amino}propylurea (10 mg, 9.03% yield).

LCMS (ESI, m/z): [M+H] ⁺ = 514.2

**7.**

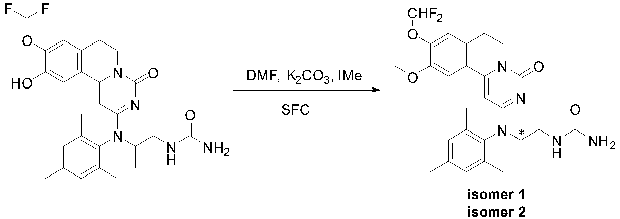

2-{[9-(difluoromethoxy)-10-hydroxy-4-oxo-6H,7H-pyrimido[4,3-a]isoquinolin-2-yl](2,4,6-trimethylphenyl)amino}propylurea (10 mg, 0.02 mmol, 1.0 equivalent) and K₂CO₃ (4 mg, 0.03 mmol, 1.5 equivalent) were added to the solvent DMF (0.5 mL), and iodomethane (4 mg, 0.03 mmol, 1.5 equivalent) was added dropwise at room temperature. The resulting mixture was stirred at 60°C under a nitrogen atmosphere for 1.5 h. Then the resulting mixture was concentrated under reduced pressure, and purified by HPLC under the following conditions: column: XBridge BEH C18, 5 µm, 30 × 150 mm; mobile phase A: water (10 mmol/L NH₄HCO₃); mobile phase B: ACN; flow rate: 60 mL/min; gradient: 40% B to 50% B over 8 min; wavelength: 254/220 nm; RT1 (min): 7.3, to give a racemic product. The racemic product was resolved by chiral HPLC under the following conditions: column, chiral ART Cellulose-SB, 2*25 cm, 5 µm; mobile phase A: MeOH: DCM = 1:1-HPLC, mobile phase B: MtBE (0.1% DEA)-HPLC; flow rate: 20 ml/min; gradient: isocratic 50; wavelength: 220 nm; RT1 (min): 4.4; RT2 (min): 10.7; sample solvent: MeOH-HPLC; injection volume: 1 ml, to give the products.
**Compound 5-1** (isomer 1, RT1 (min): 4.4, 1.54 mg, 14.87% yield)
LCMS (ESI, m/z): [M+H] ⁺ = 529.40.
¹H NMR (400 MHz, Methanol-d4) δ 7.20 - 7.05 (m, 3H), 6.95 - 6.82 (m, 1H), 6.80 (s, 1H), 5.49 (s, 1H), 4.49 (s, 1H), 4.16 -4.10 (m, 2H), 3.72 (s, 3H), 2.97 (t,*J* = 6.4 Hz, 2H), 2.37 (s, 3H), 2.23 (s, 3H), 2.10 (s, 3H), 1.35 - 1.28 (m, 2H), 1.06 (s, 3H).
**Compound 5-2** (isomer 2, RT2 (min): 10.7, 2.19 mg, 20.9% yield)
LCMS (ESI, m/z): [M+H] ⁺ = 529.45
¹H NMR (400 MHz, Methanol-d4) δ 7.20 - 7.05 (m, 3H), 6.95 - 6.82 (m, 1H), 6.80 (s, 1H), 5.49 (s, 1H), 4.49 (s, 1H), 4.16 - 4.10 (m, 2H), 3.72 (s, 3H), 2.96 (t, *J* = 6.4 Hz, 2H), 2.37 (s, 3H), 2.22 (s, 3H), 2.11 (s, 3H), 1.35 - 1.28 (m, 2H), 1.06 (s, 3H).

### Example 6, Compound 6

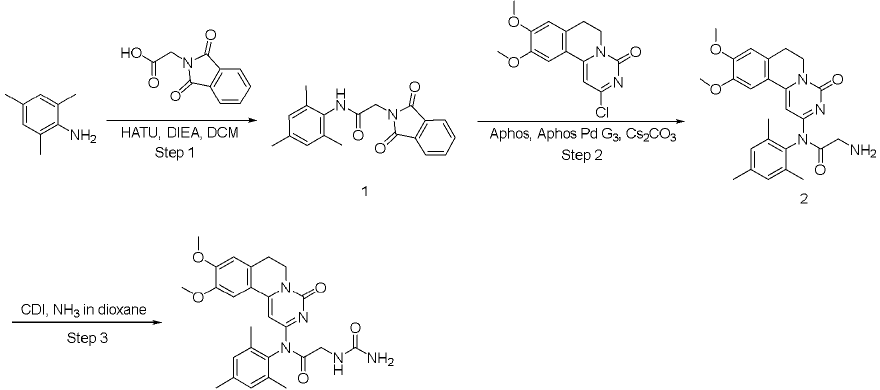

### Preparation steps:

**1.**

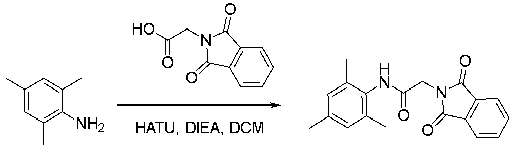

Trimethylaniline (1.0 g, 7.4 mmol, 1.0 equiv), N,N,N',N'-tetramethyl-O-(7-azabenzotriazol-1-yl)uronium hexafluorophosphate (3.4 g, 8.8 mmol, 1.2 equiv), and N,N-diisopropylethylamine (3.9 mL, 22.2 mmol, 3.0 equiv) were dissolved in dichloromethane (20 mL), and the resulting mixture was stirred at room temperature for 2 h. The mixture was concentrated under reduced pressure, and dissolved in 10 mL water. The resulting mixture was extracted with ethyl acetate (3 × 10 mL). The organic layers were washed with 20 mL of saturated aqueous sodium chloride solution, dried over anhydrous sodium sulfate, filtered, and concentrated under reduced pressure. The crude product was purified by normal-phase column chromatography under the following conditions: mobile phase A: petroleum ether; mobile phase B: ethyl acetate; flow rate: 60 mL/min; gradient: 10% B to 50% B over 20 min; wavelength: 254 nm, to give 2-(1,3-dioxo-isoindolin-2-yl)-N-(2,4,6-trimethylphenyl)acetamide (600 mg, 25.17% yield).

LCMS (ESI): [M+H] ⁺ = 323.14

**2.**

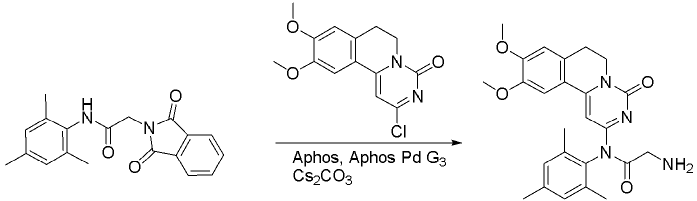

2-(1,3-Dioxoisoindolin-2-yl)-N-(2,4,6-trimethylphenyl)acetamide (600 mg, 1.8 mmol, 1.0 equiv), 2-chloro-9,10-dimethoxy-6,7-dihydro-4H-pyrimido[6,1-a]isoquinolin-4-one (653 mg, 2.2 mmol, 1.2 equiv), diphenyl (1-acetoxy-2,2,2-trichloroethyl)phosphonate (118.40 mg, 0.186 mmol, 0.1 equiv), ([4-(N,N-dimethylamino)phenyl]di-tert-butylphosphino)(2'-amino-1,1'-biphenyl-2-yl)palladium(II) methanesulfonate (49.39 mg, 0.186 mmol, 0.1 equiv), and cesium carbonate (1.5 g, 4.6 mmol, 2.5 equiv) were dissolved in 1,4-dioxane (10 mL), and the mixture was stirred at 110°C overnight under a nitrogen atmosphere. The mixture was concentrated under reduced pressure, dissolved in 10 mL water, and extracted with ethyl acetate (3 × 20 mL). The organic layers were washed with 20 mL of saturated aqueous sodium chloride solution, dried over anhydrous sodium sulfate, filtered, and concentrated under reduced pressure. The crude product was purified by reverse-phase column chromatography under the following conditions: column: C18; mobile phase A: water (10 mmol/L ammonium bicarbonate); mobile phase B: acetonitrile; flow rate: 60 mL/min; gradient: 30% B to 50% B over 20 min; wavelength: 254 nm, to give 2-amino-N-(9,10-dimethoxy-4-oxo-6,7-dihydro-4H-pyrimido[6,1-a]isoquinolin-2-yl)-N-(2,4,6-trimethylphenyl)acetamide (50 mg, 5.04% yield).

LCMS (ESI): [M+H] ⁺ = 449.16

**3.**

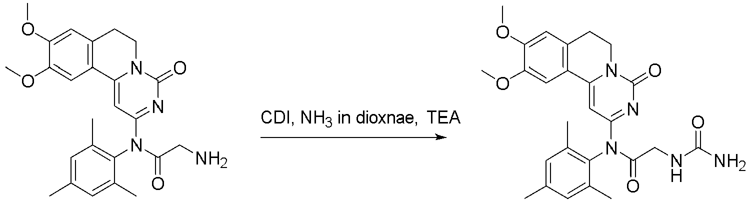

2-Amino-N-(9,10-dimethoxy-4-oxo-6,7-dihydro-4H-pyrimido[6,1-a]isoquinolin-2-yl)-N-(2,4,6-trimethylphenyl)acetamide (20 mg, 0.05 mmol, 1.0 equiv), triethylamine (14 mg, 0.15 mmol, 3.0 equiv), and N,N'-carbonyldiimidazole (10.9 mg, 0.07 mmol, 1.5 equiv) were dissolved in dichloromethane (2 mL). Under a nitrogen atmosphere, a solution of ammonia in 1,4-dioxane (2 mL) was added dropwise at 0°C, followed by stirring at room temperature for 2 h. The crude product was purified by reversed-phase column chromatography using the following conditions: column, SunFire C18, 5 m, 30 mm × 150 mm; mobile phase A, water (0.1% formic acid); mobile phase B: acetonitrile; flow rate: 60 mL/min; gradient: 45% B to 59% B over 7 min; wavelength: 254/220 nm; time: 5.92, to give N-(9,10-dimethoxy-4-oxo-6,7-dihydro-4H-pyrimido[6,1-a]isoquinolin-2-yl)-N-(2,4,6-trimethylphenyl)-2-ureidoacetamide (2.9 mg, 13.10% yield).
LCMS (ESI, m/z): [M+H] ⁺ = 475.20
¹HNMR (400 MHz, DMSO-d6) δ 8.45 (s, 1H), 7.71 (s, 1H), 7.18 (s, 1H), 7.08 - 7.01 (m, 3H), 4.77 (s, 2H), 4.08 (t, J = 6.6 Hz, 2H), 3.86 (s, 3H), 3.79 (s, 3H), 2.97 (t, J = 6.6 Hz, 2H), 2.30 (s, 3H), 2.12 - 2.10 (s, 6H).

### Example 7, Compound 7-1, Compound 7-2, and Compound 7-3

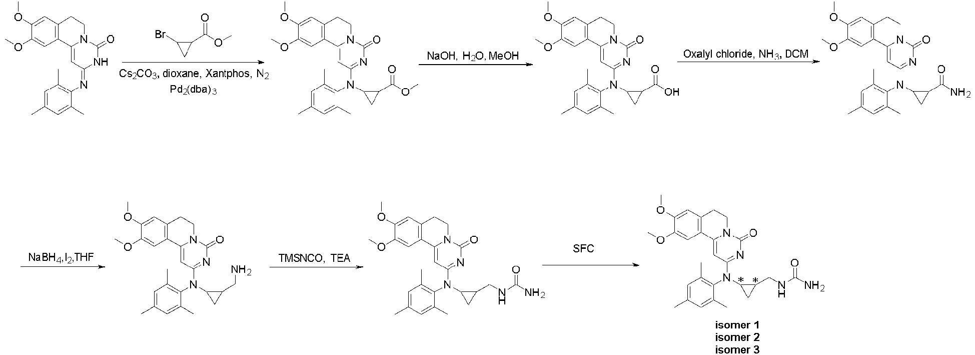

### Preparation steps:

**1.**

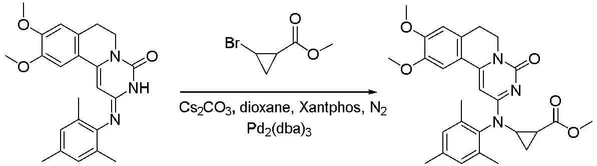

To a solution of methyl 2-bromocyclopropane-1-carboxylate (200 mg, 1.1 mmol, 1.0 equiv) and (2E)-9,10-dimethoxy-2-[(2,4,6-trimethylphenyl)imino]-3H,6H,7H-pyrimido[4,3-a]isoquinolin-4-one (437 mg, 1.1 mmol, 1.0 equiv) in 1,4-dioxane (80 mL), cesium carbonate (1.1 g, 3.4 mmol, 3.0 equiv), Xantphos (106 mg, 0.2 mmol, 0.2 equiv), and Pd₂(dba)₃ (64 mg, 0.1 mmol, 0.1 equiv) were added. The mixture was stirred at 100 °C under a nitrogen atmosphere for 12 h and then concentrated under reduced pressure. The residue was purified by silica gel column chromatography, eluting with methanol/dichloromethane (1:10), to give a crude product. The crude product was further purified by chromatography using the following conditions: column: YMC Triart C18 ExRs, 5 µm, 30 mm × 150 mm; mobile phase A: water (10 mmol/L ammonium bicarbonate); mobile phase B: acetonitrile; flow rate: 60 mL/min; gradient: 39% B to 62% B over 10 min; wavelength: 254/220 nm; RT1 (min): 8.33 min, to give methyl 2-({9,10-dimethoxy-4-oxo-6H,7H-pyrimido[4,3-a]isoquinolin-2-yl}(2,4,6-trimethylphenyl)amino)cyclopropane-1-carboxylate (100 mg, 18.3% yield).

LCMS (ESI): [M+H] ⁺ = 490.2

**2.**

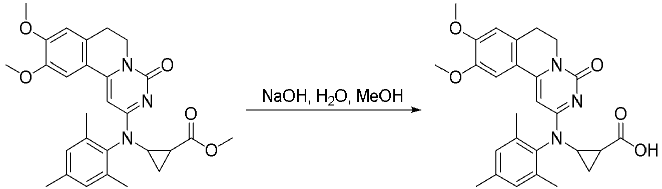

At room temperature, a solution of sodium hydroxide (65 mg, 1.6 mmol, 5.0 equiv) in water (5 mL) was added to a solution of methyl 2-({9,10-dimethoxy-4-oxo-6H,7H-pyrimido[4,3-a]isoquinolin-2-yl}(2,4,6-trimethylphenyl)amino)cyclopropane-1-carboxylate (160 mg, 0.3 mmol, 1.0 equiv) in methanol (20 mL). The mixture was stirred at 50°C for 18 h. After completion of the reaction, the reaction mixture was concentrated under reduced pressure. The resulting mixture was extracted with ethyl acetate (3 × 50 mL). The aqueous layer was acidified to pH=4 with 6 M aqueous hydrochloric acid. The resulting mixture was extracted with dichloromethane (3 × 100 mL). The combined organic layers were washed with brine, dried over anhydrous sodium sulfate, filtered, and concentrated under reduced pressure to give 2-({9,10-dimethoxy-4-oxo-6H,7H-pyrimido[4,3-a]isoquinolin-2-yl}(2,4,6-trimethylphenyl)amino)cyclopropane-1-carboxylic acid (170 mg, crude product).

LCMS (ESI): [M+H] ⁺ = 476.2

**3.**

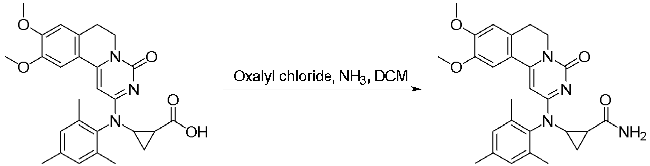

Oxalyl chloride (0.1 mL, 1.1 mmol, 3.0 equiv) was added dropwise to a solution of 2-({9,10-dimethoxy-4-oxo-6H,7H-pyrimido[4,3-a]isoquinolin-2-yl}(2,4,6-trimethylphenyl)amino)cyclopropane-1-carboxylic acid (170 mg, 0.4 mmol, 1.0 equiv) in dichloromethane (10 mL). The resulting mixture was stirred at 0 °C for 1 h under a nitrogen atmosphere. Then the resulting mixture was concentrated under reduced pressure. The resulting crude was used directly without further purification. The crude product was dissolved in dichloromethane (1 mL), and added to a solution of ammonia in tetrahydrofuran (60 mL, 1.3 M) at 0 °C. The mixture was stirred at 0 °C for 1 h, and extracted with ethyl acetate (3 × 60 mL). The combined organic layers were washed with brine, dried over anhydrous sodium sulfate, filtered, and concentrated under reduced pressure to give 2-({9,10-dimethoxy-4-oxo-6H,7H-pyrimido[4,3-a]isoquinolin-2-yl}(2,4,6-trimethylphenyl)amino)cyclopropane-1-carboxamide (180 mg, crude product),

LCMS (ESI): [M+H] ⁺ = 475.2

**4.**

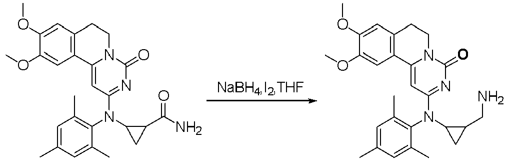

A mixture of 2-({9,10-dimethoxy-4-oxo-6H,7H-pyrimido[4,3-a]isoquinolin-2-yl}(2,4,6-trimethylphenyl)amino)cyclopropane-1-carboxamide (180 mg, 0.4 mmol, 1.0 equiv) in tetrahydrofuran (20 mL) was cooled to 0 °C, and sodium borohydride (143 mg, 3.8 mmol, 10.0 equiv) was added batchwise. Iodine powder (963 mg, 3.8 mmol, 10.0 equiv) was dissolved in tetrahydrofuran (3 mL) and then added to the above mixture. The resulting mixture was stirred at 70 °C for 18 h and then quenched with water (30 mL) at room temperature. The resulting mixture was extracted with ethyl acetate (2 × 50 mL). The combined organic layers were washed with brine, dried over anhydrous sodium sulfate, filtered, and concentrated under reduced pressure. The residue was purified by Prep-TLC (dichloromethane/methanol = 10/1) to give 2-{[2-(aminomethyl)cyclopropyl](2,4,6-trimethylphenyl)amino}-9,10-dimethoxy-6H,7H-pyrimido[4,3-a]isoquinolin-4-one (45 mg, 25.8% yield).

LCMS (ESI): [M+H] ⁺ = 461.2

**5.**

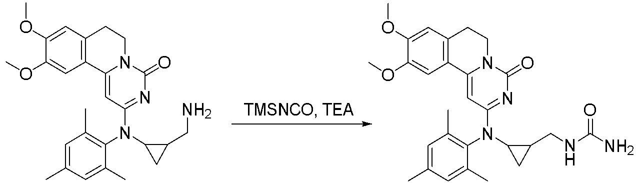

A mixture of 2- 1[2-(aminomethyl)cyclopropyl](2,4,6-trimethylphenyl)amino}-9,10-dimethoxy-6H,7H-pyrimido[4,3-a]isoquinolin-4-one (45 mg, 0.1 mmol, 1.0 equiv), triethylamine (30 mg, 0.3 mmol, 3.0 equiv), and trimethylsilyl isocyanate (23 mg, 0.2 mmol, 2.0 equiv) in DCM (5 mL) was stirred at room temperature for 3 h. The mixture was concentrated under reduced pressure. The crude product (45 mg) was purified by Prep-HPLC under the following conditions: column: XBridge BEH C18 OBD Prep Column 130, 5 m, 19 mm × 250 mm; mobile phase A: water (10 mM ammonium bicarbonate); flow rate: 25 mL/min; gradient: 45% B to 67% B over 10 min; wavelength: 254/220 nm; RT1 (min): 7.93, to give [2-({9,10-dimethoxy-4-oxo-6H,7H-pyrimido[4,3-a]isoquinolin-2-yl}(2,4,6-trimethylphenyl)amino)cyclopropyl]methylurea (20 mg, 40.65% yield).

LCMS (ESI): [M+H] ⁺ = 504.3

**6.**

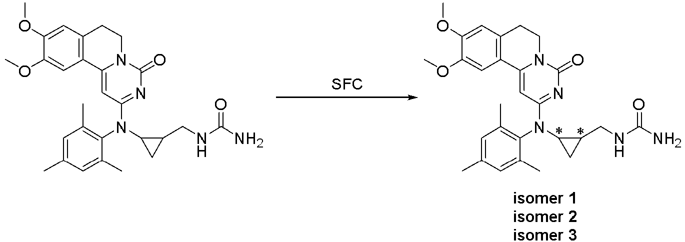

**Compound 7-1,** isomer 1 (column: CHIRAL ART Amylose-SA, 2 × 25 cm, 5 µm; mobile phase A: methanol: dichloromethane = 1:1; mobile phase B: methyl tert-butyl ether (0.1% diethylamine); flow rate: 20 mL/min; gradient: isocratic; wavelength: 254 nm; RT1 (min): 6.7; sample solvent: methanol (0.1% diethylamine); injection volume: 1 mL; number of runs: 3) was obtained as the product (3.8 mg, 12.43% yield).
LCMS (ESI): [M+H] ⁺ = 504.30
¹H NMR (400 MHz, Methanol-d4) δ 7.10 (s, 1H), 7.07 (s, 1H), 6.95 (s, 1H), 6.72 (s, 1H), 5.54 (s, 1H), 4.25 - 4.06 (m, 2H), 3.90 (s, 4H), 3.68 (s, 3H), 2.99 (t, *J* = 6.6 Hz, 3H), 2.48 - 2.40 (m, 1H), 2.36 (s, 3H), 2.23 (s, 3H), 2.03 (s, 3H), 1.43 - 1.31 (m, 2H).

**Compound 7-2,** isomer 2 (column: CHIRAL ART Amylose-SA, 2 × 25 cm, 5 µm; mobile phase A: methanol: dichloromethane = 1:1; mobile phase B: methyl tert-butyl ether (0.1% diethylamine); flow rate: 20 mL/min; gradient: isocratic; wavelength: 254 nm; RT2 (min): 10.9; sample solvent: methanol (0.1% diethylamine); injection volume: 1 mL; number of runs: 3) was obtained as the product (3.5 mg, 11.29% yield).
LCMS (ESI): [M+H] ⁺ = 504.35
¹H NMR (400 MHz, Methanol-*d4*) δ 7.10 (s, 1H), 7.07 (s, 1H), 6.95 (s, 1H), 6.72 (s, 1H), 5.54 (s, 1H), 4.25 - 4.06 (m, 2H), 3.90 (s, 4H), 3.68 (s, 3H), 2.99 (t, *J* = 6.6 Hz, 3H), 2.48 - 2.40 (m, 1H), 2.36 (s, 3H), 2.23 (s, 3H), 2.03 (s, 3H), 1.43 - 1.31 (m, 2H).

**Compound 7-3,** isomer 3 (column: XBridge BEH C18 OBD Prep Column 130, 5 m, 19 mm × 250 mm; mobile phase A: water (10 mmol/L ammonium bicarbonate); mobile phase B: acetonitrile; flow rate: 60 mL/min; gradient: 39% B to 61% B over 9 min; wavelength: 254 nm/220 nm; RT1 (min): 5.45) was obtained as the product (20 mg, 40.6% yield).
LCMS (ESI): [M+H] ⁺ = 504.35
¹H NMR (400 MHz, Methanol-*d4*) δ 7.10 (s, 1H), 7.07 (s, 1H), 7.02 - 6.89 (m, 2H), 6.70 (s, 1H), 5.57 (s, 1H), 4.61 (s, 6H), 4.27 - 4.04 (m, 2H), 4.05 - 3.91 (m, 2H), 3.90 (s, 3H), 3.85 - 3.72 (m, 1H), 3.68 (s, 3H), 3.05 - 2.95 (m, 2H), 2.92 - 2.87 (m, 1H), 2.36 (s, 3H), 2.31 - 2.14 (m, 8H).

### Example 8, Compound 8

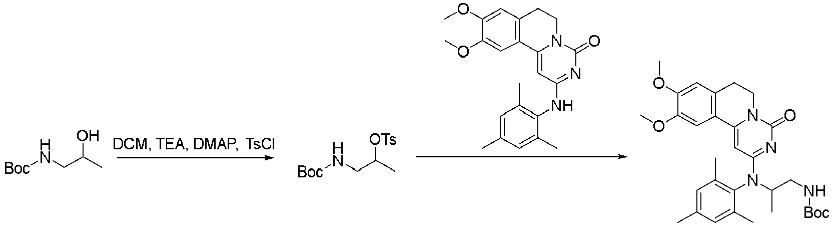

### Preparation steps

**1.**

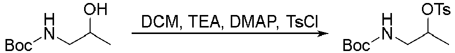

tert-Butyl N-(2-hydroxypropyl)carbamate (15.0 g, 85.6 mmol, 1.0 equiv) and TEA (26.0 g, 256.8 mmol, 1.5 equiv) were mixed in DCM (80 mL) and stirred. Then DMAP (1.1 g, 8.6 mmol, 0.1 equiv) and TsCl (21.2 g, 111.3 mmol, 1.3 equiv) were added batchwise at 0 °C. The resulting mixture was stirred at room temperature for 2 h. The resulting mixture was extracted with dichloromethane (3 × 200 mL). The organic layers were washed with brine, dried over anhydrous sodium sulfate, filtered, concentrated under reduced pressure, and purified by silica gel column chromatography, eluting with PE/EA (5/1), to give tert-butyl N-[2-[(4-methylbenzenesulfonyl)oxy]propyl]carbamate (20.3 g, 71.9% yield).

LC-MS (ESI): [M+H] ⁺ = 330.1

**2.**

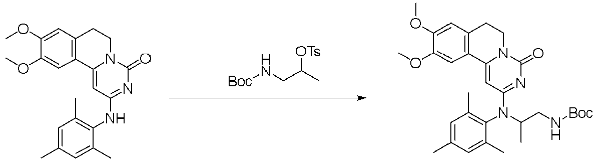

tert-Butyl N-[2-[(4-methylbenzenesulfonyl)oxy]propyl]carbamate (2.0 g, 6.1 mmol, 1.5 equiv), cesium carbonate (2.7 g, 8.2 mmol, 2.0 equiv), 4,5-bis(diphenylphosphino)-9,9-dimethylxanthene (0.5 g, 0.8 mmol, 0.2 equiv) and tris(dibenzylideneacetone)dipalladium (0.4 g, 0.4 mmol, 0.1 equiv) were added to a solution of 9,10-dimethoxy-2-[(2,4,6-trimethylphenyl)amino]-6H,7H-pyrimido[4,3-a]isoquinolin-4-one (1.6 g, 4.1 mmol, 1.0 equiv) in 1,4-dioxane (20 mL). The mixture was purged with nitrogen and heated to 100 °C, and stirred at 100°C for 5 h under a nitrogen atmosphere. The reaction system was extracted with ethyl acetate (3× 200 mL) and water (150 mL). The organic phase was dried over anhydrous sodium sulfate, filtered, and concentrated on a rotary evaporator. The residue was purified by reversed-phase HPLC under the following conditions: column: XBridge BEH C18, 5 µm, 30 × 150 mm; mobile phase A: water (10 mmol/L ammonium bicarbonate); mobile phase B: acetonitrile; flow rate, 60 mL/min; gradient: 55% B to 65% B over 7 min; wavelength: 254/220 nm; RT1 (min): 5.67/6.05, to give tert-butyl N-[2-({9,10-dimethoxy-4-oxo-6H,7H-pyrimido[4,3-a]isoquinolin-2-yl}(2,4,6-trimethylphenyl)amino)propyl]carbamate (35.33 mg, 1.55% yield).
LC-MS (ESI): [M+H] ⁺ = 549.35
¹H NMR (400 MHz, Methanol-*d*₄) δ 7.11 (d, *J =* 10.2 Hz, 2H), 6.93 (s, 1H), 6.64 (s, 1H), 5.41 (s, 1H), 4.57 - 4.39 (m, 1H), 4.18 - 4.02 (m, 2H), 3.89 (s, 3H), 3.66 (s, 3H), 3.55 - 3.48 (m, 1H), 3.40 (t, *J* = 8.2 Hz, 1H), 2.97 (t, *J* = 6.5 Hz, 2H), 2.37 (s, 3H), 2.22 (s, 3H), 2.11 (s, 3H), 1.46 (s, 9H), 1.31 (s, 1H), 1.08 (d, *J =* 6.9 Hz, 3H).

### Example 9, Compound 9-1, and Compound 9-2

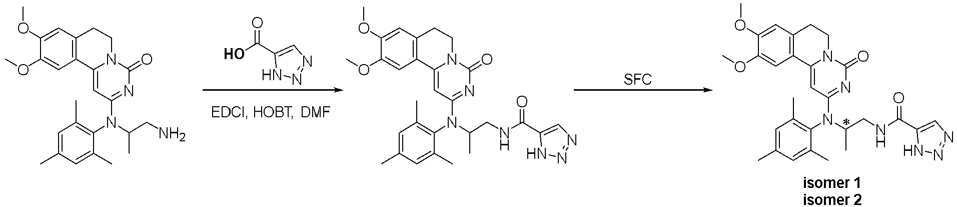

### Preparation steps:

**1.**

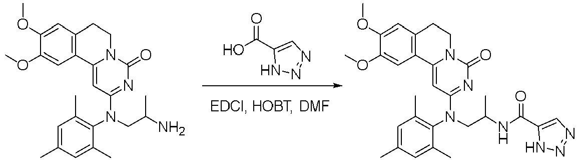

2-((2-Aminopropyl)(2,4,6-trimethylphenyl)amino)-9,10-dimethoxy-6,7-dihydro-4H-pyrimido[6,1-a]isoquinolin-4-one (150 mg, 0.33 mmol, 1.0 equiv), 3H-1,2,3-triazole-4-carboxylic acid (56 mg, 0.5 mmol, 1.5 equiv), 1-(3-dimethylaminopropyl)-3-ethylcarbodiimide (96 mg, 0.5 mmol, 1.5 equiv), and 1-hydroxybenzotriazole (67 mg, 0.5 mmol, 1.5 equiv) were dissolved in DMF (2 mL). The resulting mixture was stirred at room temperature for 1 h under a nitrogen atmosphere, and then concentrated under reduced pressure. The crude product was purified by reversed-phase column chromatography under the following conditions: column: Xselect CSH C185 m, 30 mm × 150 mm; mobile phase A: water (0.1% FA); mobile phase B: acetonitrile; flow rate: 60 mL/min; gradient: 27% B to 55% B in 9 min; wavelength: 254/220 nm; RT1 (min): 6.77/8.43, to give N-(2-((9,10-dimethoxy-4-oxo-6,7-dihydro-4H-pyrimido[6,1-a]isoquinolin-2-yl)(2,4,6-trimethylphenyl)amino)propyl)-1H-1,2,3-triazole-5-carboxamide (30 mg, 16.5% yield).

LCMS (ESI): [M+H] ⁺ = 544.25

**2.**

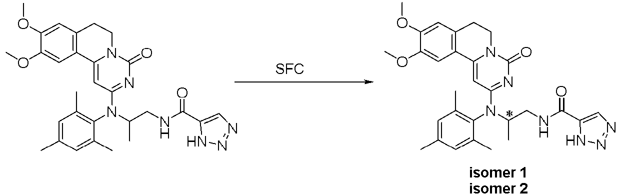

The racemate (30 mg) was purified by Chiral-SFC under the following conditions: column: CHIRALPAK IH, 3 × 25 cm, 5 µm; mobile phase A: CO₂; mobile phase B: methanol (0.1% 2M NH₃-MEOH); flow rate: 85 mL/min; gradient: isocratic 35%; column temperature (°C): 35; pressure (bar): 100; wavelength: 220 nm; RT1 (min): 2.92; RT2 (min): 4.42; sample solvent: DCM (0.1% 2M NH₃-MeOH)-HPLC; injection volume: 1 mL, to obtain the products.
**Compound 9-1** (isomer 1, RT1 (min): 2.92, 9.2 mg, 28.58% yield)
LCMS (ESI): [M+H] ⁺ = 544.25
¹H NMR (400 MHz, Methanol-*d*₄) δ 8.25 (s, 1H), 7.15 - 7.11 (m, 2H), 6.99 - 6.92 (m, 1H), 6.65 (s, 1H), 5.44 (s, 1H), 4.74 (s, 1H), 4.23 - 4.04 (m, 2H), 3.95 - 3.88 (m, 4H), 3.85 - 3.75 (m, 1H), 3.67 (s, 3H), 2.99 (t, *J* = 6.4 Hz, 2H), 2.38 (s, 3H), 2.27 (s, 3H), 2.13 (s, 3H), 1.31 (d, *J* = 4.0 Hz, 1H), 1.11 (d, *J* = 6.8 Hz, 3H).
**Compound 9-2** (isomer 2, RT2 (min): 4.42, 8.8 mg, 24.5% yield)
LCMS (ESI): [M+H] ⁺ = 544.25
¹H NMR (400 MHz, Methanol-*d*₄) δ 8.25 (s, 1H), 7.15 - 7.11 (m, 2H), 6.99 - 6.92 (m, 1H), 6.65 (s, 1H), 5.44 (s, 1H), 4.74 (s, 1H), 4.23 - 4.04 (m, 2H), 3.95 - 3.88 (m, 4H), 3.85 - 3.75 (m, 1H), 3.67 (s, 3H), 2.99 (t, *J* = 6.4 Hz, 2H), 2.38 (s, 3H), 2.27 (s, 3H), 2.13 (s, 3H), 1.31 (d, *J* = 4.0 Hz, 1H), 1.11 (d, *J* = 6.8 Hz, 3H).

### Example 10, Compound 10-1, and Compound 10-2

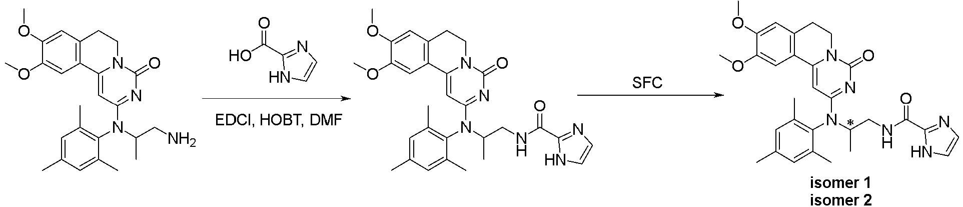

### Preparation steps:

**1.**

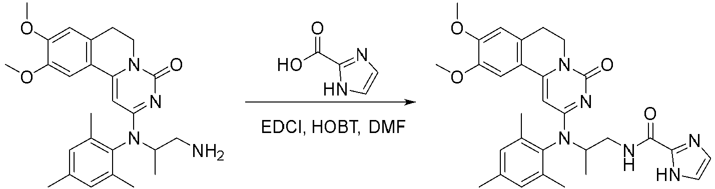

2-((1-Aminopropan-2-yl)(2,4,6-trimethylphenyl)amino)-9,10-dimethoxy-6,7-dihydro-4H-pyrimido[6,1-a]isoquinolin-4-one (150 mg, 0.33 mmol, 1.0 equiv), imidazole-2-carboxylic acid (56 mg, 0.5 mmol, 1.5 equiv), 1-(3-dimethylaminopropyl)-3-ethylcarbodiimide (96 mg, 0.5 mmol, 1.5 equiv), and 1-hydroxybenzotriazole (67 mg, 0.5 mmol, 1.5 equiv) were dissolved in DMF (2 mL). The resulting mixture was stirred at room temperature for 1 h under a nitrogen atmosphere, and then concentrated under reduced pressure. The crude product was purified by reversed-phase column chromatography under the following conditions: column: Xselect CSH C185 m, 30 mm × 150 mm; mobile phase A: water (0.1% FA); mobile phase B: acetonitrile; flow rate: 60 mL/min; gradient: 27% B to 55% B in 9 min; wavelength: 254/220 nm; RT1 (min): 6.77/8.43, to give N-(2-((9,10-dimethoxy-4-oxo-6,7-dihydro-4H-pyrimido[6,1-a]isoquinolin-2-yl)(2,4,6-trimethylphenyl)amino)propyl)-1H-imidazole-2-carboxamide (70 mg, 44.0% yield).

LCMS (ESI): [M+H] ⁺ = 544.25

**2.**

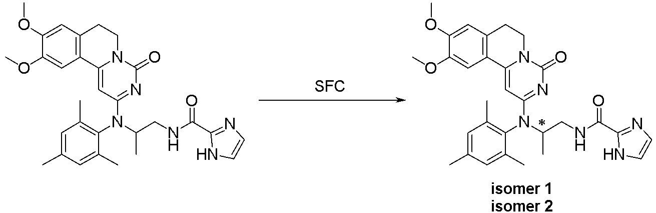

The racemate (70 mg) was purified by preparative Chiral-SFC under the following conditions, column: CHIRAL ART Cellulose-SB, 3 × 25 cm, 5 µm; mobile phase A: CO₂; mobile phase B: MeOH (0.1% 2M NH₃-MeOH); flow rate: 100 mL/min; gradient: isocratic 25% B; column temperature (°C): 35; pressure (bar): 100; wavelength: 220 nm; RT1 (min): 9.4; RT2 (min): 10.72; sample solvent: MeOH (0.1% DEA)-HPLC; injection volume: 1 mL, to obtain the products.
**Compound 10-1** (isomer 1, RT1 (min): 9.4, 24.9 mg, 34.5% yield)
LCMS (ESI): [M+H] ⁺ = 543.25
¹H NMR (400 MHz, Methanol-*d*₄) δ 7.21 (s, 3H), 7.16 - 7.08 (m, 2H), 6.94 (s, 1H), 6.65 (s, 1H), 5.43 (s, 1H), 4.70 (s, 1H), 4.21 - 4.02 (m, 2H), 3.97 - 3.91 (m, 1H), 3.89 (s, 4H), 3.82 - 3.84 (m, 1H), 3.67 (s, 3H), 3.05 - 2.94 (m, 2H), 2.38 (s, 3H), 2.27 (s, 3H), 2.14 (s, 3H), 1.31 (s, 1H), 1.15 (d, *J =* 6.8 Hz, 3H).
**Compound 10-2** (isomer 2, RT2 (min): 10.72, 24.4 mg, 34.5% yield)
LCMS (ESI): [M+H] ⁺ = 543.25
¹H NMR (400 MHz, Methanol-*d*₄) δ 7.21 (s, 3H), 7.16 - 7.08 (m, 2H), 6.94 (s, 1H), 6.65 (s, 1H), 5.43 (s, 1H), 4.70 (s, 1H), 4.21 - 4.02 (m, 2H), 3.97 - 3.91 (m, 1H), 3.89 (s, 4H), 3.84 - 3.76 (m, 1H), 3.67 (s, 3H), 3.05 - 2.94 (m, 2H), 2.38 (s, 3H), 2.27 (s, 3H), 2.14 (s, 3H), 1.31 (s, 1H), 1.15 (d, *J =* 6.8 Hz, 3H).

Compounds 98 to 125 (derivatives and deuterated forms of Compound 10) were synthesized separately. The structures of the compounds are shown in Table 1, and the MS data are summarized in Table 3.

**Table 3**

| Example No. | MS value [M+H⁺] | Example No. | MS value [M+H⁺] |
|---|---|---|---|
| Compound **98** | 577.23 | Compound **112** | 549.30 |
| Compound **99** | 561.25 | Compound **113** | 544.27 |
| Compound **100** | 559.26 | Compound **114** | 544.27 |
| Compound **101** | 559.26 | Compound **115** | 545.28 |
| Compound **102** | 559.26 | Compound **116** | 545.28 |
| Compound **103** | 573.27 | Compound **117** | 552.32 |
| Compound **104** | 559.26 | Compound **118** | 544.27 |
| Compound **105** | 591.25 | Compound **119** | 544.27 |
| Compound **106** | 559.26 | Compound **120** | 545.28 |
| Compound **107** | 557.28 | Compound **121** | 546.28 |
| Compound **108** | 571.30 | Compound **122** | 545.28 |
| Compound **109** | 585.31 | Compound **123** | 544.27 |
| Compound **110** | 546.28 | Compound **124** | 544.27 |
| Compound **111** | 546.28 | Compound **125** | 546.28 |

### Example 11, Compound 11-1, and Compound 11-2

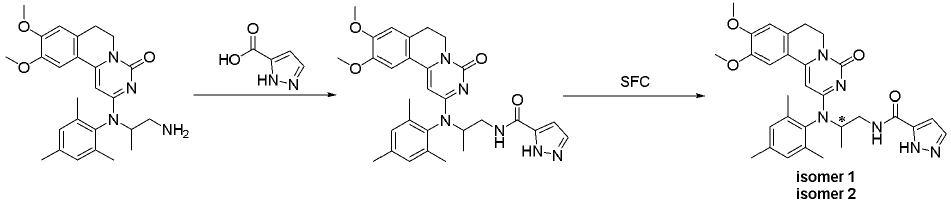

### Preparation steps:

**1.**

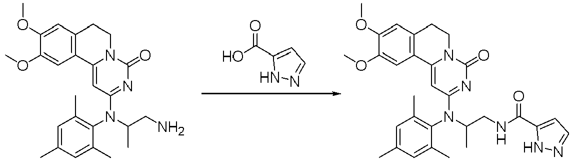

2H-Pyrazole-3-carboxylic acid (25 mg, 0.2 mmol, 1.0 equiv) was dissolved in SOCl₂ (1 mL), and stirred at room temperature for 2 h under a nitrogen atmosphere. The mixture was concentrated by rotary evaporation under vacuum. The above mixture was dissolved in DCM (1 mL) and added dropwise to a solution of 2-[(1-aminopropan-2-yl)(2,4,6-trimethylphenyl)amino]-9,10-dimethoxy-6H,7H-pyrimido[4,3-a]isoquinolin-4-one (100 mg, 0.2 mmol, 1.0 equiv) and N,N-diisopropylethylamine (86 mg, 0.7 mmol, 3.0 equiv) in DCM (5 mL) at 0 °C under a nitrogen atmosphere. The reaction mixture was stirred at room temperature for 2 h under nitrogen, quenched with water (5 mL) at room temperature, extracted with ethyl acetate (3 × 10 mL), dried over anhydrous sodium sulfate, filtered, and concentrated under reduced pressure. The residue was purified by reverse-phase chromatography under the following conditions: column, C18 silica gel; mobile phase, ACN in water (0.1% FA), 10% to 50% gradient over 10 min; UV detection, 254 nm. After purification, N-[2-({9,10-dimethoxy-4-oxo-6H,7H-pyrimido[4,3-a]isoquinolin-2-yl}(2,4,6-trimethylphenyl)amino)propyl]-2H-pyrazole-3-carboxamide (40 mg, 32.0% yield) was obtained.

**2.**

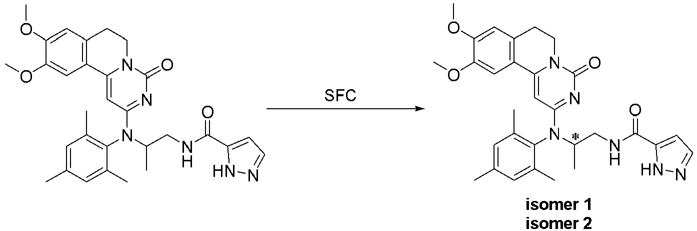

The crude product (40 mg) was purified by column chromatography under the following conditions: column: CHIRALPAK IH, 5 µm, 3 cm × 25 cm; mobile phase A: CO₂; mobile phase B: MEOH (0.1% DEA); flow rate: 80 mL/min; gradient: isocratic 40% B; column temperature (°C): 10°C; column head temperature (°C): 10°C; 80 mL/min; gradient: 40% B isocratic; column temperature (°C): 35; pressure (bar): 100; wavelength: 220 nm; RT1 (min): 2.73; RT2 (min): 4.17; Sample solvent: Injection volume: 4.8 mL, to give the products.
**Compound 11-1** (isomer 1, RT1 (min): 2.73, 12.1 mg, 9.6% yield)
LCMS (ESI): [M+H] ⁺ = 543.55
¹H NMR (400 MHz, Methanol-*d*₄) δ 7.71 (d, *J =* 2.4 Hz, 1H), 7.13 (d, *J =* 13.6 Hz, 2H), 6.93 (s, 1H), 6.78 (d, *J* = 2.4 Hz, 1H), 6.65 (s, 1H), 5.43 (s, 1H), 4.70 (s, 1H), 4.16 - 4.10 (m, 2H), 3.91 - 3.78 (m, 5H), 3.67 (s, 3H), 2.98 (t, *J =* 6.4 Hz, 2H), 2.38 (s, 3H), 2.27 (s, 3H), 2.13 (s, 3H), 1.11 (d, *J* = 6.8 Hz, 3H).
**Compound 11-2** (isomer 2, RT2 (min): 4.17, 10.9 mg, 26.6% yield)
LCMS (ESI): [M+H] ⁺ = 543.35
¹H NMR (400 MHz, Methanol-*d*₄) δ 7.71 (d, *J* = 2.4 Hz, 1H), 7.18 - 7.12 (m, 2H), 6.93 (s, 1H), 6.78 (d, *J =* 2.4 Hz, 1H), 6.65 (s, 1H), 5.43 (s, 1H), 4.75 - 4.55 (m, 1H), 4.16 - 4.10 (m, 2H), 3.91 - 3.78 (m, 5H), 3.67 (s, 3H), 2.98 (t, *J =* 6.4 Hz, 2H), 2.38 (s, 3H), 2.27 (s, 3H), 2.13 (s, 3H), 1.11 (d, *J =* 6.8 Hz, 3H).

### Example 12, Compound 12-1, and Compound 12-2

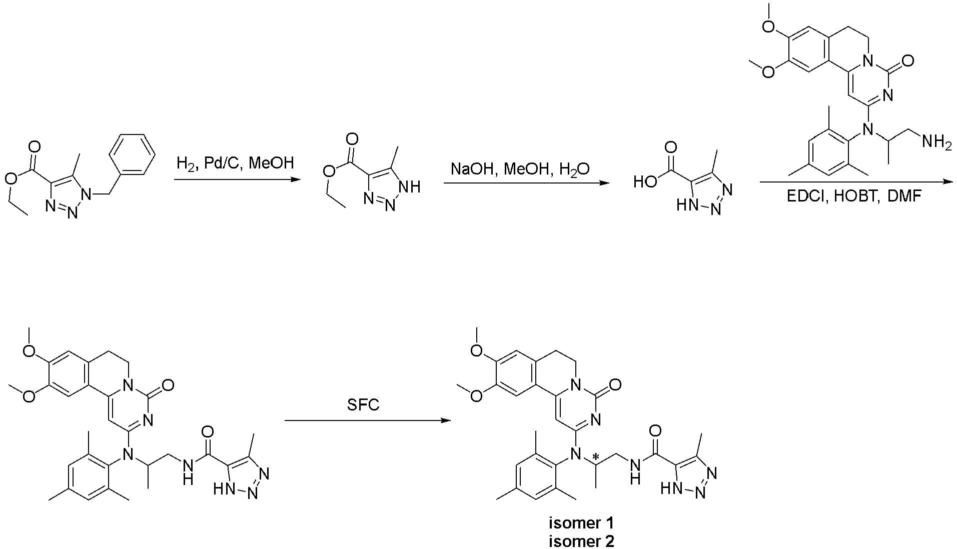

### Preparation steps:

**1.**

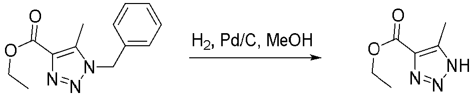

In a high-pressure reactor, Pd/C (10%, 2.90 g) was added to a solution of ethyl 1-benzyl-5-methyl-1,2,3-triazole-4-carboxylate (850 mg, 3.5 mmol, 1.0 equiv) in methanol (150 mL). The mixture was stirred at room temperature under a hydrogen atmosphere at 20 bar for 18 h, filtered through diatomaceous earth, and concentrated under reduced pressure. The residue was purified by silica gel column chromatography, eluting with PE/EA (1/1), to give ethyl 5-methyl-1H-1,2,3-triazole-4-carboxylate (260 mg, 48.4% yield).

LCMS (ESI, m/z): [M+H] ⁺ = 156.1

**2.**

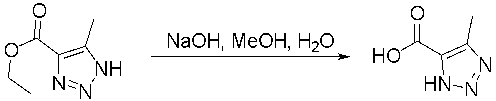

A mixture of ethyl 5-methyl-1H-1,2,3-triazole-4-carboxylate (260 mg, 1.7 mmol, 1.0 equiv) and sodium hydroxide (201 mg, 5.1 mmol, 3.0 equiv) in methanol (10 mL) and water (10 mL) was stirred at 60 °C for 18 h. Then the resulting mixture was concentrated under reduced pressure, acidified to pH=5 with 2 M aqueous hydrochloric acid, and was concentrated under reduced pressure. The residue was purified by reverse-phase chromatography under the following conditions: column: C18 silica gel; mobile phase: acetonitrile, water, 0-10% gradient over 10 min; detector: UV, 254 nm, to give 5-methyl-3H-1,2,3-triazole-4-carboxylic acid (200 mg, 93.9% yield).

LCMS (ESI): [M+H] ⁺ = 128.0

**3.**

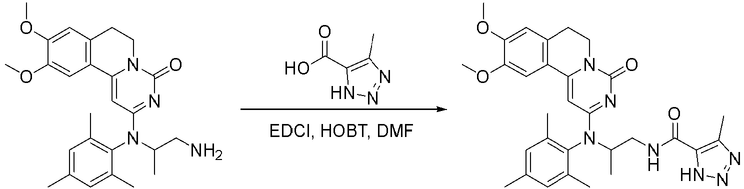

2-[(1-Aminopropan-2-yl)(2,4,6-trimethylphenyl)amino]-9,10-dimethoxy-6H,7H-pyrimido[4,3-a]isoquinolin-4-one (100 mg, 0.2 mmol, 1.0 equiv) and 5-methyl-3H-1,2,3-triazole-4-carboxylic acid (28.3 mg, 0.2 mmol, 1.0 equiv) were dissolved in DMF (3 mL) at room temperature. 1-(3-dimethylaminopropyl)-3-ethylcarbodiimide hydrochloride (64 mg, 0.3 mmol, 1.5 equiv) and 1-hydroxybenzotriazole (45 mg, 0.3 mmol, 1.5 equiv) were then added thereto. The resulting mixture was stirred at room temperature for 2 h, and concentrated under reduced pressure to obtain a crude product. The crude product was purified by reverse-phase chromatography under the following conditions: column: XBridge BEH Shield RP18, 5 m, 30 mm × 150 mm; mobile phase A: water (10 mmol/L ammonium bicarbonate); mobile phase B: acetonitrile; flow rate: 60 mL/min; gradient: 36% B to 54% B over 10 min; wavelength: 254/220 nm; RT1 (min): 6.18 min, to give N-[2-({9,10-dimethoxy-4-oxo-6H,7H-pyrimido[4,3-a]isoquinolin-2-yl}(2,4,6-trimethylphenyl)amino)propyl]-5-methyl-3H-1,2,3-triazole-4-carboxamide (30 mg, 24.1% yield).

LCMS (ESI): [M+H] ⁺ = 558.3

**4.**

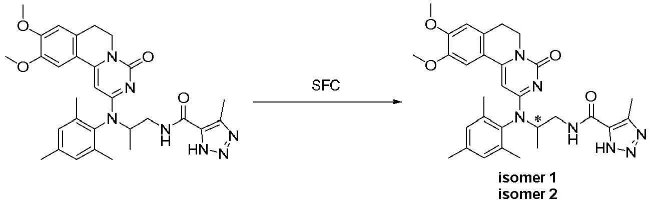

The crude product (30 mg) was purified by Chiral-HPLC under the following conditions: column: CHIRALPAK IH, 3 × 25 cm, 5 µm; mobile phase A: CO₂; mobile phase B: methanol (0.1% diethylamine); flow rate: 85 mL/min; gradient: isocratic 35% B; column temperature (°C): 25 °C; back pressure (bar): 100; wavelength: 254 nm; RT1 (min): 2.85; RT2 (min): 4.5; sample solvent: methanol/dichloromethane = 1:1-HPLC; injection volume: 1 mL, to obtain the products.
**Compound 12-1** (isomer 1, RT1 (min): 2.85, 1.6 mg, 5.14% yield)
LCMS (ESI, m/z): [M+H] ⁺ = 558.35
¹H NMR (400 MHz, Methanol-*d*₄) δ 7.17 - 7.10 (m, 2H), 6.93 (s, 1H), 6.65 (s, 1H), 5.43 (s, 1H), 4.78 - 4.69 (m, 1H), 4.20 - 4.04 (m, 2H), 3.97 - 3.77 (m, 5H), 3.67 (s, 3H), 2.98 (t, *J =* 6.5 Hz, 2H), 2.59 - 2.52 (m, 3H), 2.38 (s, 3H), 2.27 (s, 3H), 2.13 (s, 3H), 1.11 (d, *J =* 6.9 Hz, 3H).
**Compound 12-2** (isomer 2, RT2 (min): 4.5, 3.2 mg, 9.86% yield)
LCMS (ESI, m/z): [M+H] ⁺ = 558.46
¹H NMR (400 MHz, Methanol-*d*₄) δ 7.17 - 7.10 (m, 2H), 6.93 (s, 1H), 6.65 (s, 1H), 5.43 (s, 1H), 4.78 - 4.69 (m, 1H), 4.20 - 4.04 (m, 2H), 3.97 - 3.77 (m, 5H), 3.67 (s, 3H), 2.98 (t, *J =* 6.5 Hz, 2H), 2.59 - 2.52 (m, 3H), 2.38 (s, 3H), 2.27 (s, 3H), 2.13 (s, 3H), 1.11 (d, *J =* 6.9 Hz, 3H).

### Example 13, Compound 13-1, and Compound 13-2

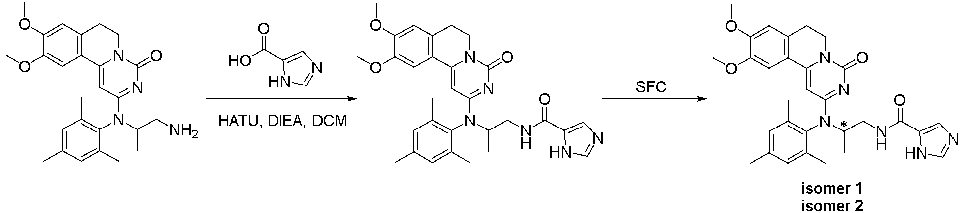

### Preparation steps:

**1.**

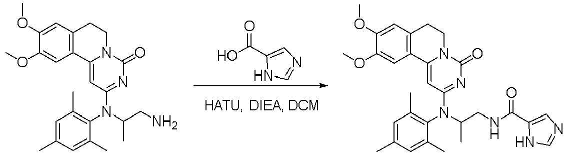

2-[(1-Aminopropan-2-yl)(2,4,6-trimethylphenyl)amino]-9,10-dimethoxy-6H,7H-pyrimido[4,3-a]isoquinolin-4-one (100 mg, 0.2 mmol, 1.0 equiv), 3H-imidazole-4-carboxylic acid (25 mg, 0.2 mmol, 1.0 equiv), HOBT (45 mg, 0.3 mmol, 1.5 equiv), and EDCI (64 mg, 0.3 mmol, 1.5 equiv) were dissolved in DMF (5 mL). The resulting solution was stirred at room temperature under a nitrogen atmosphere for 2 h, and quenched with water (10 mL) at room temperature. The aqueous layer was extracted with ethyl acetate (3 × 20 mL). The organic layer was washed with saturated brine (3 × 50 mL), dried over anhydrous sodium sulfate, filtered, and concentrated under reduced pressure. The resultant was purified by reverse-phase chromatography under the following conditions: column: C18 silica gel; mobile phase: acetonitrile/water (0.1% FA); gradient: 10%-50% over 10 min; detector: UV 254 nm. After purification, N-[2-({9,10-dimethoxy-4-oxo-6H,7H-pyrimido[4,3-a]isoquinolin-2-yl}(2,4,6-trimethylphenyl)amino)propyl]-3H-imidazole-4-carboxamide was obtained.

**2.**

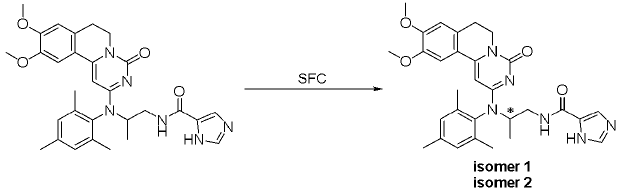

The crude product (20 mg) was purified by Chiral-HPLC under the following conditions: column: CHIRALPAK IH, 3 × 25 cm, 5 µm; mobile phase A: CO₂; mobile phase B: MeOH/DCM = 2:1 (0.1% 2M NH₃-MeOH); flow rate: 100 mL/min; gradient: isocratic 18% B; column temperature (°C): 20; column head temperature (°C): 20; 100 mL/min; gradient: isocratic 18% B; column temperature (°C): 35; back pressure (bar): 100; wavelength: 220 nm; RT1 (min): 14.20; RT2 (min): 21.37; sample solvent: DCM-HPLC; injection volume: 2 mL, to give the products.
**Compound 13-1** (isomer 1, RT1 (min): 14.2, 2.04 mg, 1.6% yield)
LCMS (ESI): [M+H] ⁺ = 543.20
¹H NMR (400 MHz, Methanol-*d*₄) δ 7.75 - 7.70 (m, 2H), 7.15 - 7.11 (m, 2H), 6.98 - 6.92 (m, 1H), 6.65 (s, 1H), 5.43 (s, 1H), 4.75 - 4.55 (m, 1H), 4.15 - 4.09 (m, 2H), 3.90 (s, 3H), 3.85 - 3.80 (m, 2H), 3.67 (s, 3H), 2.98 (t, *J* = 6.4 Hz, 2H), 2.38 (s, 3H), 2.33 - 2.27 (m, 3H), 2.15 (s, 3H), 1.16 (d, *J =* 6.9 Hz, 3H).
**Compound 13-2** (isomer 2, RT2 (min): 21.37, 2.72 mg, 13.0% yield)
LCMS (ESI): [M+H] ⁺ = 543.20
¹H NMR (400 MHz, Methanol-*d*₄) δ 7.75 - 7.70 (m, 2H), 7.15 - 7.11 (m, 2H), 6.98 - 6.92 (m, 1H), 6.65 (s, 1H), 5.43 (s, 1H), 4.75 - 4.55 (m, 1H), 4.15 - 4.09 (m, 2H), 3.90 (s, 3H), 3.85 - 3.80 (m, 2H), 3.67 (s, 3H), 2.98 (t, *J* = 6.4 Hz, 2H), 2.38 (s, 3H), 2.33 - 2.27 (m, 3H), 2.15 (s, 3H), 1.16 (d, *J =* 6.9 Hz, 3H).

### Example 14, Compound 14

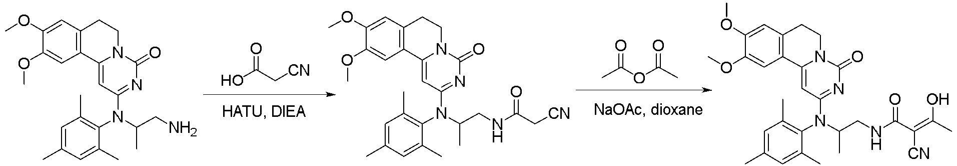

### Preparation steps:

**1.**

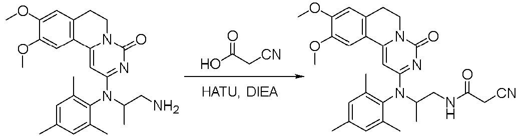

2-[(1-Aminopropan-2-yl)(2,4,6-trimethylphenyl)amino]-9,10-dimethoxy-6H,7H-pyrimido[4,3-a]isoquinolin-4-one (250 mg, 0.6 mmol, 1.0 equiv), DCM (5 mL), DIEA (216 mg, 1.7 mmol, 3.0 equiv), cyanoacetic acid (57 mg, 0.7 mmol, 1.2 equiv), and HATU (318 mg, 0.8 mmol, 1.5 equiv) were added sequentially to a single-necked flask. A reaction was allowed to proceed at room temperature overnight. The solvent was removed by concentration, and the residue was purified by reverse-phase chromatography under the following conditions: column: C18 silica gel; mobile phase: acetonitrile water (10mmol/L NH₄HCO₃); gradient: 10% to 50% acetonitrile over 30 min; detection wavelength: 254 nm, to give 2-cyano-N-[2-({9,10-dimethoxy-4-oxo-6H,7H-pyrimido[4,3-a]isoquinolin-2-yl}(2,4,6-trimethylphenyl)amino)propyl]acetamide (200 mg, 69.6% yield).

LCMS (ESI, m/z): [M+H] ⁺ = 516.2

**2.**

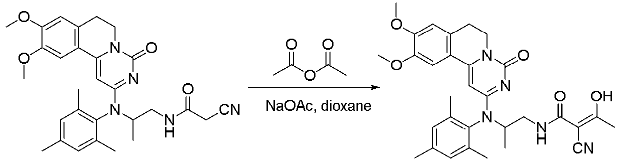

2-Cyano-N-[2-({9,10-dimethoxy-4-oxo-6H,7H-pyrimido[4,3-a]isoquinolin-2-yl}(2,4,6-trimethylphenyl)amino)propyl]acetamide (200 mg, 0.4 mmol, 1.0 equiv), 1,4-dioxane (30 mL), sodium acetate (95 mg, 1.2 mmol, 3.0 equiv), and acetic anhydride (48 mg, 0.5 mmol, 1.2 equiv) were added sequentially to a single-necked flask. A reaction was allowed to proceed at 100 °C overnight. The solvent was removed by concentration, and the residue was purified by reverse-phase chromatography under the following conditions: column: XBridge BEH Shield RP18, 5 m, 30 mm × 150 mm; mobile phase A: water (10 mmol/L NH₄HCO₃); mobile phase B: acetonitrile; flow rate: 60 mL/min; gradient: 21% B to 44% B over 9 min; wavelength: 254/220 nm; RT1 (min): 6.82, to give (2Z)-2-cyano-N-[2-({9,10-dimethoxy-4-oxo-6H,7H-pyrimido[4,3-a]isoquinolin-2-yl}(2,4,6-trimethylphenyl)amino)propyl]-3-hydroxybut-2-enamide (13.27 mg, 6.1% yield).
LCMS (ESI, m/z): [M+H] ⁺ = 558.25
¹H NMR (400 MHz, Methanol-*d4*) δ 7.15 - 7.05 (m, 2H), 7.07 - 6.88 (m, 2H), 6.63 (s, 1H), 5.40 (s, 1H), 4.68 - 4.59 (m, 1H), 4.21 - 4.12 (m, 2H), 4.11 - 4.05 (m, 1H), 3.89 (s, 3H), 3.66 (s, 3H), 2.98 (t, *J* = 6.5 Hz, 2H), 2.37 (s, 3H), 2.33 (s, 1H), 2.25 - 2.23 (m, 6H), 2.14 (s, 3H), 1.09 (d, *J =* 6.7 Hz, 3H).

### Example 15, Compound 15-1, and Compound 15-2

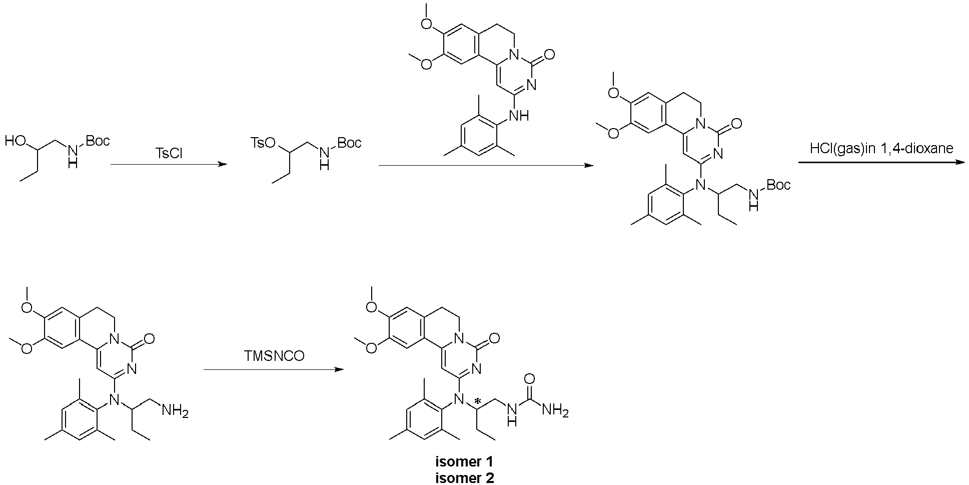

### Preparation steps:

**1.**

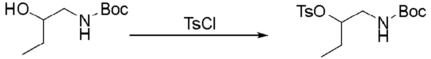

Tert-butyl N-(2-hydroxybutyl)carbamate (500 mg, 2.6 mmol, 1.0 equiv) and TEA (1.1 mL, 7.9 mmol, 3.0 equiv) were dissolved in DCM (5 mL) at 0 °C under air. TsCl (1.2 g, 3.9 mmol, 1.5 equiv) and DMAP (32 mg, 0.3 mmol, 0.1 equiv) were added separately. The resulting mixture was stirred at room temperature under a nitrogen atmosphere for 1.5 h. The reaction was quenched with water at room temperature. The resulting mixture was extracted with dichloromethane (2 × 50 mL). The combined organic layers were washed with saturated brine (2 × 100 mL), dried over anhydrous sodium sulfate, filtered, and concentrated under reduced pressure. The residue was purified by reverse-phase chromatography under the following conditions: C18 silica gel column; mobile phase, ACN water (10 mmol/L NH₄HCO₃); gradient, 10%-50% over 10 min; detector, UV 254 nm, to give tert-butyl N-{2-[(4-methylbenzenesulfonyl)oxy]butyl}carbamate (700 mg, 69% yield).

LCMS (ESI): [M+H] ⁺ = 361.2

**2.**

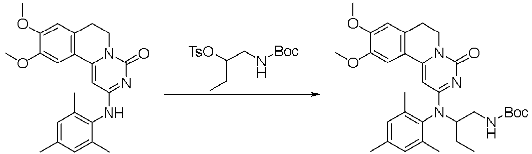

9,10-Dimethoxy-2-[(2,4,6-trimethylphenyl)amino]-6H,7H-pyrimido[4,3-a]isoquinolin-4-one (1.0 g, 2.5 mmol, 1.0 equiv), tert-butyl N-{2-[(4-methylbenzenesulfonyl)oxy]butyl}carbamate (1.3 g, 3.8 mmol, 1.5 equiv), and cesium carbonate (1.7 g, 5.108 mmol, 2.0 equiv) were dissolved in N,N-dimethylformamide (10 mL). Then XantPhos (295 mg, 0.5 mmol, 0.2 equiv) and Pd₂(dba)₃ (233 mg, 0.3 mmol, 0.1 equiv) were added sequentially to the mixture at room temperature under air. The resulting mixture was stirred at 100 °C overnight under a nitrogen atmosphere. The reaction was quenched with water at room temperature. The resulting mixture was extracted with ethyl acetate (2 × 50 mL). The combined organic layers were washed with saturated brine (2 × 100 mL), dried over anhydrous sodium sulfate, filtered, and concentrated under reduced pressure. The residue was purified by reverse-phase chromatography under the following conditions: column, C18 silica gel; mobile phase, acetonitrile water (10 mmol/L NH₄HCO₃); gradient, 50%-60% over 10 min; detector, UV 254 nm, to give tert-butyl N-[2-({9,10-dimethoxy-4-oxo-6H,7H-pyrimido[4,3-a]isoquinolin-2-yl}(2,4,6-trimethylphenyl)amino)butyl]carbamate (330 mg, crude product).

LCMS (ESI): [M+H] ⁺= 563.2

3.

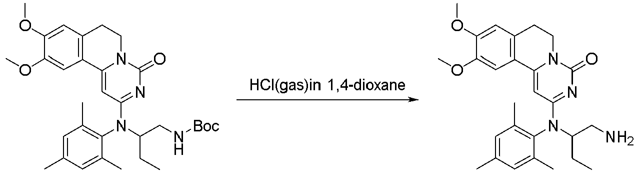

tert-Butyl N-[2-({9,10-dimethoxy-4-oxo-6H,7H-pyrimido[4,3-a]isoquinolin-2-yl}(2,4,6-trimethylphenyl)amino)butyl]carbamate (330 mg, 0.6 mmol, 1.0 equiv) was dissolved in 1,4-dioxane (2 mL) at room temperature under a nitrogen atmosphere, and HCl in 1,4-dioxane (4 M, 2 mL) was added. The mixture was stirred at room temperature under a nitrogen atmosphere for 1.5 h. The reaction was quenched with water at room temperature. The mixture was neutralized to pH=9 with saturated sodium carbonate solution, and extracted with ethyl acetate (2 × 50 mL). The combined organic layers were washed with saturated brine (2 × 100 mL), dried over anhydrous sodium sulfate, filtered, and concentrated under reduced pressure. The residue was purified by Prep-TLC (DCM/MeOH=20/1), to give 2-[(1-aminobutan-2-yl)(2,4,6-trimethylphenyl)amino]-9,10-dimethoxy-6H,7H-pyrimido[4,3-a]isoquinolin-4-one (150 mg, 49% yield).

LCMS (ESI): [M+H] ⁺= 463.2

4.

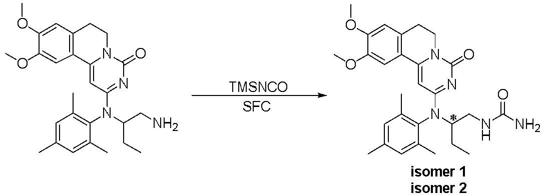

2-[(1-Aminobutan-2-yl)(2,4,6-trimethylphenyl)amino]-9,10-dimethoxy-6H,7H-pyrimido[4,3-a]isoquinolin-4-one (100 mg, 0.2 mmol, 1 equiv) and triethylamine (65 mg, 0.6 mmol, 3.0 equiv) were dissolved in DCM (1 mL). Trimethylsilyl isocyanide (37 mg, 0.3 mmol, 1.5 equiv) was added to the mixture at 0 °C under air. The resultant mixture was stirred at room temperature under a nitrogen atmosphere for 2 h, and concentrated under reduced pressure. The crude product (50 mg) was purified by preparative liquid chromatography under the following conditions: column, XBridge BEH C185, 19 × 250 mm; mobile phase A, water (10 mmol/L NH₄HCO₃); mobile phase B, acetonitrile; flow rate, 25 mL/min; gradient, 41% B to 48% B over 10 min; wavelength: UV at 254/220 nm; RT (min), 7.25/8.32 min, to give the products.
**Compound 15-1** (isomer 1, RT (min): 7.25, 4.02 mg, 4% yield)
LCMS (ESI): [M+H] + = 506.25
¹H NMR (400 MHz, Methanol-d4) δ 7.15 - 7.06 (m, 2H), 6.93 (s, 1H), 6.63 (s, 1H), 5.41 (s, 1H), 4.21 - 4.02 (m, 3H), 3.89 (s, 3H), 3.66 (s, 4H), 2.97 (t, J = 8.0. Hz, 2H), 2.37 (s, 3H), 2.21 (s, 3H), 2.11 (s, 3H), 1.66 - 1.52 (m, 2H), 0.98 (t, J = 7.4 Hz, 3H).
**Compound 15-2** (isomer 2, RT (min): 8.32, 4.02 mg, 4% yield)
LCMS (ESI): [M+H] + = 506.25
¹H NMR (400 MHz, Methanol-d4) δ 7.15 - 7.06 (m, 2H), 6.93 (s, 1H), 6.63 (s, 1H), 5.41 (s, 1H), 4.21 - 4.02 (m, 3H), 3.89 (s, 3H), 3.66 (s, 4H), 2.97 (t, J = 8.0. Hz, 2H), 2.37 (s, 3H), 2.21 (s, 3H), 2.11 (s, 3H), 1.66 - 1.52 (m, 2H), 0.98 (t, J = 7.4 Hz, 3H).

### Example 16, Compound 16

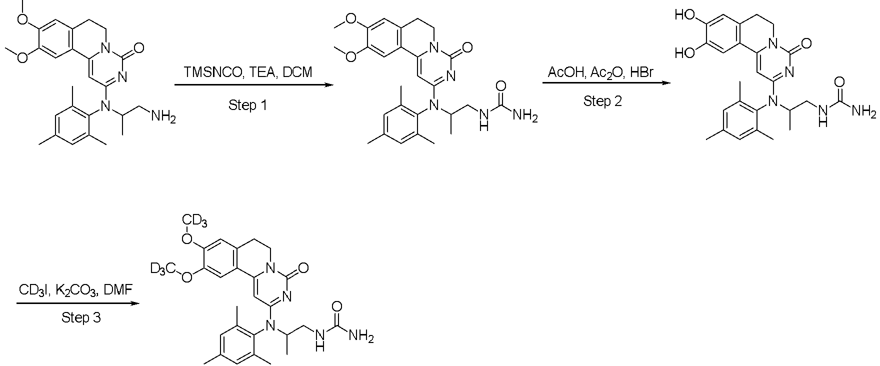

### Preparation steps

1.

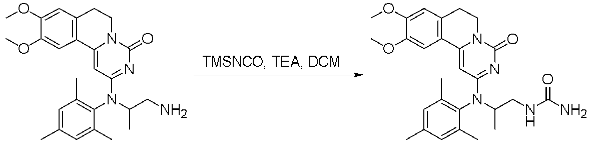

Trimethylsilyl isocyanate (77 mg, 0.7 mmol, 1.5 equiv) was added to a solution of 2-[(1-aminopropan-2-yl)(2,4,6-trimethylphenyl)amino]-9,10-dimethoxy-6H,7H-pyrimido[4,3-a]isoquinolin-4-one (200 mg, 0.4 mmol, 1.0 equiv) and triethylamine (135 mg, 1.3 mmol, 3.0 equiv) in dichloromethane (5 mL) at room temperature. The reaction solution was stirred for reaction at room temperature for 2 h. The reaction mixture was extracted with dichloromethane (3× 20 mL). The organic phases were combined, washed with saturated brine, dried over anhydrous sodium sulfate, filtered, and concentrated under reduced pressure to give 2-({9,10-dimethoxy-4-oxo-6H,7H-pyrimido[4,3-a]isoquinolin-2-yl}(2,4,6-trimethylphenyl)amino)propylurea (200 mg, crude product).

LCMS (ESI): [M+H] ⁺= 492.3

2.

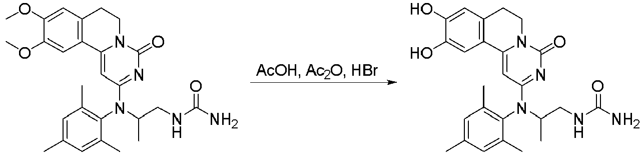

2-({9,10-Dimethoxy-4-oxo-6H,7H-pyrimido[4,3-a]isoquinolin-2-yl}(2,4,6-trimethylphenyl)amino)propylurea (200 mg, 0.4 mmol, 1.0 equiv), acetic acid (1 mL), acetic anhydride (1 mL), and hydrobromic acid (1 mL) were added to an 8 mL sample vial at room temperature. The reaction solution was stirred for reaction at 120 °C overnight. The reaction mixture was basified to pH=6 with saturated aqueous sodium bicarbonate solution, and extracted with dichloromethane and methanol (3× 50 mL). The organic phases were combined, washed with saturated brine (3 × 50mL), dried over anhydrous sodium sulfate, filtered, and concentrated under reduced pressure to give 2-({9,10-dihydroxy-4-oxo-6H,7H-pyrimido[4,3-a]isoquinolin-2-yl}(2,4,6-trimethylphenyl)amino)propylurea (180 mg, 95.4% yield).

LCMS (ESI): [M+H] ⁺= 464.2

3.

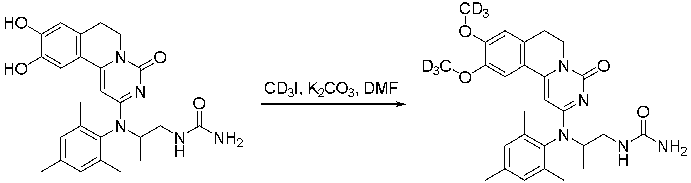

Potassium carbonate (268 mg, 1.8 mmol, 6.0 equiv) and deuterated methyl iodide (234 mg, 1.5 mmol, 5.0 equiv) were added to a solution of 2-({9,10-dihydroxy-4-oxo-6H,7H-pyrimido[4,3-a]isoquinolin-2-yl}(2,4,6-trimethylphenyl)amino)propylurea (150 mg, 0.3 mmol, 1.0 equiv) in N,N-dimethylformamide (9 mL) at room temperature. The reaction solution was stirred for reaction at room temperature overnight. The crude product was purified by reverse-phase column chromatography (Conditions, Column: Xbridge BEH Shield RP18 5 µm, 30 × 150 mm; mobile phase A: water (10 mmol/L ammonium bicarbonate); mobile phase B: acetonitrile; flow rate: 60 mL/min; gradient: 35% B to 45% B in 7 min; wavelength: 254 nm/220 nm; RT1 (min): 6.23) to give 2-{[9,10-bis(²H3)methoxy-4-oxo-6H,7H-pyrimido[4,3-a]isoquinolin-2-yl](2,4,6-trimethylphenyl)amino}propylurea (25.92 mg, 15.52% yield)..
LCMS (ESI): [M+H] ⁺= 498.30
¹H NMR (400 MHz, Methanol-d₄) δ 7.11 (d, *J =* 10.6 Hz, 2H), 6.93 (s, 1H), 6.63 (s, 1H), 5.40 (s, 1H), 4.60 (s, 2H), 4.49 (s, 1H), 4.20 - 4.01 (m, 2H), 2.97 (t, *J =* 6.5 Hz, 2H), 2.37 (s, 3H), 2.22 (s, 3H), 2.09 (s, 3H), 1.04 - 0.96 (m, 3H).

### Example 17, Compound 17

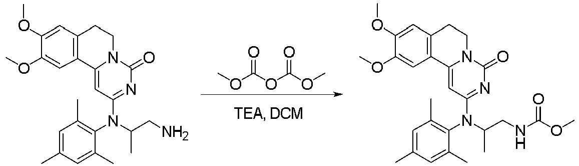

To a solution of 2-[(1-aminopropan-2-yl)(2,4,6-trimethylphenyl)amino]-9,10-dimethoxy-6H,7H-pyrimido[4,3-a]isoquinolin-4-one (60 mg, 0.18 mmol, 1.0 equiv) in DCM (2 mL), triethylamine (41 mg, 0.40 mmol, 3.0 equiv) and dimethyl dicarbonate (36 mg, 0.27 mmol, 2.0 equiv) were added. After the mixture was stirred for 2 h, it was concentrated by rotary evaporation under reduced pressure. The crude product (80 mg) was purified by normal-phase Prep-TLC using EA/PE = 1/3 to give methyl N-[2-({9,10-dimethoxy-4-oxo-6H,7H-pyrimido[4,3-a]isoquinolin-2-yl}(2,4,6-trimethylphenyl)amino)propyl]carbamate (23.01 mg, 32.94% yield).
LCMS (ESI): [M+H] ⁺= 507.25
¹H NMR (400 MHz, Methanol-d₄) δ 7.09 (m, 2H), 6.90 (s, 1H), 6.61 (s, 1H), 5.38 (s, 1H), 4.48 - 4.42 (m, 1H), 4.20 - 4.02 (m, 2H), 3.86 (s, 3H), 3.64 (s, 6H), 3.60 - 3.53 (m, 1H), 3.48 - 3.38 (m, 1H), 2.94 (t, *J* = 6.5 Hz, 2H), 2.35 (s, 3H), 2.19 (s, 3H), 2.09 (s, 3H), 1.06 (d, *J* = 6.9 Hz, 3H).

### Example 18, Compound 18

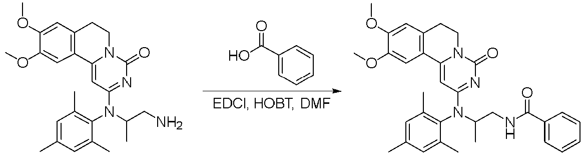

To a solution of benzoic acid (76 mg, 0.62 mmol, 3.5 equiv) in DMF (1 mL), 2-[(1-aminopropan-2-yl)(2,4,6-trimethylphenyl)amino]-9,10-dimethoxy-6H,7H-pyrimido[4,3-a]isoquinolin-4-one (80 mg, 0.18 mmol, 1.0 equiv), 1-(3-dimethylaminopropyl)-3-ethylcarbodiimide hydrochloride (103 mg, 0.53 mmol, 3.0 equiv), and 1-hydroxybenzotriazole (72 mg, 0.53 mmol, 3.0 equiv) were added. The mixture was stirred for 2 h, and concentrated under reduced pressure. The crude product (100 mg) was purified by Prep-TLC (EA/PE = 1/5) to give N-[2-({9,10-dimethoxy-4-oxo-6H,7H-pyrimido[4,3-a]isoquinolin-2-yl}(2,4,6-trimethylphenyl)amino)propyl]benzamide (18.91 mg, 19.11% yield).
LC-MS (ESI): [M+H] ⁺= 553.3
¹H NMR (400 MHz, Methanol-d₄) δ 7.92 - 7.87 (m, 2H), 7.59 - 7.45 (m, 3H), 7.17 - 7.08 (m, 2H), 6.91 (s, 1H), 6.63 (s, 1H), 5.42 (s, 1H), 4.69 - 4.60 (m, 1H), 4.19 - 4.02 (m, 2H), 3.93 - 3.82 (m, 4H), 3.79 - 3.72 (m, 1H), 3.64 (s, 3H), 2.96 (t, *J =* 6.5 Hz, 2H), 2.36 (s, 3H), 2.25 (s, 3H), 2.11 (s, 3H), 1.12 (d, *J* = 7.0 Hz, 3H).

### Example 19, Compound 19

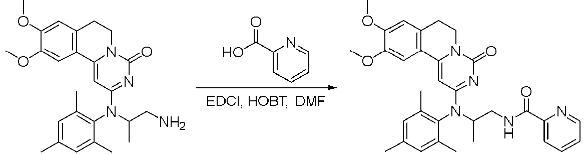

To a solution of 2-picolinic acid (41 mg, 0.34 mmol, 1.0 equiv) in DMF (1 mL), 2-[(1-aminopropan-2-yl)(2,4,6-trimethylphenyl)amino]-9,10-dimethoxy-6H,7H-pyrimido[4,3-a]isoquinolin-4-one (100 mg, 0.22 mmol, 1.0 equiv), 1-(3-dimethylaminopropyl)-3-ethylcarbodiimide hydrochloride (64 mg, 0.34 mmol, 1.5 equiv), and 1-hydroxybenzotriazole (45 mg, 0.34 mmol, 1.5 equiv) were added. The mixture was stirred for 2 h, and concentrated under reduced pressure. The crude product (100 mg) was purified by Prep-HPLC to give N [2-((9,10-dimethoxy-4-oxo-6H,7H-pyrimido[4,3-a]isoquinolin-2-yl)(2,4,6-trimethylphenyl)amino)propyl]pyridine-2-carboxamide (17.35 mg, 13.93% yield).
LCMS (ESI): [M+H] ⁺= 554.25
¹H NMR (400 MHz, Methanol-d₄) δ 8.69 - 8.60 (m, 1H), 8.06 (d, *J* = 7.7 Hz, 1H), 8.02 - 7.90 (m, 1H), 7.58 - 7.50 (m, 1H), 7.15 - 7.09 (m, 2H), 6.91 (s, 1H), 6.62 (s, 1H), 5.40 (s, 1H), 4.83 - 4.76 (m, 1H), 4.18 - 4.04 (m, 2H), 3.86 (s, 3H), 3.85 (d, *J* = 6.4 Hz, 2H), 3.64 (s, 3H), 2.95 (t, *J* = 6.5 Hz, 2H), 2.36 (s, 3H), 2.25 (s, 3H), 2.12 (s, 3H), 1.09 (d, *J=* 6.9 Hz, 3H).

### Example 20, Compound 20

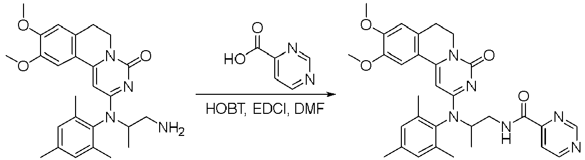

9,10-Dimethoxy-2-[(1-aminopropan-2-yl)(2,4,6-trimethylphenyl)amino]-6H,7H-pyrimido[4,3-a]isoquinolin-4-one (70 mg, 0.2 mmol, 1.0 equiv) and pyrimidine-4-carboxylic acid (58 mg, 0.5 mmol, 3.0 equiv) were dissolved in DMF (2 mL). At room temperature, HOBT (63 mg, 0.5 mmol, 3.0 equiv) and EDCI (90 mg, 0.5 mmol, 3.0 equiv) were then added to the mixture. The resultant was stirred at room temperature for 2 h. The crude product was purified by Prep-HPLC under the following conditions: column: XBridge BEH C18, 5 µm, 30 × 150 mm; mobile phase A: water (10 mmol/L ammonium bicarbonate); mobile phase B: acetonitrile; flow rate: 60 mL/min; gradient: 40% B to 47% B over 7 min; wavelengths: 254/220 nm; RT1 (min): 6.45, to give N-[2-((9,10-dimethoxy-4-oxo-6H,7H-pyrimido[4,3-a]isoquinolin-2-yl)(2,4,6-trimethylphenyl)amino)propyl]pyrimidine-4-carboxamide (19.00 mg, yield: 21.34%).
LCMS (ESI): [M+H] ⁺= 555.35
¹H NMR (400 MHz, Methanol-d₄) δ 9.32 (s, 1H), 9.03 (d, *J* = 4.8 Hz, 1H), 8.16 - 8.02 (m, 1H), 7.18 - 7.10 (m, 2H), 6.94 (s, 1H), 6.64 (s, 1H), 5.43 (s, 1H), 4.60 (s, 1H), 4.23 - 4.08 (m, 2H), 3.89 (d, *J* = 6.5 Hz, 5H), 3.67 (s, 3H), 2.98 (t, *J* = 6.5 Hz, 2H), 2.38 (s, 3H), 2.27 (s, 3H), 2.14 (s, 3H), 1.23 - 0.98 (m, 3H).

### Example 21, Compound 21

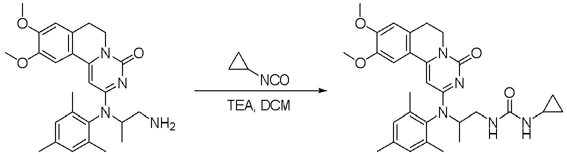

9,10-Dimethoxy-2-[(1-aminopropan-2-yl)(2,4,6-trimethylphenyl)amino]-6H,7H-pyrimido[4,3-a]isoquinolin-4-one (50 mg, 0.1 mmol, 1.0 equiv) was dissolved in DCM (3 mL). Cyclopropyl isocyanate (14 mg, 0.2 mmol, 1.5 equiv) and triethylamine (34 mg, 0.3 mmol, 3.0 equiv) were added to the above mixture at room temperature. The mixture was stirred at room temperature for 2 h. The crude product was purified by Prep-HPLC under the following conditions: column: Xbridge BEH Shield RP 18, 5 µm, 19 × 250 mm; mobile phase A: water (10 mmol/L ammonium bicarbonate); mobile phase B: acetonitrile; flow rate: 60 mL/min; gradient: 36% B to 50% B over 7 min; wavelengths: 254/220 nm; RT1 (min): 6.05/6.50, to give 3-cyclopropyl-1-[2-((9,10-dimethoxy-4-oxo-6H,7H-pyrimido[4,3-a]isoquinolin-2-yl)(2,4,6-trimethylphenyl)amino)propyl]urea (17.19 mg, yield: 28.92%).
LCMS (ESI): [M+H] ⁺= 532.30
¹H NMR (400 MHz, Methanol-d₄) δ 7.15 - 7.05 (m, 2H), 6.93 (s, 1H), 6.63 (s, 1H), 5.40 (s, 1H), 4.57 - 4.49 (m, 1H), 4.17 - 4.04 (m, 2H), 3.88 (s, 3H), 3.75 - 3.60 (m, 4H), 3.55 - 3.45 (m, 1H), 2.96 (t, *J* = 6.5 Hz, 2H), 2.57 - 2.48 (m, 1H), 2.37 (s, 3H), 2.23 (s, 3H), 2.11 (s, 3H), 1.09 (d, *J* = 6.9 Hz, 3H), 0.79 - 0.67 (m, 2H), 0.57 - 0.46 (m, 2H).

### Example 22, Compound 22

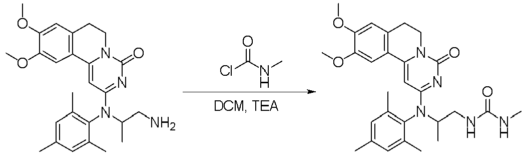

9,10-Dimethoxy-2-[(1-aminopropan-2-yl)(2,4,6-trimethylphenyl)amino]-6H,7H-pyrimido[4,3-a]isoquinolin-4-one (70 mg, 0.2 mmol, 1.0 equiv) and triethylamine (47 mg, 0.5 mmol, 3.0 equiv) were dissolved in DCM (3 mL) at room temperature. N-methylcarbamoyl chloride (22 mg, 0.2 mmol, 1.5 equiv) was added to the above mixture at 0 °C. The mixture was stirred at room temperature for 2 h. The crude product was purified by Prep-HPLC under the following conditions: column: XBridge BEH C18, 5 µm, 30 × 150 mm; mobile phase A: water (10 mmol/L ammonium bicarbonate); mobile phase B: acetonitrile; flow rate: 60 mL/min; gradient: 36% B to 48% B over 7 min; wavelengths: 254/220 nm; RT1 (min): 6.37, to give 1-[2-((9,10-dimethoxy-4-oxo-6H,7H-pyrimido[4,3-a]isoquinolin-2-yl)(2,4,6-trimethylphenyl)amino)propyl]-3-methylurea (16.82 mg, yield: 21.25%).
LCMS (ESI): [M+H] ⁺= 506.30
¹H NMR (400 MHz, Methanol-d₄) δ 7.16 - 7.07 (m, 2H), 6.93 (s, 1H), 6.63 (s, 1H), 5.39 (s, 1H), 4.60 (s, 1H), 4.46 (t, *J =* 7.6 Hz, 1H), 4.21 - 4.00 (m, 2H), 3.95 - 3.85 (m, 4H), 3.66 (s, 3H), 3.05 - 2.80 (m, 5H), 2.37 (s, 3H), 2.21 (s, 3H), 2.08 (s, 3H), 1.02 (d, *J* = 6.9 Hz, 3H).

### Example 23, Compound 23-1, and Compound 23-2

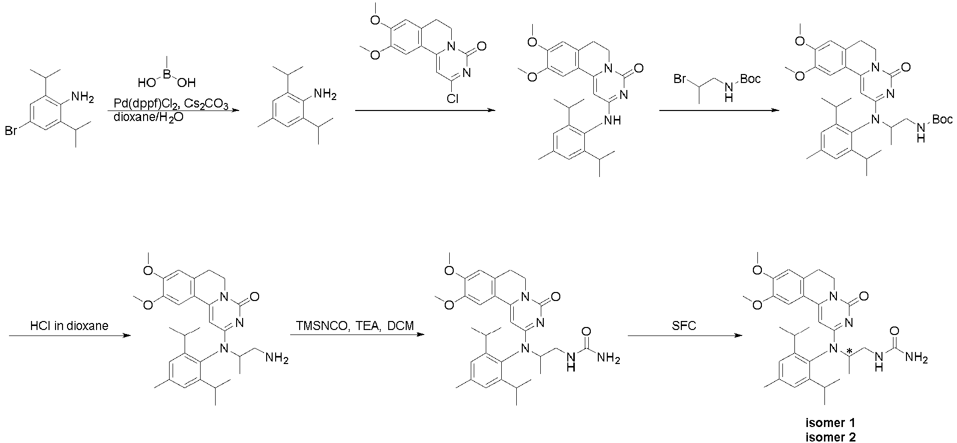

### Preparation steps:

1.

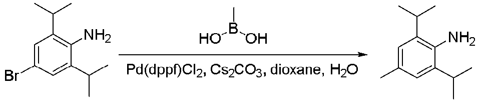

4-Bromo-2,6-diisopropylaniline (5 g, 20 mmol, 1.0 equiv), cesium carbonate (20.0 g, 60 mmol, 3.0 equiv), [1,1'-bis(diphenylphosphino)ferrocene]palladium dichloride dichloromethane complex (1.6 g, 2 mmol, 0.1 equiv), methylboronic acid (5.8 g, 98 mmol, 5 equiv), and 2-dicyclohexylphosphino-2',6'-dimethoxybiphenyl (1.6 g, 4 mmol, 0.2 equiv) were dissolved in 1,4-dioxane (90 mL) and water (15 mL). The mixture was stirred at 100 °C under a nitrogen atmosphere overnight, then cooled to room temperature, and dissolved in 100 mL of water. The resulting mixture was extracted with ethyl acetate (3 × 100 mL). The organic layers were washed with 100 mL saturated aqueous sodium chloride solution, dried over anhydrous sodium sulfate, filtered, and concentrated under reduced pressure. The crude product was purified by normal-phase column chromatography under the following conditions: mobile phase A: dichloromethane; mobile phase B: methanol; flow rate: 100 mL/min; gradient: 0% B to 10% B in 30 min; wavelength: 254 nm, to give 2,6-diisopropyl-4-methylaniline (2.1 g, yield: 56.2%).

LCMS (ESI): [M+H] ⁺= 192.17

2.

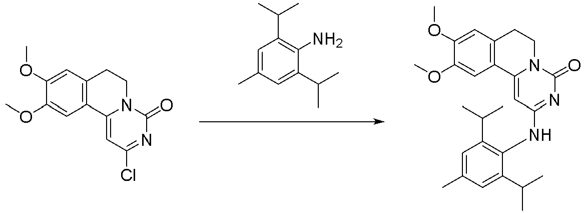

2,6-Diisopropyl-4-methylaniline (1 g, 5.2 mmol, 1 equiv) and 2-chloro-9,10-dimethoxy-6,7-dihydro-4H-pyrimido[6,1-a]isoquinolin-4-one (1.68 g, 5.8 mmol, 1.1 equiv) were dissolved in isopropanol (2 mL), and the resulting solution was stirred for reaction at 90 °C under a nitrogen atmosphere overnight. The resulting reaction mixture was concentrated under reduced pressure and filtered. The filter cake was collected and washed with ethyl acetate (2 × 10 mL) to give 2-[(2,6-diisopropyl-4-methylphenyl)amino]-9,10-dimethoxy-6H,7H-pyrimido[4,3-a]isoquinolin-4-one (1.0 g, yield: 42.7%).

LCMS (ESI): [M+H] ⁺= 448.25

3.

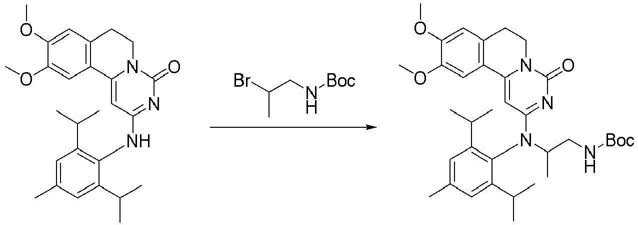

2-[(2,6-Diisopropyl-4-methylphenyl)amino]-9,10-dimethoxy-6H,7H-pyrimido[4,3-a]isoquinolin-4-one (700 mg, 1.6 mmol, 1.0 equiv), cesium carbonate (1.6 g, 4.8 mmol, 3.0 equiv), and tert-butyl N-(2-bromopropyl)carbamate (560 mg, 2.3 mmol, 1.5 equiv) were dissolved in 1,4-dioxane (10 mL). The mixture was stirred at 100 °C under a nitrogen atmosphere overnight, then cooled to room temperature, and dissolved in 20 mL of water. The resulting mixture was extracted with ethyl acetate (3 × 10 mL). The organic layers were washed with 20 mL of saturated aqueous sodium chloride solution, dried over anhydrous sodium sulfate, filtered, and concentrated under reduced pressure. The crude product was purified by reversed-phase column chromatography under the following conditions: column: C18; mobile phase A: water (10 mmol/L ammonium bicarbonate); mobile phase B: acetonitrile; flow rate: 60 mL/min; gradient: 50% B to 80% B over 20 min; wavelength: 254 nm, to give tert-butyl N-{2-[(2,6-diisopropyl-4-methylphenyl)((9,10-dimethoxy-4-oxo-6H,7H-pyrimido[4,3-a]isoquinolin-2-yl))amino]propyl} car bamate (600 mg, yield: 63.4%).

LCMS (ESI): [M+H] ⁺= 605.35

4.

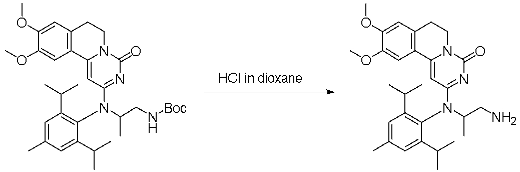

tert-Butyl N-{2-[(2,6-diisopropyl-4-methylphenyl)((9,10-dimethoxy-4-oxo-6H,7H-pyrimido[4,3-a]isoquinolin-2-yl))amino]propyl}carbamate (500 mg, 0.91 mmol, 1.0 equiv) was dissolved in a HCl solution in 1,4-dioxane (4 M, 10 mL), and the mixture was stirred at room temperature for 1 h. The mixture was concentrated under reduced pressure, dissolved in 10 mL water, and extracted with ethyl acetate (3 × 10 mL). The organic layers were washed with 20 mL of saturated aqueous sodium chloride solution, dried over anhydrous Na₂SO₄, filtered, and concentrated under reduced pressure to give 2-((1-aminopropan-2-yl)(2,6-diisopropyl-4-methylphenyl)amino)-9,10-dimethoxy-6H,7H-pyrimido[4,3-a]isoquinolin-4-one (300 mg, yield: 73.3%).

LCMS (ESI): [M+H]⁺ = 505.30

5.

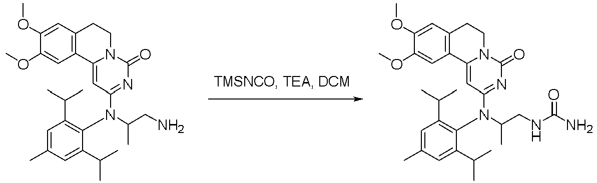

2-((1-Aminopropan-2-yl)(2,6-diisopropyl-4-methylphenyl)amino)-9,10-dimethoxy-6,7-dihydro-4H-pyrimido[6,1-a]isoquinolin-4-one (300 mg, 0.59 mmol, 1.0 equiv), triethylamine (180 mg, 1.8 mmol, 3.0 equiv), and trimethylsilyl isocyanate (103 mg, 1.2 mmol, 2.0 equiv) were dissolved in DCM (10 mL). The mixture was stirred at room temperature for 2 h, concentrated under reduced pressure, and dissolved in 10 mL of water. The resulting mixture was extracted with ethyl acetate (3 × 10 mL). The organic layers were washed with 20 mL of saturated aqueous sodium chloride solution, dried over anhydrous Na₂SO₄, filtered, and concentrated under reduced pressure. The crude product was purified by reversed-phase column chromatography under the following conditions: column: XBridge BEH C18, 5 µm, 30 × 150 mm; mobile phase A: water (10 mmol/L ammonium bicarbonate); mobile phase B: acetonitrile; flow rate: 60 mL/min; gradient: 43% B to 55% B over 8 min; wavelengths: 254/220 nm; RT1: 5.77 min, to give 1-(2-((2,6-diisopropyl-4-methylphenyl)(9,10-dimethoxy-4-oxo-6,7-dihydro-4H-pyrimido[6,1-a]isoquinolin-2-yl)amino)propyl)urea (150 mg, yield: 45.0%).

6.

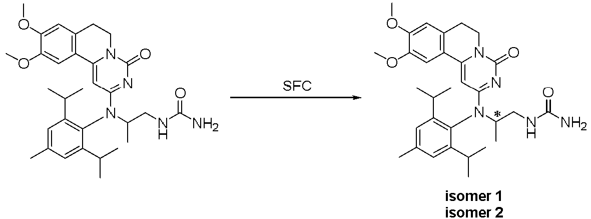

The racemate (150 mg) was purified by Chiral-SFC under the following conditions: column: CHIRALPAK IH, 2 × 25 cm, 5 µm; mobile phase A: methanol/dichloromethane = 1:1-HPLC; mobile phase B: methyl tert-butyl ether (0.1% ethylenediamine)-HPLC; flow rate: 20 mL/min; gradient: isocratic; column temperature (°C): 35; pressure (bar): 100 ; wavelength: 220 nm; RT1 (min): 10; RT2 (min): 5; sample solvent: methanol-HPLC; injection volume: 1 mL, to obtain the products.
**Compound 23-1** (isomer 1, RT1 (min): 10, 16.5 mg, 6.4% yield)
LCMS (ESI): [M+H] ⁺= 548.30
¹H NMR (400 MHz, Methanol-d₄) δ 7.25 - 7.18 (m, 2H), 6.94 (s, 1H), 6.61 (s, 1H), 5.45 (s, 1H), 4.48 (s, 1H), 4.15 - 4.08 (m, 2H), 3.89 (s, 3H), 3.63 (s, 3H), 3.11 - 2.93 (m, 4H), 2.75 (s, 1H), 2.44 (s, 3H), 1.36 - 1.25 (m, 8H), 1.17 (d, *J* = 6.8 Hz, 3H), 1.02 (d, *J* = 6.6 Hz, 4H).
**Compound 23-2** (isomer 2, RT2 (min): 5, 16.42 mg, 6.4% yield)
LCMS (ESI): [M+H] ⁺= 548.30
¹H NMR (400 MHz, Methanol-d₄) δ 7.26 - 7.18 (m, 2H), 6.94 (s, 1H), 6.61 (s, 1H), 5.45 (s, 1H), 4.48 (s, 1H), 4.16 - 4.08 (m, 2H), 3.89 (s, 3H), 3.63 (s, 3H), 3.11 - 2.93 (m, 3H), 2.75 (s, 1H), 2.44 (s, 3H), 1.35 - 1.28 (m, 7H), 1.17 (d, *J* = 6.8 Hz, 3H), 1.02 (d, *J* = 6.6 Hz, 3H).

### Example 24, Compound 24-1, and Compound 24-2

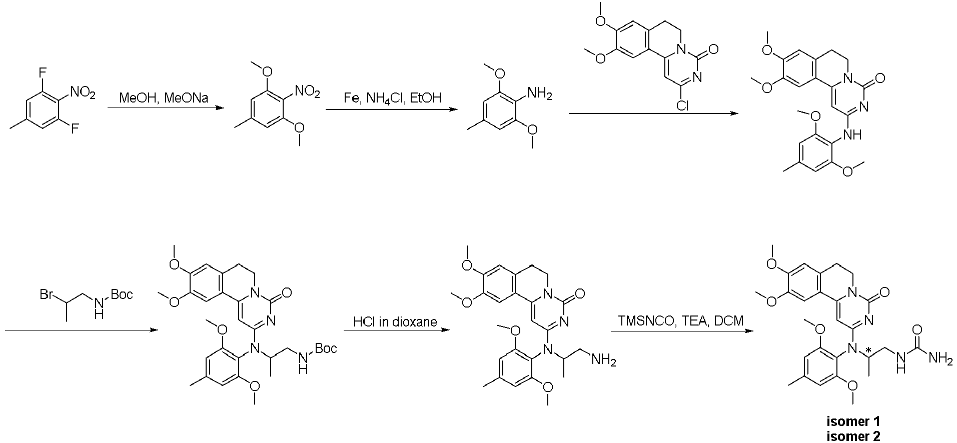

### Preparation steps:

1.

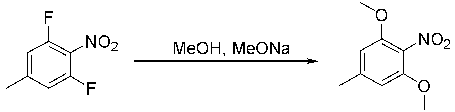

1,3-Difluoro-5-methyl-2-nitrobenzene (200 mg, 1.1 mmol, 1 equiv) was dissolved in MeOH (5 mL) at room temperature under air. A sodium methoxide solution in methanol (3 mL, 60%) was added thereto. A reaction was allowed to proceed at room temperature overnight. The mixture was dried by evaporation under reduced pressure, water (50 mL) was added thereto, and the resultant was extracted with ethyl acetate (2 × 50 mL). The combined organic layers were washed with saturated brine (2 × 100 mL), dried over anhydrous sodium sulfate, filtered, and concentrated under reduced pressure, to give 1,3-dimethoxy-5-methyl-2-nitrobenzene (200 mg, crude product).

2.

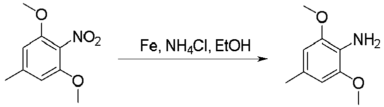

1,3-Dimethoxy-5-methyl-2-nitrobenzene (190 mg, 0.9 mmol, 1 equiv) and H₂O (1 mL) were added to EtOH (5 mL) at room temperature under air. Then NH₄Cl (154 mg, 3 mmol, 3 equiv) and Fe (8.5 mg, 0.2 mmol, 3 equiv) were added thereto. A reaction was carried out at 80°C for 2h. The resulting mixture was filtered, and the filter cake was washed with ethyl acetate (2 × 30 mL). The reactants were quenched with water at room temperature. The resulting mixture was extracted with ethyl acetate (2 × 100 mL). The combined organic layers were washed with saturated brine (2 × 150 mL), dried over anhydrous sodium sulfate, filtered, and concentrated under reduced pressure to give 2,6-dimethoxy-4-methylaniline (150 mg, crude product).

LCMS (ESI): [M+H] ⁺= 168.2

3.

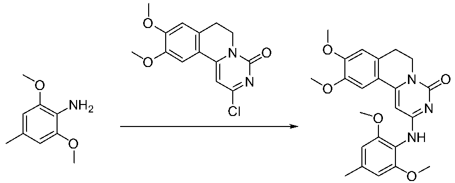

2-Chloro-9,10-dimethoxy-6H,7H-pyrimido[4,3-a]isoquinolin-4-one (273 mg, 0.9 mmol, 1.2 equiv) was dissolved in isopropanol (3 mL) at room temperature under air. 2,6-Dimethoxy-4-methylaniline (152 mg, 0.9 mmol, 1.2 equiv) was then added thereto. The resulting mixture was concentrated under reduced pressure to give 2-[(2,6-dimethoxy-4-methylphenyl)amino]-9,10-dimethoxy-6H,7H-pyrimido[4,3-a]isoquinolin-4-one (300 mg, crude product).

LCMS (ESI): [M+H] ⁺ = 424.2

4.

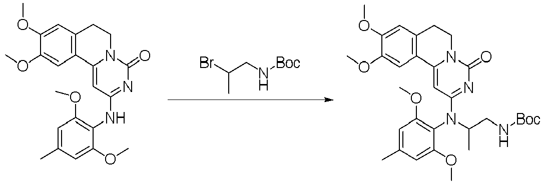

2-[(2,6-Dimethoxy-4-methylphenyl)amino]-9,10-dimethoxy-6H,7H-pyrimido[4,3-a]isoquinolin-4-one (300 mg, 0.7 mmol, 1 equiv), tert-butyl N-(2-bromopropyl)carbamate (168 mg, 0.7 mmol, 1.0 equiv), and Cs₂CO₃ (461 mg, 1.4 mmol, 2.0 equiv) were dissolved in 1,4-dioxane (5 mL). XantPhos (81 mg, 0.1 mmol, 0.1 equiv) and Pd₂(dba)₃ (64 mg, 0.1 mmol, 0.1 equiv) were added sequentially to the mixture. The resulting mixture was stirred at 100 °C under a nitrogen atmosphere overnight. The filtrate was concentrated under reduced pressure, and the residue was purified by reversed-phase flash chromatography under the following conditions: column: C18 silica gel; mobile phase: acetonitrile in water (10 mmol/L NH₄HCO₃); gradient: 10% to 50% acetonitrile over 10 min; detector: UV, 254 nm. Finally, tert-butyl N-{2-[(2,6-dimethoxy-4-methylphenyl)((9,10-dimethoxy-4-oxo-6H,7H-pyrimido[4,3-a]isoquinolin-2-yl))amino]propyl}carbamate (270 mg, yield: 59%) was obtained.

LCMS (ESI): [M+H] ⁺ = 581.2

5.

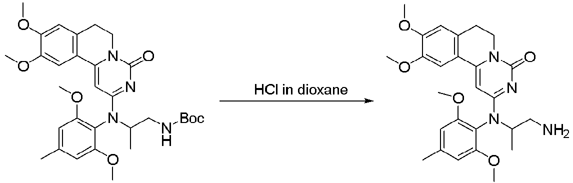

tert-Butyl N-{2-[(2,6-dimethoxy-4-methylphenyl)((9,10-dimethoxy-4-oxo-6H,7H-pyrimido[4,3-a]isoquinolin-2-yl))amino]propyl}carbamate (300 mg, 0.6 mmol, 1.0 equiv) was dissolved in 1,4-dioxane (2 mL) at room temperature under a nitrogen atmosphere, and a HCl solution in 1,4-dioxane (2 mL) was added. The mixture was stirred at room temperature under a nitrogen atmosphere for 1.5 h. The reaction was quenched with water at room temperature. The mixture was neutralized to pH=9 with saturated sodium carbonate solution. The resulting mixture was extracted with ethyl acetate (2 × 50 mL). The combined organic layers were washed with saturated brine (2 × 100 mL), dried over anhydrous sodium sulfate, filtered, and concentrated under reduced pressure. The residue was purified by Prep-TLC (CH₂Cl₂ / MeOH = 20/1), to give 2-[(1-aminopropan-2-yl)(2,6-dimethoxy-4-methylphenyl)amino]-9,10-dimethoxy-6H,7H-pyrimido[4,3-a]isoquinolin-4-one (150 mg, yield: 49%).

LCMS (ESI): [M+H] ⁺ = 481.2

6.

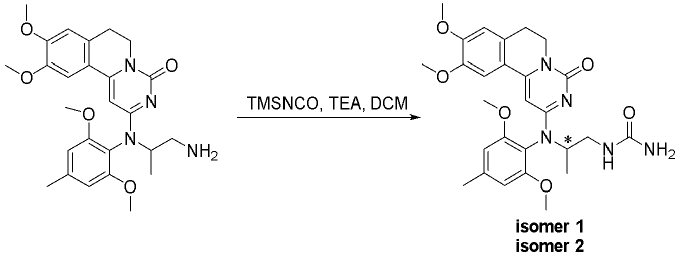

2-[(1-Aminopropan-2-yl)(2,6-dimethoxy-4-methylphenyl)amino]-9,10-dimethoxy-6H,7H-pyrimido[4,3-a]isoquinolin-4-one (400 mg, 0.8 mmol, 1.0 equiv) and TEA (0.4 mL, 2.5 mmol, 3.0 equiv) were dissolved in DCM (4 mL). Trimethylsilyl isocyanate (191 mg, 1.6 mmol, 2.0 equiv) was added to the solution. The resulting mixture was stirred at room temperature for 2 h under a nitrogen atmosphere, and the reaction mixture was concentrated under reduced pressure. The crude product (420 mg) was purified by HPLC under the following conditions: column: Sunfire C18, 5 µm, 30 mm × 4.5 mm; mobile phase A: water (0.1% FA); mobile phase B: acetonitrile; flow rate: 60 mL/min; gradient: 51% B to 74% B over 10 min; wavelength: 254/220 nm; RT1 (min): 6.03; 6.62, to give 1-(2-((2,6-dimethoxy-4-methylphenyl)(9,10-dimethoxy-4-oxo-6,7-dihydro-4H-pyrimido[6,1-a]isoquinolin-2-yl)amino)propyl)urea (130 mg).

The product was further purified by Chiral-HPLC under the following conditions: column: JW-CHIRALPAK IG-3, 3.0 × 50 mm, 3 µm; mobile phase A: MeOH: DCM = 1:1-HPLC; mobile phase B: MtBE (0.1% DEA)-HPLC grade; flow rate: 20 mL/min; gradient: isocratic; wavelength: 220 nm; RT1 (min): 12; RT2 (min): 16.5; sample solvent: MeOH/DCM = 1:1-HPLC; injection volume: 0.7 mL; number of runs: 5, to give the products.
**Compound 24-1** (isomer 1, RT1 (min): 12, 30.12 mg, 6.8% yield)
LCMS (ESI): [M+H] ⁺= 524.25
¹H NMR (400 MHz, Methanol-d₄) δ 6.92 (s, 1H), 6.72 - 6.66 (m, 3H), 5.53 (s, 1H), 4.11 - 4.05 (m, 2H), 3.89 (s, 3H), 3.81 (d, *J* = 10.1 Hz, 6H), 3.69 (s, 3H), 2.95 (t, *J=* 6.5 Hz, 2H), 2.47 (s, 3H).
**Compound 24-2** (isomer 2, RT2 (min): 16.5, 44.03 mg, 10% yield)
LCMS (ESI): [M+H] ⁺= 524.25
¹H NMR (400 MHz, Methanol-d₄) δ 6.92 (s, 1H), 6.72 - 6.66 (m, 3H), 5.53 (s, 1H), 4.11 - 4.05 (m, 2H), 3.89 (s, 3H), 3.81 (d, *J* = 10.1 Hz, 6H), 3.69 (s, 3H), 2.95 (t, *J=* 6.5 Hz, 2H), 2.47 (s, 3H).

### Example 25, Compound 25-1, and Compound 25-2

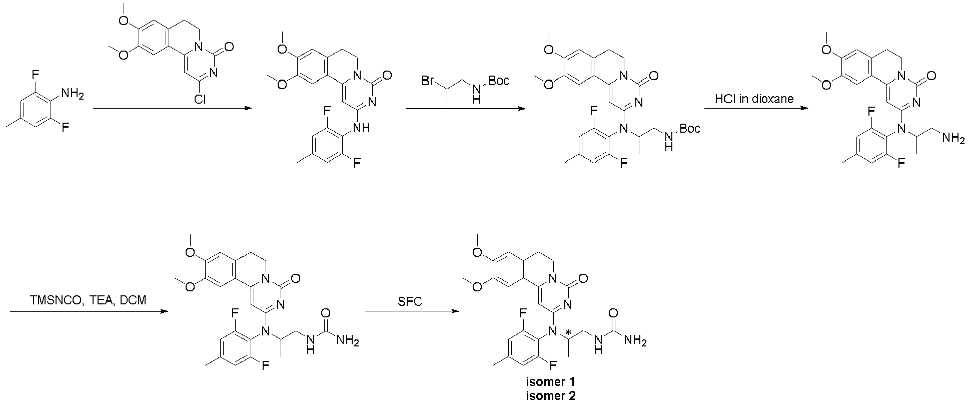

### Preparation steps:

**1.**

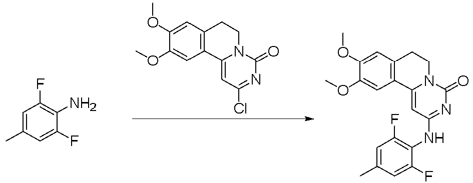

A mixture of 2,6-difluoro-4-methylaniline (1.0 g, 6.9 mmol, 1 equiv) and 2-chloro-9,10-dimethoxy-6H,7H-pyrimido[4,3-a]isoquinolin-4-one (2.6 g, 9.0 mmol, 1.3 equiv) in IPA (40 mL) was stirred at 90°C under a nitrogen atmosphere overnight. Then the resulting mixture was concentrated under reduced pressure, basified to pH=8 with saturated NaHCO₃, and extracted with EtOEt (3 × 50 mL). The combined organic layers were washed with brine (2 × 100 mL), dried over anhydrous Na₂SO₄, filtered, and concentrated under reduced pressure. The residue was purified by silica gel column chromatography, eluting with CH₂Cl₂/MeOH (80/1~60/1), to give 2-[(2,6-difluoro-4-methylphenyl)amino]-9,10-dimethoxy-6H,7H-pyrimido[4,3-a]isoquinolin-4-one (2.0 g, yield: 71.6%).

LCMS (ESI): [M+H] ⁺= 400.15

**2.**

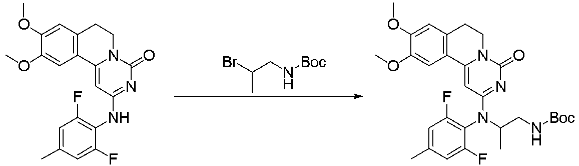

2-[(2,6-Difluoro-4-methylphenyl)amino]-9,10-dimethoxy-6H,7H-pyrimido[4,3-a]isoquinolin-4-one (440 mg, 1.1 mmol, 1 equiv) and tert-butyl N-(2-bromopropyl)carbamate (288.5 mg, 1.2 mmol, 1.1 equiv) were dissolved in DMF (15 mL) under stirring. The mixture was stirred at 100°C overnight under a nitrogen atmosphere. The resulting mixture was concentrated under reduced pressure. The residue was purified by silica gel column chromatography eluting with CH₂Cl₂ / MeOH (10/1), to give tert-butyl N-{2-[(2,6-difluoro-4-methylphenyl)((9,10-dimethoxy-4-oxo-6H,7H-pyrimido[4,3-a]isoquinolin-2-yl))amino]propyl}carbamate (200 mg, yield: 32.6%).

LCMS (ESI): [M+H] ⁺= 557.25

**3.**

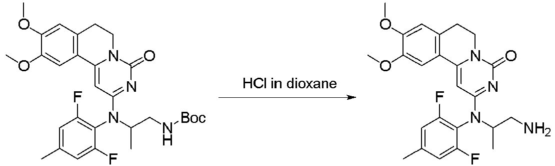

The N-{2-[(2,6-difluoro-4-methylphenyl)({9,10-dimethoxy-4-oxo-6H,7H-pyrimido[4,3-a]isoquinolin-2-yl})amino]propyl}carbamate (200 mg, 0.359 mmol, 1 equiv) was stirred in 1,4-dioxane (15 mL) at room temperature under a nitrogen atmosphere for 3 h. Then the resulting mixture was concentrated under reduced pressure, to give 2-[(1-aminopropan-2-yl)(2,6-difluoro-4-methylphenyl)amino]-9,10-dimethoxy-6H,7H-pyrimido[4,3-a]isoquinolin-4-one (150 mg, yield: 91.45%). The resulting crude product was used directly in the next step without further purification.

LCMS (ESI): [M+H] ⁺ = 457.2

**4.**

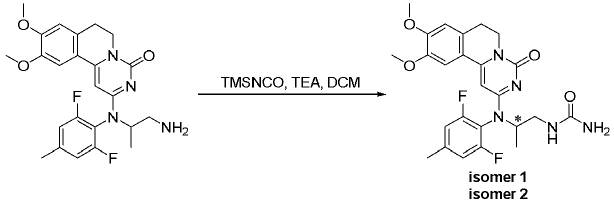

2-[(1-Aminopropan-2-yl)(2,6-difluoro-4-methylphenyl)amino]-9,10-dimethoxy-6H,7H-pyrimido[4,3-a]isoquinolin-4-one (50 mg, 0.11 mmol, 1.0 equiv) and trimethylsilyl isocyanate (12.6 mg, 0.11 mmol, 1.0 equiv) were dissolved in DCM (5 mL) under stirring at room temperature overnight under a nitrogen atmosphere. Then the resulting mixture was concentrated under reduced pressure. The crude product (60 mg) was purified by Prep-HPLC under the following conditions: column: XSelect CSH Fluoro-Phenyl, 5 µm, 19 × 250 mm; mobile phase A: water (0.1% FA); mobile phase B: ACN; flow rate: 25 mL/min; gradient: 42% B to 65% B over 10 min; wavelengths: 254/220 nm; RT1 (min): 5.67, to give (2R)-2-((2,6-difluoro-4-methylphenyl)(9,10-dimethoxy-4-oxo-6H,7H-pyrimido[4,3-a]isoquinolin-2-yl)amino)propylurea (50 mg).

The product was further purified by Chiral-HPLC under the following conditions: column: CHIRALPAK IH, 2 × 25 cm, 5 µm; mobile phase A: MeOH/DCM = 1:1-HPLC; mobile phase B: MtBE (0.1% DEA)-HPLC; flow rate: 20 mL/min; gradient: isocratic; wavelength: 220 nm; RT1 (min): 11.2; RT2 (min): 15.0; sample solvent: MeOH/DCM = 1:1-HPLC; injection volume: 1.5 mL; number of runs: 5, to give the products.
**Compound 25-1** (isomer 1, RT1 (min): 11.2, 13.41 mg, 24.49% yield)
LCMS (ESI): [M+H] ⁺= 500.25
¹H NMR (400 MHz, Methanol-d₄) δ 6.90 (s, 1H), 6.87 (s, 1H), 6.85 (s, 1H), 6.83 (s, 1H), 5.70 (t, *J* = 1.7 Hz, 1H), 5.63 (s, 1H), 4.00 (t, *J* = 6.3 Hz, 2H), 3.96 - 3.90 (m, 1H), 3.88 (s, 3H), 3.73 (s, 3H), 3.65 - 3.56 (m, 1H), 2.95 (t, *J* = 6.2 Hz, 2H), 2.35 (s, 3H), 1.54 (d, *J=* 6.9 Hz, 3H).
**Compound 25-2** (isomer 2, RT2 (min): 15.0, 15.09 mg, 21.59% yield)
LCMS (ESI): [M+H] ⁺= 500.25
¹H-NMR (400 MHz, Methanol-d₄) δ 6.89 (s, 1H), 6.87 (s, 1H), 6.86 - 6.84 (m, 1H), 6.84 - 6.81 (m, 1H), 5.70 (t, *J* = 1.6 Hz, 1H), 5.63 (s, 1H), 4.00 (t, *J =* 6.3 Hz, 2H), 3.96 - 3.90 (m, 1H), 3.88 (s, 3H), 3.73 (s, 3H), 2.97 - 2.93 (m, 2H), 2.35 (s, 3H), 1.54 (d, *J=* 6.9 Hz, 3H).

### Example 26, Compound 26

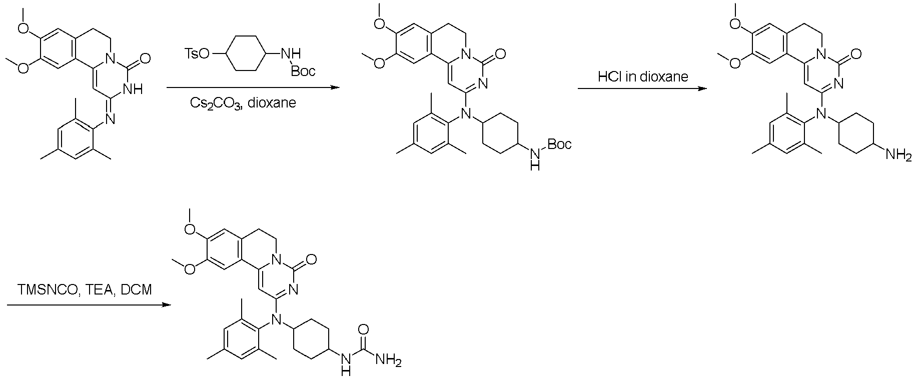

### Preparation steps

**1.**

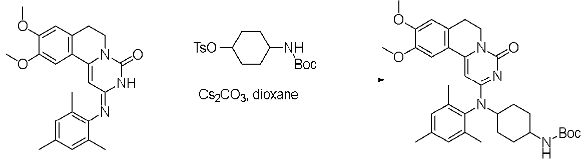

(2E)-9,10-Dimethoxy-2-[(2,4,6-trimethylphenyl)imino]-3H,6H, 7H-pyrimido[4,3-a]isoquinolin-4-one (1.0 g, 2.6 mmol, 1.0 equiv), dioxane (100 mL), cesium carbonate (1.7 g, 5.1 mmol, 2.0 equiv), and tert-butyl N-{4-[(4-methylbenzenesulfonyl)oxy]cyclohexyl}carbamate (1.4 g, 3.8 mmol, 1.5 equiv) were added sequentially to a single-neck flask. A reaction was carried out at 100 °C overnight. The mixture was filtered, and the filtrate was concentrated to dryness. The resulting residue was purified by reversed-phase column chromatography under the following conditions: column: C18; mobile phase: water and acetonitrile; gradient: 10% to 100% acetonitrile over 30 min; detector: UV at 220 nm, to give tert-butyl (4-((9,10-dimethoxy-4-oxo-6,7-dihydro-4H-pyrimido[6,1-a]isoquinolin-2-yl)(2,4,6-trimethylphenyl)amino)cyclohexyl)carbamate (110 mg, yield: 7.3%).

LCMS (ESI, m/z): [M+H] ⁺ = 589.3

2.

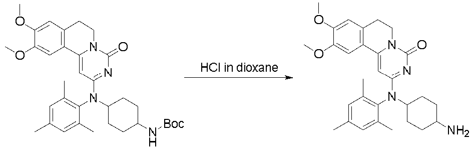

tert-Butyl (4-((9,10-dimethoxy-4-oxo-6,7-dihydro-4H-pyrimido[6,1-a]isoquinolin-2-yl)(2,4,6-trimethylphenyl)amino)cyclohexyl)carbamate (100 mg, 0.2 mmol, 1.0 equiv) and a HCl solution in 1,4-dioxane (4 M, 15 mL) were added sequentially to a single-neck flask. A reaction was carried out at room temperature for 2 h. The solvent was removed by concentration, to give 2-[(4-aminocyclohexyl)(2,4,6-trimethylphenyl)amino]-9,10-dimethoxy-6H,7H-pyrimido[4,3-a]isoquinolin-4-one (80 mg, yield: 96.4%).

LCMS (ESI, m/z): [M+H] ⁺= 489.3

**3.**

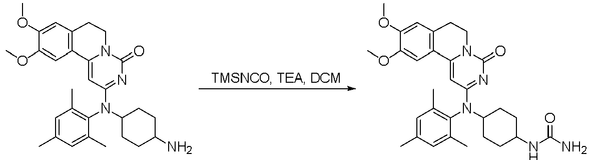

2-[(4-Aminocyclohexyl)(2,4,6-trimethylphenyl)amino]-9,10-dimethoxy-6H,7H-pyrimido[4,3-a]isoquinolin-4-one (140 mg, 0.3 mmol, 1.0 equiv) was dissolved in solvent DCM (8 mL). Triethylamine (145 mg, 1.5 mmol, 5.0 equiv) and trimethylsilyl isocyanate (40 mg, 0.4 mmol, 1.2 equiv) were added thereto at room temperature. Then the mixture was stirred at room temperature for 3 h. The crude product was purified by Prep-HPLC under the following conditions: column: Xbridge BEH Shield RP18, 5 µm, 19 × 250 mm; mobile phase A: water (10 mmol/L ammonium bicarbonate); mobile phase B: acetonitrile; flow rate: 25 mL/min; gradient: 39% B to 4% B over 10 min; wavelengths: 254/220 nm; RT1 (min): 9.4 min, to give 4-((9,10-dimethoxy-4-oxo-6H,7H-pyrimido[4,3-a]isoquinolin-2-yl)(2,4,6-trimethylphenyl)amino)cyclohexylurea (35.71 mg, yield: 22.81%).
LCMS (ESI): [M+H] + = 532.25
¹H NMR (400 MHz, Methanol-d₄) δ 7.10 (s, 2H), 6.92 (s, 1H), 6.62 (s, 1H), 5.39 (s, 1H), 4.18 - 4.03 (m, 3H), 3.88 (s, 4H), 3.66 (s, 3H), 2.96 (t, *J* = 6.4 Hz, 2H), 2.36 (s, 3H), 2.21 (s, 6H), 2.05 - 1.92 (m, 4H), 1.88 - 1.80 (m, 2H), 1.75 - 1.62 (m, 2H).

### Example 27, Compound 27

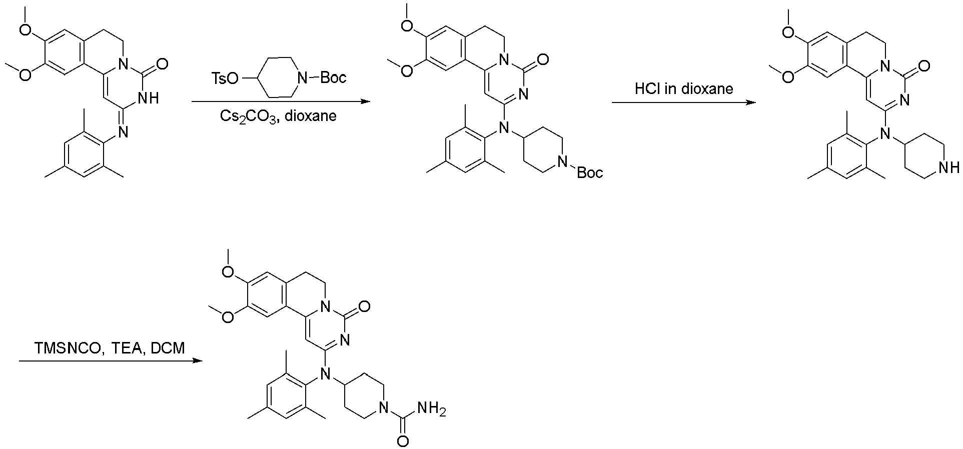

**Preparation steps**

**1.**

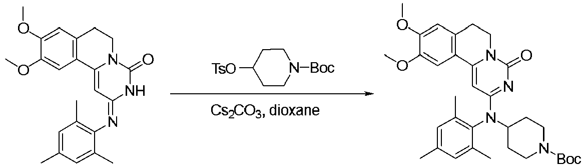

(2E)-9,10-Dimethoxy-2-[(2,4,6-trimethylphenyl)imino]-3H,6H,7H-pyrimido[4,3-a]isoquinolin-4-one (1.0 g, 2.6 mmol, 1.0 equiv), 1,4-dioxane (100 mL), cesium carbonate (2.5 g, 7.7 mmol, 3.0 equiv), and tert-butyl 4-[(4-methylbenzenesulfonyl)oxy]piperidine-1-carboxylate (1.0 g, 2.8 mmol, 1.1 equiv) were added sequentially to a single-neck flask. A reaction was carried out at 100 °C overnight. The mixture was filtered, and the filtrate was concentrated. The residue was purified by reversed-phase column chromatography under the following conditions: mobile phase: water and acetonitrile; gradient: 10% to 100% over 30 min; detector: UV at 220 nm, to give tert-butyl 4-((9,10-dimethoxy-4-oxo-6,7-dihydro-4H-pyrimido[6,1-a]isoquinolin-2-yl)(2,4,6-trimethylphenyl)amino)piperidine-1-carboxylate (120 mg, yield: 8.17%).

LCMS (ESI, m/z): [M+H] ⁺= 575.3

**2.**

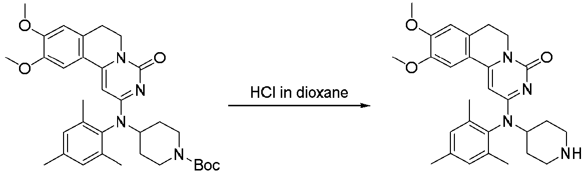

tert-Butyl 4-((9,10-dimethoxy-4-oxo-6,7-dihydro-4H-pyrimido[6,1-a]isoquinolin-2-yl)(2,4,6-trimethylphenyl)amino)piperidine-1-carboxylate (100 mg, 0.2 mmol, 1.0 equiv) and HCl in 1,4-dioxane (17 mL) were added sequentially to a single-neck flask. A reaction was carried out at room temperature for 2 h. The solvent was removed by concentration, to give 2-(2,4,6-trimethylphenyl(piperidin-4-yl)amino)-9,10-dimethoxy-6,7-dihydro-4H-pyrimido[6,1-a]isoquinolin-4-one (80 mg, yield: 96.9%).

LCMS (ESI, m/z): [M+H] ⁺= 475.3

3.

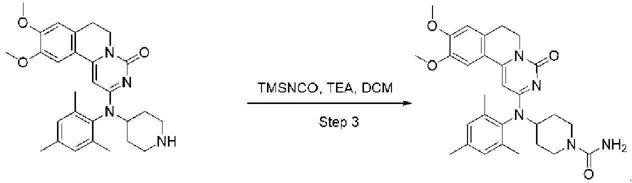

To a solution of 2-(2,4,6-trimethylphenyl(piperidin-4-yl)amino)-9,10-dimethoxy-6,7-dihydro-4H-pyrimido[6,1-a]isoquinolin-4-one (1050 mg, 2.2 mmol, 1.0 equiv) in dichloromethane (20 mL), triethylamine (640 mg, 6.3 mmol, 3.0 equiv) and trimethylsilyl isocyanate (364 mg, 3.2 mmol, 1.5 equiv) were added. The mixture was stirred at room temperature for 2 h. The mixture was concentrated by rotary evaporation under reduced pressure, and the residue was purified by reversed-phase column chromatography under the following conditions: column: XBridge BEH Shield RP18, 5 µm, 19 × 250 mm; mobile phase A: water (10 mmol/L ammonium bicarbonate); mobile phase B: acetonitrile; flow rate: 25 mL/min; gradient: 35% B to 40% B over 10 min; wavelengths: 254/220 nm; RT1 (min): 8.57, to give 4-([(2E)-9,10-dimethoxy-4-oxo-6H,7H-pyrimido[4,3-a]isoquinolin-2-yl](2,4,6-trimethylphenyl)amino)piperidine-1-carboxamide (39.74 mg, yield: 3.63%).
LCMS (ESI): [M+H] ⁺= 518.40
¹H NMR (400 MHz, Methanol-d₄) δ 7.10 (s, 2H), 6.92 (s, 1H), 6.62 (s, 1H), 5.39 (s, 1H), 4.73 - 4.62 (m, 1H), 4.18 - 3.97 (m, 4H), 3.88 (s, 3H), 3.66 (s, 3H), 3.02 - 2.90 (m, 4H), 2.36 (s, 3H), 2.17 (s, 8H), 1.43 - 1.29 (m, 2H).

### Example 28, Compound 28

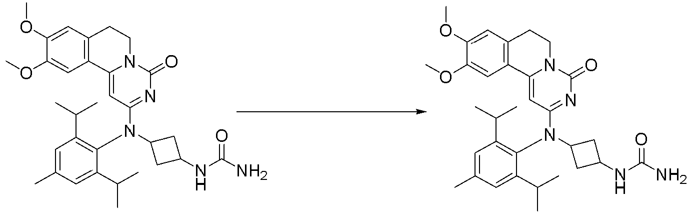

A crude product (150 mg) was purified by Chiral-SFC under the following conditions: column: CHIRALPAK IH, 2 × 25 cm, 5 µm; mobile phase A: methanol/dichloromethane= 1:1-HPLC; mobile phase B: tert-butyl methyl ether (0.1% ethylenediamine)-HPLC; flow rate: 20 mL/min; gradient: isocratic; wavelength: 220 nm; RT1 (min): 8; RT2 (min): 13.0; sample solvent: methanol-HPLC; injection volume: 1 mL, to give 1-(3-((2,6-diisopropyl-4-methylphenyl)(9,10-dimethoxy-4-oxo-6,7-dihydro-4H-pyrimido[6,1-a]isoquinolin-2-yl)amino)cyclobutyl)urea (13.00 mg, 14.9% yield).
LCMS (ESI): [M+H]⁺ = 560.30
¹H NMR (400 MHz, Methanol-d₄) δ 7.21 (s, 2H), 6.93 (s, 1H), 6.64 (s, 1H), 5.46 (s, 1H), 4.51 - 4.44 (m, 1H), 4.14 - 4.06 (m, 2H), 3.97 - 3.88 (m, 1H), 3.89 (s, 3H), 3.64 (s, 3H), 2.94 - 2.90 (m, 4H), 2.74 - 2.78 (m, 2H), 2.44 (s, 3H), 2.20 - 2.08 (m, 2H), 1.30 (d, *J* = 6.8 Hz, 7H), 1.11 (d, *J* = 6.8 Hz, 6H).

### Example 29, Compound 29

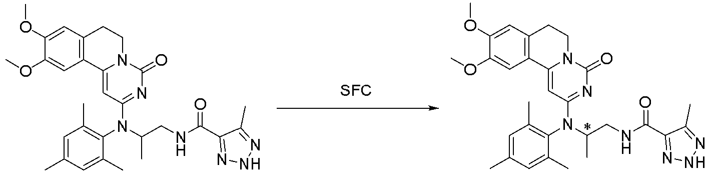

A crude product (30 mg) was purified by Chiral-HPLC under the following conditions: column: CHIRALPAK IH, 3 × 25 cm, 5 µm; mobile phase A: CO₂; mobile phase B: methanol (0.1% diethylamine); flow rate: 85 mL/min; gradient: isocratic 35% B; column temperature (°C): 25 °C; back pressure (bar): 100; wavelength: 254 nm; RT1 (min): 2.85; RT2 (min): 4.5; sample solvent: methanol/dichloromethane = 1:1-HPLC; injection volume: 1 mL, to obtain the product.
**Compound 29** (isomer 1, RT1 (min): 2.85, 3.2 mg, 10.3% yield)
LCMS (ESI, m/z): [M+H] ⁺= 558.35
¹H NMR (400 MHz, Methanol-d₄) δ 7.17 - 7.10 (m, 2H), 6.93 (s, 1H), 6.65 (s, 1H), 5.43 (s, 1H), 4.78 - 4.69 (m, 1H), 4.20 - 4.04 (m, 2H), 3.97 - 3.77 (m, 5H), 3.67 (s, 3H), 2.98 (t, *J =* 6.5 Hz, 2H), 2.59 - 2.52 (m, 3H), 2.38 (s, 3H), 2.27 (s, 3H), 2.13 (s, 3H), 1.11 (d, *J* = 6.9 Hz, 3H).

### Example 30, Compound 30

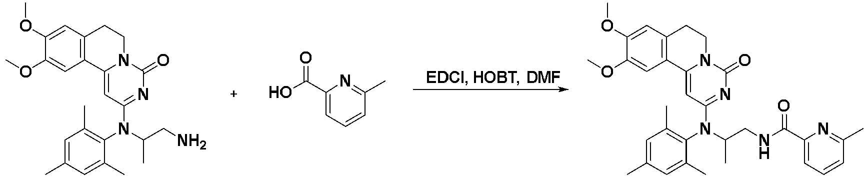

2-((1-Aminopropan-2-yl)(2,4,6-trimethylphenyl)amino)-9,10-dimethoxy-6,7-dihydro-4H-pyrimido[6,1-a]isoquinolin-4-one (100 mg, 0.22 mmol), 6-methylpyridine-2-carboxylic acid (45.3 mg, 0.33 mmol), EDCI (63.3 mg, 0.33 mmol), HOBT (44.6 mg, 0.33 mmol), and DMF (2 mL) were added to a 25 mL single-neck flask at room temperature. The mixture was stirred at room temperature for 24 h. Upon completion of a reaction, the reaction mixture was filtered and purified by reverse-phase Flash chromatography (mobile phase A: 0.5% aqueous FA; mobile phase B: acetonitrile; flow rate: 40 mL/min; gradient: 0-55% B over 20 min; product eluted at 50% B; wavelength: 254/220 nm) to give the product (19.2 mg, 10.3%).
LC-MS (ESI): [M+H]⁺= 568.2
¹H NMR (400 MHz, CDCl₃) 8.76-8.74 (m, 1H), 7.86 (d, J = 8.0 Hz, 1H), 7.63 (t, J = 12.0 Hz, 1H), 7.19-7.16 (m, 1H), 6.91 (d, J = 8.0 Hz, 1H), 6.62 (s, 1H),6.52 (s, 1H), 5.22 (s, 1H), 4.96-4.95 (m, 1H), 4.16 - 4.10 (m, 2H), 3.83 (s, 3H), 3.82-3.75 (m, 2H), 3.63 (s, 3H), 2.84 (t, J = 8.0 Hz, 2H), 2.54 (s, 3H), 2.26 (s, 3H), 2.17 (s, 3H), 2.05 (s, 3H), 1.05 (d, J = 8.0 Hz, 3H).

### Example 31, Compound 31

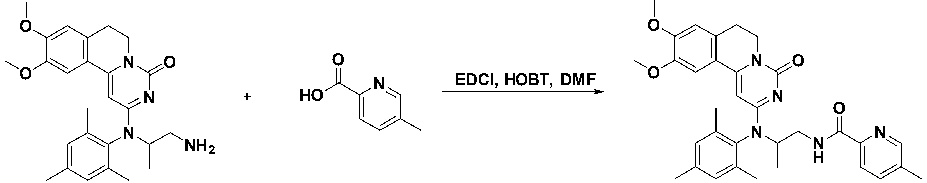

2-((1-Aminopropan-2-yl)(2,4,6-trimethylphenyl)amino)-9,10-dimethoxy-6,7-dihydro-4H-pyrimido[6,1-a]isoquinolin-4-one (150 mg, 0.33 mmol), 5-methylpyridine-2-carboxylic acid (68.6 mg, 0.50 mmol), EDCI (95.9 mg, 0.50 mmol), HOBT (67.6 mg, 0.50 mmol), and DMF (3 mL) were added to a 25 mL single-neck flask at room temperature. The mixture was stirred at room temperature for 24 h. Upon completion of a reaction, the reaction mixture was filtered and purified by reverse-phase Flash chromatography (mobile phase A: 0.5% aqueous FA; mobile phase B: acetonitrile; flow rate: 40 mL/min; gradient: 0-55% B over 20 min; product eluted at 50% B; wavelength: 254/220 nm) to give the product (37.5 mg, 19.7%).
LC-MS (ESI): [M+H]⁺= 568.2
¹H NMR (400 MHz, CDCl₃) 8.81 (s, 1H), 8.35 (s, 1H), 7.95 (d, J = 8.0 Hz, 1H), 7.54-7.51 (m, 1H), 6.93 (d, J = 8.0 Hz, 1H), 6.62 (s, 1H), 6.52 (s, 1H), 5.21 (s, 1H), 4.92-4.87 (m, 1H), 4.17 - 4.07 (m, 1H), 3.83 (s, 3H), 3.82-3.75 (m, 2H), 3.63 (s, 3H), 2.84 (t, J = 8.0 Hz, 2H), 2.31 (s, 3H), 2.25 (s, 3H), 2.17 (s, 3H), 2.05 (s, 3H), 1.09 (d, J = 8.0 Hz, 3H).

### Example 32, Compound 32

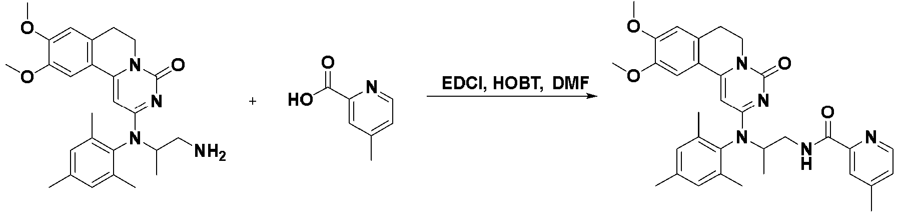

2-((1-Aminopropan-2-yl)(2,4,6-trimethylphenyl)amino)-9,10-dimethoxy-6,7-dihydro-4H-pyrimido[6,1-a]isoquinolin-4-one (150 mg, 0.33 mmol), 4-methylpyridine-2-carboxylic acid (68.6 mg, 0.50 mmol), EDCI (95.9 mg, 0.50 mmol), HOBT (67.6 mg, 0.50 mmol), and DMF (3 mL) were added to a 25 mL single-neck flask at room temperature. The mixture was stirred at room temperature for 24 h. Upon completion of a reaction, the reaction mixture was filtered and purified by reverse-phase Flash chromatography (mobile phase A: 0.5% aqueous FA; mobile phase B: acetonitrile; flow rate: 40 mL/min; gradient: 0-55% B over 20 min; product eluted at 50% B; wavelength: 254/220 nm) to give the product (32.3 mg, 17.3%).
LC-MS (ESI): [M+H]⁺= 568.2
¹H NMR (400 MHz, CDCl₃) 8.87 (s, 1H), 8.37 (d, J = 8.0 Hz, 1H), 8.21 (s, 1H), 7.91 (s, 1H), 7.15 (d, J = 8.0 Hz, 1H), 6.91 (d, J = 12Hz, 1H), 6.62 (s, 1H), 6.51 (s, 1H), 5.23 (s, 1H), 4.87-4.82 (m, 1H), 4.19 - 4.07 (m, 2H), 3.83 (s, 3H), 3.82-3.75 (m, 2H), 3.64 (s, 3H), 2.85 (t, J = 8.0 Hz, 2H), 2.34 (s, 3H), 2.26 (s, 3H), 2.17 (s, 3H), 1.98 (s, 3H), 1.07 (d, J = 4.0 Hz, 3H).

### Example 33, Compound 33

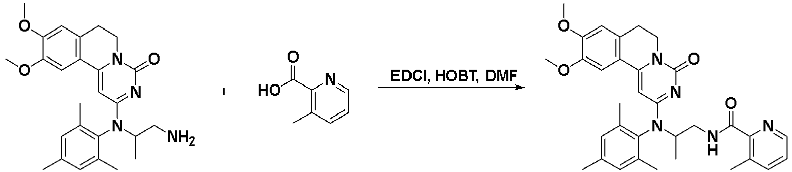

2-((1-Aminopropan-2-yl)(2,4,6-trimethylphenyl)amino)-9,10-dimethoxy-6,7-dihydro-4H-pyrimido[6,1-a]isoquinolin-4-one (150 mg, 0.33 mmol), 3-methylpyridine-2-carboxylic acid (68.6 mg, 0.50 mmol), EDCI (95.9 mg, 0.50 mmol), HOBT (67.6 mg, 0.50 mmol), and DMF (3 mL) were added to a 25 mL single-neck flask at room temperature. The mixture was stirred at room temperature for 24 h. Upon completion of a reaction, the reaction mixture was filtered and purified by reverse-phase Flash chromatography (mobile phase A: 0.5% aqueous FA; mobile phase B: acetonitrile; flow rate: 40 mL/min; gradient: 0-55% B over 20 min; product eluted at 50% B; wavelength: 254/220 nm) to give the product (12.8 mg, 6.8%).
LC-MS (ESI): [M+H]⁺= 568.2
¹H NMR (400 MHz, CDCl₃) 8.86 (s, 1H), 8.35 (d, J = 4.0 Hz, 1H), 7.47 (d, J = 8.0 Hz, 1H), 7.22-7.20 (m, 1H), 6.91 (d, J = 8.0 Hz, 1H), 6.62 (s, 1H), 6.52 (s, 1H), 5.22 (s, 1H), 4.96-4.91 (m, 1H), 4.19 - 4.03 (m, 2H), 3.83 (s, 3H), 3.82-3.75 (m, 2H), 3.63 (s, 3H), 2.84 (t, J = 8.0 Hz, 2H), 2.64 (s, 3H), 2.26 (s, 3H), 2.18 (s, 3H), 2.02 (s, 3H), 1.08 (d, J = 4.0 Hz, 3H).

### Example 34, Compound 34

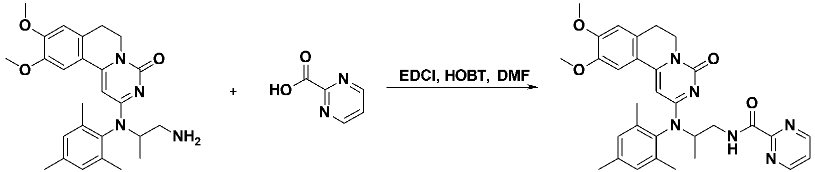

2-((1-Aminopropan-2-yl)(2,4,6-trimethylphenyl)amino)-9,10-dimethoxy-6,7-dihydro-4H-pyrimido[6,1-a]isoquinolin-4-one (100 mg, 0.22 mmol), pyrimidine-2-carboxylic acid (40.9 mg, 0.33 mmol), EDCI (63.3 mg, 0.33 mmol), HOBT (44.6 mg, 0.33 mmol), and DMF (2 mL) were added to a 25 mL single-neck flask at room temperature. The mixture was stirred at room temperature for 24 h. Upon completion of a reaction, the reaction mixture was filtered and purified by reverse-phase Flash chromatography (mobile phase A: 0.5% aqueous FA; mobile phase B: acetonitrile; flow rate: 40 mL/min; gradient: 0-55% B over 20 min; product eluted at 50% B; wavelength: 254/220 nm) to give the product (20 mg, 10.9%).
LC-MS (ESI): [M+H]⁺= 555.2
¹H NMR (400 MHz, DMSO-d6) 9.56 (t, J = 8.0 Hz, 1H), 9.03-9.00 (m, 2H), 8.16 (s, 1H), 7.87 (s, 1H), 7.74-7.71 (m, 1H), 7.12-7.10 (m, 2H), 7.02 (s, 1H), 4.74 - 4.70 (m, 1H), 4.16 (s, 3H), 4.15-4.10 (m, 2H), 3.98-3.95 (m, 2H), 3.89 (s, 3H), 3.05-3.00 (m, 2H), 2.31 (s, 3H), 2.19 (s, 3H), 2.03 (s, 3H), 0.83 (d, J = 8.0 Hz, 3H).

### Example 35, Compound 35

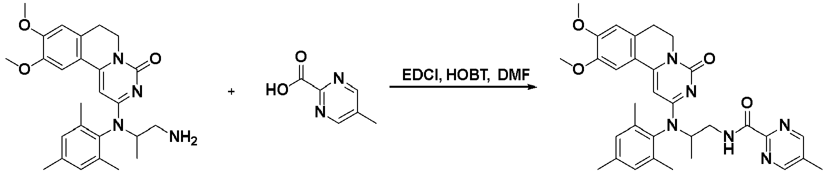

2-((1-Aminopropan-2-yl)(2,4,6-trimethylphenyl)amino)-9,10-dimethoxy-6,7-dihydro-4H-pyrimido[6,1-a]isoquinolin-4-one (100 mg, 0.22 mmol), 5-methylpyrimidine-2-carboxylic acid (45.6 mg, 0.33 mmol), EDCI (63.3 mg, 0.33 mmol), HOBT (44.6 mg, 0.33 mmol), and DMF (2 mL) were added to a 25 mL single-neck flask at room temperature. The mixture was stirred at room temperature for 24 h. Upon completion of a reaction, the reaction mixture was filtered and purified by reverse-phase Flash chromatography (mobile phase A: 0.5% aqueous FA; mobile phase B: acetonitrile; flow rate: 40 mL/min; gradient: 0-55% B over 20 min; product eluted at 50% B; wavelength: 254/220 nm) to give the product (27.6 mg, 14.7%).
LC-MS (ESI): [M+H]⁺= 569.2
¹H NMR (400 MHz, CDCl₃) 9.28 (s, 1H), 8.64 (s, 1H), 6.91 (d, J = 8.0 Hz, 1H), 6.62 (s, 1H), 6.51 (s, 1H), 5.22 (s, 1H), 4.93-4.90 (m, 1H), 4.13 - 4.10 (m, 2H), 3.83 (s, 3H), 3.82-3.75 (m, 2H), 3.63 (s, 3H), 2.85-2.83 (m, 2H), 2.31 (s, 3H), 2.26 (s, 3H), 2.16 (s, 3H), 2.04 (s, 3H), 1.05 (d, J = 8.0 Hz, 3H).

### Example 36, Compound 36

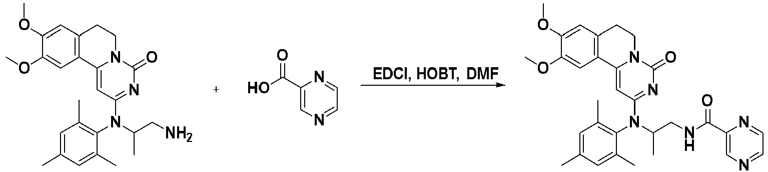

2-((1-Aminopropan-2-yl)(2,4,6-trimethylphenyl)amino)-9,10-dimethoxy-6,7-dihydro-4H-pyrimido[6,1-a]isoquinolin-4-one (100 mg, 0.22 mmol), pyrazine-2-carboxylic acid (40.9 mg, 0.33 mmol), EDCI (63.3 mg, 0.33 mmol), HOBT (44.6 mg, 0.33 mmol), and DMF (2 mL) were added to a 25 mL single-neck flask at room temperature. The mixture was stirred at room temperature for 24 h. Upon completion of a reaction, the reaction mixture was filtered and purified by reverse-phase Flash chromatography (mobile phase A: 0.5% aqueous FA; mobile phase B: acetonitrile; flow rate: 40 mL/min; gradient: 0-55% B over 20 min; product eluted at 50% B; wavelength: 254/220 nm) to give the product (9.5 mg, 5.2%).
LC-MS (ESI): [M+H]⁺= 555.2
¹H NMR (400 MHz, DMSO-d6) 9.51 (t, J = 8.0 Hz, 1H), 9.13-9.12 (m, 1H), 8.92 (d, J = 4.0 Hz, 1H), 8.78-8.77 (m, 1H), 7.98 (s, 1H), 7.60 (s, 1H), 7.10-7.08 (m, 1H), 7.05 (s, 1H), 7.02 (s, 1H),4.74 - 4.70 (m, 1H), 4.08 (s, 3H), 4.07-4.05 (m, 2H), 3.98-3.95 (m, 2H), 3.89 (s, 3H), 3.05-3.00 (m, 2H), 2.31 (s, 3H), 2.19 (s, 3H), 2.03 (s, 3H), 0.83 (d, J = 8.0 Hz, 3H).

### Example 37, Compound 37

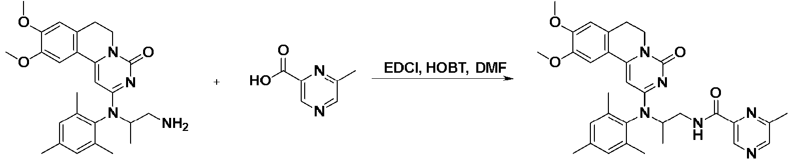

2-((1-Aminopropan-2-yl)(2,4,6-trimethylphenyl)amino)-9,10-dimethoxy-6,7-dihydro-4H-pyrimido[6,1-a]isoquinolin-4-one (100 mg, 0.22 mmol), 6-methylpyrazine-2-carboxylic acid (45.6 mg, 0.33 mmol), EDCI (63.3 mg, 0.33 mmol), HOBT (44.6 mg, 0.33 mmol), and DMF (2 mL) were added to a 25 mL single-neck flask at room temperature. The mixture was stirred at room temperature for 24 h. Upon completion of a reaction, the reaction mixture was filtered and purified by reverse-phase Flash chromatography (mobile phase A: 0.5% aqueous FA; mobile phase B: acetonitrile; flow rate: 40 mL/min; gradient: 0-55% B over 20 min; product eluted at 50% B; wavelength: 254/220 nm) to give the product (29.3 mg, 15.6%).
LC-MS (ESI): [M+H]⁺= 569.2
¹H NMR (400 MHz, CDCl₃) 9.17 (s, 1H), 8.97 (s, 1H), 8.59 (s, 1H), 7.00 (d, J = 8.0 Hz, 2H), 6.70 (s, 1H), 6.60 (s, 1H), 5.31 (s, 1H), 5.08-5.07 (m, 1H), 4.23 - 4.17 (m, 2H), 3.92 (s, 3H), 3.92-3.85 (m, 2H), 3.72 (s, 3H), 2.94-2.91 (m, 2H), 2.68 (s, 3H), 2.34 (s, 3H), 2.25 (s, 3H), 2.12 (s, 3H), 1.11 (d, J = 8.0 Hz, 3H).

### Example 38, Compound 38

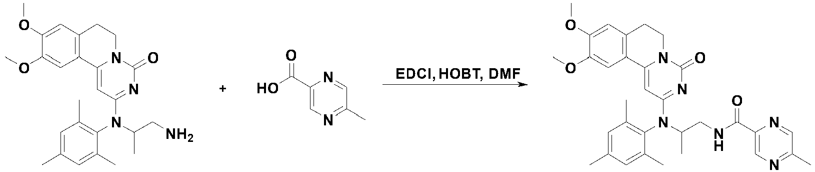

2-((1-Aminopropan-2-yl)(2,4,6-trimethylphenyl)amino)-9,10-dimethoxy-6,7-dihydro-4H-pyrimido[6,1-a]isoquinolin-4-one (150 mg, 0.33 mmol), 5-methylpyrazine-2-carboxylic acid (69.1 mg, 0.50 mmol), EDCI (95.8 mg, 0.50 mmol), HOBT (67.6 mg, 0.50 mmol), and DMF (3 mL) were added to a 25 mL single-neck flask at room temperature. The mixture was stirred at room temperature for 24 h. Upon completion of a reaction, the reaction mixture was filtered and purified by reverse-phase Flash chromatography (mobile phase A: 0.5% aqueous FA; mobile phase B: acetonitrile; flow rate: 40 mL/min; gradient: 0-55% B over 20 min; product eluted at 50% B; wavelength: 254/220 nm) to give the product (50.2 mg, 26.8%).
LC-MS (ESI): [M+H]⁺= 569.2
¹H NMR (400 MHz, CDCl₃) 9.23 (s, 1H), 8.48 (s, 1H), 8.59 (s, 1H), 7.00 (d, J = 12.0 Hz, 2H), 6.70 (s, 1H), 6.60 (s, 1H), 5.31 (s, 1H), 4.98-4.93 (m, 1H), 4.23 - 4.17 (m, 2H), 3.92 (s, 3H), 3.92-3.85 (m, 2H), 3.72 (s, 3H), 2.94-2.91 (m, 2H), 2.65 (s, 3H), 2.35 (s, 3H), 2.25 (s, 3H), 2.12 (s, 3H), 1.14 (d, J = 8.0 Hz, 3H).

### Biological Tests and Evaluation

### I. PDE Enzyme Activity Inhibition Assay I

The inhibitory activity of the compounds of the Examples against PDE enzymes was evaluated using a fluorescence polarization (FP) assay. RPL-554, the compound disclosed in Example 1 of CN100415743C, was used as the positive control.

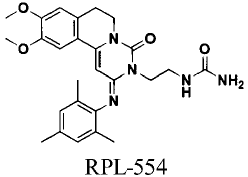

In a reaction buffer, i.e., 1× IMAP Reaction Buffer containing 0.1% BSA and supplemented with 1 mM DTT, a PDE enzyme solution and a substrate solution (FAM-cyclic AMP/cyclic AMP) were prepared. The positive control was prepared at an initial concentration of 1/10 µM for the PDE assay and was diluted 3-fold, with a 10+0 dose design. Using the acoustic liquid handling technology (Echo 655), 0.05 µL of each compound in 100% DMSO was transferred into a 384-well plate (Corning 4514), followed by centrifugation at 1,000 rpm for 1 min. Then, 2.5 µL of the PDE enzyme solution was transferred into the 384-well reaction plate, followed by centrifugation at 1,000 rpm for 1 min and incubation at 25 °C for 10 min. Subsequently, 2.5 µL of the substrate solution was transferred into the 384-well reaction plate, followed by centrifugation at 1,000 rpm for 1 min and incubation at 25 °C for 60 min. Then, 15 µL of a binding reagent mixture was transferred into the 384-well reaction plate, followed by centrifugation at 1,000 rpm for 1 min and incubation at 25 °C for 60 min. Finally, the FP signal was measured using a BMG PHERAstar FSX plate reader. IC₅₀ values and nonlinear regression curve fitting were obtained using GraphPad Prism software.

Table 4 shows the IC₅₀ values of the compounds of the Comparative Examples and the Examples in the enzymatic activity assay. A lower IC₅₀ value indicates that a smaller amount of compound is required to achieve 50% inhibition of the corresponding PDE enzyme, demonstrating stronger inhibitory activity.

**Table 4**

| **Compound No.** | **PDE3A (nM)** | **PDE3B (nM)** | **PDE4B2 (nM)** | **PDE4D2 (nM)** |
|---|---|---|---|---|
| RPL-554 | 0.5789 | 0.7748 | 106.8 | 74.42 |
| Comparative Example **D1** | 1206 | 972.8 | 3067 | 1808 |
| Comparative Example **D2** | 2.197 | 2.886 | 1119 | 669.3 |
| Comparative Example **D3** | 0.2408 | 0.1652 | 814.4 | 198.6 |
| Comparative Example **D4** | 21.24 | >40 | 15198 | 13756 |
| Comparative Example **D5** | 0.1249 | 0.1734 | 87.23 | 60.73 |
| Comparative Example **D6** | 0.2489 | 0.4602 | 113.3 | 120.8 |
| | | | | |

| **Compound of Present disclosure** | **PDE3A (nM)** | **PDE3B (nM)** | **PDE4B2 (nM)** | **PDE4D2 (nM)** |
|---|---|---|---|---|
| **1-1** | 0.09958 | 0.06978 | 75.41 | 61.36 |
| **1-2** | 0.08997 | 0.05435 | 23.83 | 15.35 |
| **2** | 0.07702 | 0.1225 | 130 | 98.66 |
| **3-1** | 0.09455 | 0.1033 | 186.9 | 226.6 |
| **3-2** | 0.0979 | 0.1 | 152.2 | 215.1 |
| **4-1** | >40 | >40 | 468.9 | 490.5 |
| **4-2** | >40 | >40 | 425.3 | 516.6 |
| **5-1** | 0.1089 | 0.1015 | 39.78 | 44.1 |
| **5-2** | 0.1117 | 0.1011 | 20.05 | 17.32 |
| **6** | 13.9 | 27.31 | 1286 | 1402 |
| **7-1** | 0.1408 | 0.2875 | 151.7 | 150.3 |
| **7-2** | 0.1138 | 0.2563 | 204.9 | 276.7 |
| **7-3** | 0.0896 | 0.1492 | 139.2 | 164.8 |
| **8** | 0.04497 | 0.103 | 489.4 | 274.8 |
| **9-1** | 0.0726 | 0.1392 | 18.07 | 19.96 |
| **9-2** | 0.07512 | 0.08175 | 162 | 159.7 |
| **10-1** | 0.04769 | 0.06416 | 35.49 | 40.24 |
| **10-2** | 0.03318 | 0.04673 | 10.91 | 9.789 |
| **11-1** | >40 | >40 | 5.073 | 4.554 |
| **11-2** | 0.06964 | 0.1 | 154.9 | 157 |
| **12-1** | 0.04507 | 0.06946 | 1.239 | 1.684 |
| **12-2** | 0.06906 | 0.07273 | 105.7 | 78.75 |
| **13-1** | 0.1117 | 0.1576 | 9.778 | 5.838 |
| **13-2** | 0.06613 | 0.07678 | 139 | 119.5 |
| **14** | 0.08874 | 0.1357 | 598.5 | 367.7 |
| **15-1** | 0.07961 | 0.08241 | 73.66 | 64.67 |
| **15-2** | 0.07565 | 0.0767 | 21.79 | 13.23 |
| **16** | 0.1142 | 0.1057 | 52.36 | 41.84 |
| **17** | 0.09994 | 0.1223 | 30.36 | 22.96 |
| **18** | 0.1724 | 0.4613 | 26.58 | 15.56 |
| **19** | 0.07852 | 0.1113 | 2.997 | 1.613 |
| **20** | 0.1014 | 0.1693 | 151.7 | 108.4 |
| **21** | 0.08215 | 0.1182 | 122.1 | 115.2 |
| **22** | 0.07804 | 0.1335 | 23.27 | 17.2 |
| **23-1** | 3.89 | 6.942 | 11.61 | 3.874 |
| **23-2** | 0.1355 | 0.171 | 85.29 | 63.39 |
| **24-1** | 2.913 | 4.469 | 201.2 | 319.1 |
| **24-2** | 7.628 | 12.67 | 76.5 | 58 |
| **25-1** | 0.2808 | 0.488 | 14.47 | 8.636 |
| **25-2** | 6.376 | 11.01 | 938.3 | 800.6 |
| **26** | 0.07115 | 0.08478 | 64.84 | 29.47 |
| **27** | 0.06952 | 0.08831 | 29.25 | 23.3 |
| **28** | 6.062 | 17.79 | 44.07 | 30.66 |
| **29** | 0.1259 | 0.1012 | 1.225 | 1.416 |
| **30** | 0.1143 | 0.1258 | 1.397 | 0.8306 |
| **31** | 0.1073 | 0.1076 | 6.3 | 5.748 |
| **32** | 0.1247 | 0.1352 | 4.896 | 2.857 |
| **33** | 0.1079 | 0.1217 | 1.01 | 0.5679 |
| **37** | 0.09047 | 0.1099 | 5.135 | 4.04 |

Conclusion: The compounds of the present disclosure exhibit potent inhibitory activity against PDE3 and PDE4 superior to the prior art, and show promising development potential.

### II. PDE Enzyme Activity Inhibition Assay II

The inhibitory activities of the compounds against PDE enzymes were evaluated using the FP method, with RPL-554 and compound D7 as control compounds. The detailed test method was the same as that described in PDE Enzyme Activity Inhibition Assay I. The structural formulae of RPL-554 and compound D7 are shown below.

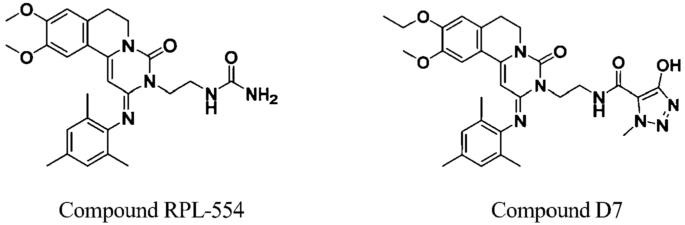

Table 5 shows the IC₅₀ values obtained in the enzymatic activity assay for reference compounds RPL-554 and D7, and compounds 1-1 and 1-2 of the present disclosure.

**Table 5**

| Table 5. IC50, nM | Control compound RPL-554 | Control compound D7 | Compound **1-1** of the present disclosure | Compound **1-2** of the present disclosure |
|---|---|---|---|---|
| PDE1A | >10000 | | 4026 | 5465 |
| PDE1B | >10000 | | 616 | 521.2 |
| PDE1C | >10000 | | 1773 | 3788 |
| PDE2A1 | 3167 | | 1487 | 1349 |
| PDE3A | 0.5789 | 0.1918 | 0.09958 | 0.08997 |
| PDE3B | 0.7748 | 0.2607 | 0.06978 | 0.05435 |
| PDE4A1 | 74.7 | | 158 | 47.75 |
| PDE4B1 | 449.8 | | 820.2 | 269.4 |
| PDE4B2 | 106.8 | 1.412 | 75.41 | 23.83 |
| PDE4C1 | 546.7 | | 2160 | 618.1 |
| PDE4D2 | 74.42 | 0.8177 | 61.36 | 15.35 |
| PDE4D3 | 181.7 | | 252.4 | 96.22 |
| PDE5A1 | >10000 | 2443 | 5823 | 7578 |
| PDE6C | >10000 | | 6207 | 9326 |
| PDE7A1 | >10000 | | >10000 | >10000 |
| PDE7B | >10000 | | >10000 | 9947 |
| PDE8A1 | >10000 | | 1795 | 266.4 |
| PDE9A2 | >10000 | | >10000 | >10000 |
| PDE10A1 | 891.7 | | 59.88 | 65.9 |
| PDE10A2 | 679.9 | | 40.27 | 48.34 |
| PDE11A4 | >10000 | | 3070 | 1639 |

### III. Evaluation of Effects of the Compounds on Viability of Bronchial Smooth Muscle Cells

Cells in the logarithmic growth phase were collected, and human bronchial smooth muscle cells were seeded into 96-well plates at a density of 1 × 10⁵ cells/mL. The cells were cultured in an incubator until they adhered and reached approximately 80% confluence. The cells were then divided into nine groups, with six replicates in each group. The cells were treated with the compounds at concentrations of 100, 50, 25, 12.5, 6.25, 3.125, 1.56, and 0.78 µmol/L. A basic medium was added to the blank control group. After incubation for 24 h, the medium in each well was aspirated and discarded. The MTS reagent and basic medium were mixed at a ratio of 1:4 to prepare the MTS working solution. Then, 100 µL of the MTS working solution was added to each well, and blank control wells were also included. The plates were incubated at 37°C in the dark for 2 h. The absorbance of each well was measured using a microplate reader at 490 nm and 630 nm. The results were recorded, and the IC₅₀ values were calculated.

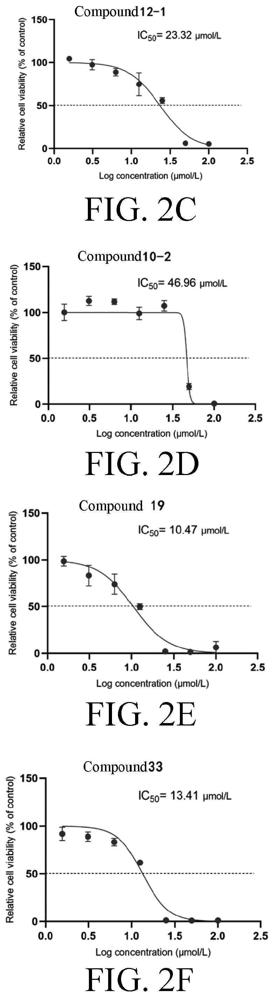

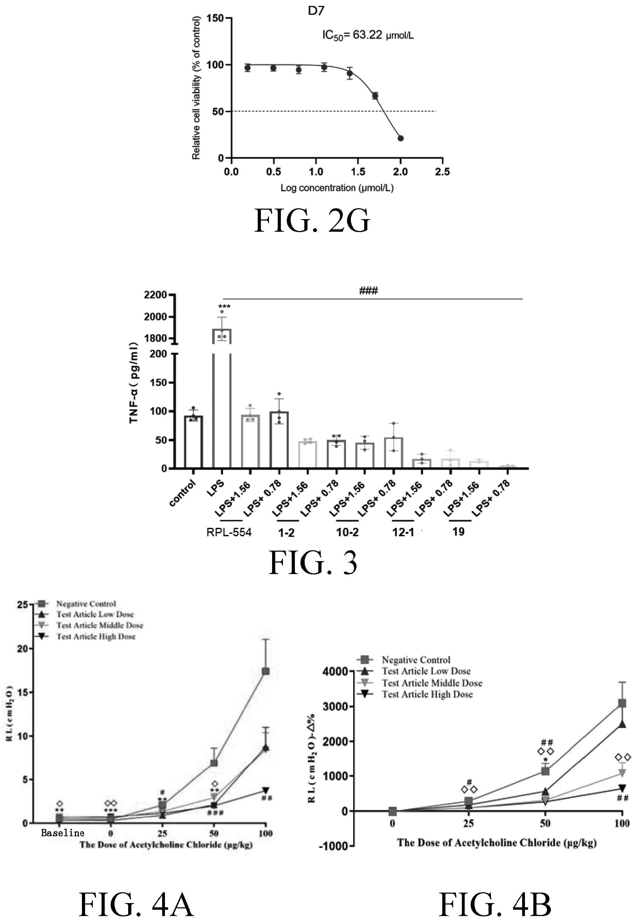

Human bronchial smooth muscle cells were treated with the compounds at concentrations of 100, 50, 25, 12.5, 6.25, 3.125, 1.56, and 0.78 µmol/L for 24 h, and cell viability was evaluated using the MTS assay. The results are shown in Figures 2A to 2G. Compared with the control group, the compounds of the present disclosure inhibited the growth of human bronchial smooth muscle cells at all test concentrations in a dose-dependent manner.

### IV. Pharmacodynamic Evaluation of the Compounds in LPS-Induced Inflammatory Model

Human monocytes were resuspended in RPMI 1640 complete medium containing 1% fetal bovine serum (FBS) and seeded into 96-well plates at a density of 1 × 10⁶ cells/mL, with 100 µL of cell suspension each well. The cells were divided into 14 groups, with six replicates per group, including a normal control group, a model control group, RPL-554 low- and high-dose positive control groups (0.78 and 1.56 µmol/L), Compound **1-2** low- and high-dose groups (0.78 and 1.56 µmol/L), Compound **10-2** low- and high-dose groups (0.78 and 1.56 µmol/L), Compound **12-1** low- and high-dose groups (0.78 and 1.56 µmol/L), and Compound **19** low- and high-dose groups (0.78 and 1.56 µmol/L). The working concentration of LPS in the model control group and compound-treated groups was 1 µg/mL. The cells were further incubated in the absence or presence of LPS and the compounds (5% CO₂, 37°C). After 24 h, the cell culture supernatants were collected and centrifuged at 2,000-3,000 × g for 5 min. The precipitate was removed, and the supernatants were collected for determination of TNF-α levels.

The TNF-α levels in the cell culture supernatants are shown in Figure 3. Compared with the normal control group, the TNF-α level in the cell culture supernatant of the model control group was significantly increased. Compared with the model control group, the RPL-554 positive control group and the compound groups of the present disclosure showed a decrease with statistic significance.

### V. Pharmacodynamic Study of the Compounds in Guinea Pig Model of Airway Constriction Induced by Acetylcholine Chloride

(1) The inhibitory effect of the inhaled test compound, Compound **10-2** of the present disclosure, on acetylcholine chloride-induced airway constriction in guinea pigs was evaluated. 40 male Hartley guinea pigs were randomly divided into four groups: a negative control group (clean air, 60-min inhalation), a low-dose test group (10-min inhalation), a medium-dose test group (20-min inhalation), and a high-dose test group (60-min inhalation), with 10 animals per group. The animals received a single-inhalation administration. Approximately 2 h after inhalation, the animals were anesthetized. The trachea and jugular vein were surgically exposed. The trachea was cannulated, and a catheter was inserted into the jugular vein. Each animal was placed on the support platform of a whole-body plethysmograph and connected to the respiratory circuit. The distal end of the venous catheter was led outside the plethysmograph chamber, and the chamber was then closed. Acetylcholine chloride was administered at doses of 25 µg/kg, 50 µg/kg, and 100 µg/kg via the jugular venous catheter. Airway resistance was measured using an AniRes2005 animal pulmonary function analysis system.

The results are shown in FIGS. 4A and 4B. Two hours after a single nebulized inhalation administration at actual delivered doses of 0.171 mg/kg, 0.341 mg/kg, and 1.024 mg/kg, the test compound (Compound **10-2** of the present disclosure) significantly inhibited airway constriction induced by acetylcholine at doses ranging from 25 µg/kg to 100 µg/kg, and exhibited a dose-response relationship.

(2) The inhibitory effect of the inhaled test compound, Compound **1-2** of the present disclosure, on acetylcholine chloride-induced airway constriction in guinea pigs was evaluated. 40 male Hartley guinea pigs were randomly divided into four groups: a negative control group (clean air, 30-min inhalation), a low-dose test group (6-min inhalation), a medium-dose test group (12-min inhalation), and a high-dose test group (30-min inhalation), with 10 animals per group. The animals received a single inhalation administration. Approximately 2 h after inhalation, the animals were anesthetized. The trachea and jugular vein were surgically exposed. The trachea was cannulated, and a catheter was inserted into the jugular vein. Each animal was placed on the support platform of a whole-body plethysmograph and connected to the respiratory circuit. The distal end of the venous catheter was led outside the plethysmograph chamber, and the chamber was then closed. Acetylcholine chloride was administered at different doses via the jugular venous catheter, and airway resistance was measured using the AniRes2005 animal pulmonary function analysis system.

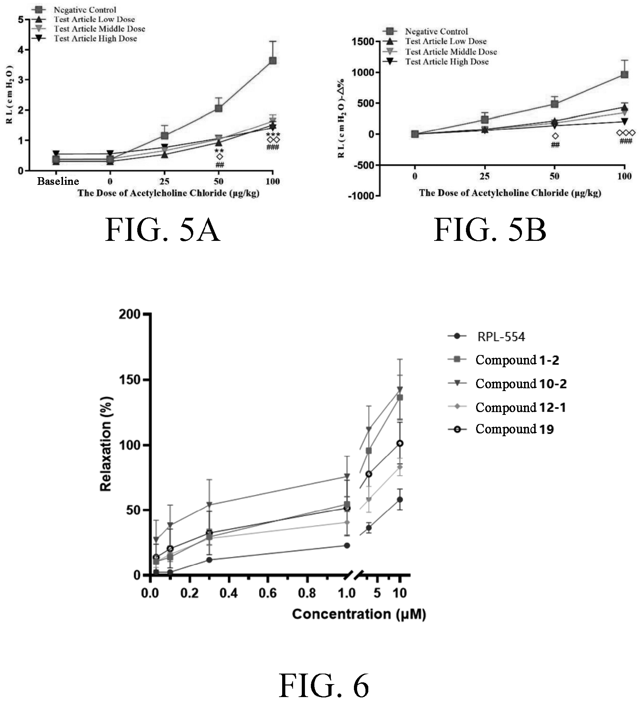

The results are shown in FIGS. 5A and 5B. Two hours after a single nebulized inhalation administration at actual delivered doses of 0.235 mg/kg, 0.470 mg/kg, and 1.174 mg/kg, the test compound (Compound **1-2** of the present disclosure) significantly inhibited airway constriction induced by acetylcholine at doses ranging from 25 µg/kg to 100 µg/kg at 2 h post-dose, and exhibited a dose-response relationship.

### VI. Effect of the Compounds on Nebulized Acetylcholine-Induced Airway Constriction in Mice

Preparation of test compound: the test compounds were weighed as pure compounds without considering impurities due to the high purity of the test compounds. The test compound was prepared at a concentration of 10 mg/mL, using a pH 3.2 citric acid-disodium hydrogen phosphate buffer containing 25% DMSO as the solvent.

Administration of test compounds: The test compounds were administered to mice by oronasal nebulization using a NAM system from DSI. Each mouse received a total volume of 400 µL, delivered in two nebulization sessions of 5 min each. The model group received an equal volume of vehicle by nebulization.

Newly received experimental animals were quarantined for 3 days and grouped as described above. Airway nebulized administration was performed using the NAM system, with the nebulization procedure consisting of 5-min nebulization (200 µL), a 3-min interval, and another 5-min nebulization (200 µL). Approximately 0.5 h after completion of nebulized administration, acetylcholine was nebulized using the RC system (15 mg/mL, 20 µL, 30 s, prepared in 0.9% normal saline) to challenge the animals to induce airway constriction, and changes in airway resistance (RL) were simultaneously measured using the RC instrument. The results are shown in Table 6:

**Table 6**

| Groups | Baseline value | 15 mg/mL ACh challenge | Challenge-induced increase | Mean | SD | Inhibition (%) |
|---|---|---|---|---|---|---|
| RPL-554 (14 mg/mL) | 1 | 5.5 | 4.5 | | | |
| | 0.9 | 10.3 | 9.4 | | | |
| | 1 | 9.9 | 8.9 | 7.6 | 2.7 | 28.3 |
| Compound **1-1** (14 mg/ml) | 1.0 | 13.2 | 12.2 | | | |
| | 0.9 | 4.1 | 3.2 | | | |
| | 1.1 | 9.7 | 8.6 | 8.0 | 4.5 | 24.5 |
| Compound **1-2** (14 mg/ml) | 1.0 | 3.1 | 2.1 | | | |
| | 1.0 | 1.9 | 0.9 | | | |
| | 1.0 | 2.3 | 1.3 | 1.4 | 0.6 | 86.5 |

### VII. Oral Bioavailability Study in Rats

Test Objective: To evaluate the oral bioavailability and other pharmacokinetic parameters of RPL-554, and Compounds **1-2, 10-2, 67,** and **69** of the present disclosure in rats following oral gavage at 1 mg/kg.

Test Compounds: RPL-554, and Compounds **1-2, 10-2, 67,** and **69** of the present disclosure.

Animals: Male SD rats, n = 30.

The dosing solution for the RPL-554-iv-1 mg/kg group and the RPL-554-po-1 mg/kg group was prepared as follows: approximately 3.11 mg of RPL-554 was weighed, 0.3 mL of DMSO was added, then 29.7 mL of normal saline was added, and the mixture was vortexed for 2 min. The solution was freshly prepared before use.

The dosing solution for the Compound **10-2-**iv-1 mg/kg group and the Compound **10-2-po-1** mg/kg group was prepared as follows: approximately 3.0 mg of Compound **10-2** was weighed and mixed with 0.3 mL of DMSO. After thorough mixing, 29.7 mL of 10% sulfobutyl ether-β-cyclodextrin in normal saline was added, and the mixture was vortexed for 2 min until completely dissolved. The solution was freshly prepared before use.

The dosing solution for the Compound **1-2-iv-1** mg/kg group and the Compound **1-2-**po-1 mg/kg group was prepared as follows: approximately 3.0 mg of Compound **1-2** was weighed and mixed with 0.3 mL of DMSO, followed by addition of 29.7 mL of normal saline. The mixture was vortexed for 2 min. The solution was freshly prepared before use.

The dosing solution for the Compound **67**-iv-1 mg/kg group and the Compound **67**-po-1 mg/kg group was prepared as follows: approximately 3.0 mg of Compound 67 was weighed and mixed with 1.5 mL of purified polyoxyethylene 35 castor oil, followed by addition of 28.5 mL of normal saline. The mixture was vortexed for 2 min and sonicated until completely dissolved. The solution was freshly prepared before use.

The dosing solution for the Compound **69**-iv-1 mg/kg group and the Compound **69**-po-1 mg/kg group was prepared as follows: approximately 3.0 mg of Compound **69** was weighed and mixed with 1.5 mL of PEG-400. After thorough mixing, 28.5 mL of normal saline was added, and the mixture was vortexed for 2 min and sonicated until completely dissolved. The solution was freshly prepared before use.

Dosing Regimen: Rats in the intravenous groups were administered by tail vein injection, and rats in the oral gavage groups were administered by oral gavage. See Table 7.

**Table 7**

| Group | Number of animals | Dose (mg/kg) |
|---|---|---|
| | ♂ | |
| RPL-554-iv-1 mg/kg group | 3 | 1 (RPL-554) |
| RPL-554-po-1 mg/kg group | 3 | 1 (RPL-554) |
| Compound 1-2-iv-1 mg/kg group | 3 | 1 (Compound 1-2) |
| Compound **1-2**-po-1 mg/kg group | 3 | 1 (Compound **1-2)** |
| Compound **10-2**-iv-1 mg/kg group | 3 | 1 (Compound **10-2)** |
| Compound **10-2**-po-1 mg/kg group | 3 | 1 (Compound **10-2)** |
| Compound **67**-iv-1 mg/kg group | 3 | 1 (Compound **67)** |
| Compound **67**-po-1 mg/kg group | 3 | 1 (Compound **67)** |
| Compound **69**-iv-1 mg/kg group | 3 | 1 (Compound **69)** |
| Compound **69**-po-1 mg/kg group | 3 | 1 (Compound **69)** |

Rat dosing and sampling: The rats were divided into ten groups and received the corresponding test compounds at a dosing volume of 10 mL/kg by either intravenous injection via the tail vein or oral gavage. Blood samples of 0.2 mL were collected at 5, 15, and 30 min and at 1, 2, 4, 6, 8, 12, 24, and 48 h after dosing. Each blood sample was placed in a disposable anticoagulant tube and centrifuged at 3,500 rpm for 10 min at 4 °C. Plasma was then separated and stored at -20 °C until analysis.

Plasma Sample Preparation and LCMS Analysis: 50 µL of each plasma sample was transferred to a 1.5 mL centrifuge tube, and 200 µL of an internal standard working solution was added. The mixture was vortexed for 5 min and then centrifuged at 12,000 rpm for 10 min. The supernatant was transferred to an autosampler vial for LC/MS analysis, and chromatographic data were acquired.

Pharmacokinetic Results: The major pharmacokinetic profiles of Compounds **1-2, 10-2, 67,** and **69** in rats were generally comparable to that of the reference compound RPL-554. All tested compounds exhibited very low oral bioavailability. The results are shown in Tables 8 and 9.

**Table 8**

| PK Parameters | T_{1/2} | C₀ | AUCₗₐₛₜ | AUC_{INF_obs} |
|---|---|---|---|---|
| | h | ng/mL | h*ng/mL | h*ng/mL |
| RPL-554-iv-1 mg/kg group | 1.62 | 333.09 | 210.79 | 211.41 |
| Compound **10-2**-iv-1 mg/kg group | 0.58 | 706.51 | 381.25 | 381.58 |
| Compound **1-2**-iv-1 mg/kg group | 1.10 | 748.58 | 347.00 | 347.40 |
| Compound **67**-iv-1 mg/kg group | 1.08 | 339.49 | 193.67 | 196.57 |
| Compound **69**-iv-1 mg/kg group | 2.03 | 407.83 | 215.26 | 237.56 |

**Table 9**

| PK Parameters | T_{1/2} | Tₘₐₓ | Cₘₐₓ | AUCₗₐₛₜ | AUC_{INF_obs} | F |
|---|---|---|---|---|---|---|
| | h | h | ng/mL | h*ng/mL | h*ng/mL | % |
| RPL-554-po-1 mg/kg group | 4.60 | 0.25 | 0.91 | 1.59 | 3.13 | 1.48 |
| Compound **10-2**-po-1 mg/kg group | 2.05 | 0.42 | 3.83 | 4.02 | 4.69 | 1.23 |
| Compound **1-2**-po-1 mg/kg group | 9.61 | 0.28 | 1.15 | 2.23 | 5.21 | 1.50 |
| Compound **67**-po-1 mg/kg group | 2.76 | 0.83 | 0.76 | 1.04 | 2.43 | 1.24 |
| Compound **69**-po-1 mg/kg group | 3.42 | 0.67 | 2.62 | 3.04 | 4.36 | 1.84 |

### VIII. In Vitro Human PK Study - Plasma Protein Binding

Test Objective: To investigate the *in vitro* human plasma protein binding of RPL-554, Compound 1-2, Compound 10-2, Compound 67, and Compound 69.

Test Compounds: RPL-554, and Compounds 1-2, 10-2, 67, and 69.

Plasma: Human plasma.

Incubation conditions: test compound incubation time, 5 h; test compound final concentration, 0.5 µM; species, human; temperature, 37.0 °C.

### Experimental Procedure:

Stock solutions of the test compounds and Warfarin were prepared, diluted, and added to the plasma matrix so that the final concentration of each test compound in the system was 10 µM, and the final concentration of Warfarin was 1 µM. Immediately after preparation, 50 µL of the sample was transferred into termination solution, and 50 µL of a blank buffer was added to obtain the T0 sample for recovery calculation.

For the test groups and system control group, 100 µL of plasma solution containing Warfarin or a test compound, or 100 µL of a buffer solution containing a test compound, was added to the donor chamber of an equilibrium dialysis device.

The donor and receiver chambers were incubated at 37 °C and 100 rpm for 5 h.

For the stability test group, each test compound was incubated with plasma or a blank buffer for 0 or 5 h.

At the incubation, 50 µL sample was collected from the donor chamber for each group, and 50 µL of a blank buffer was added. In addition, 50 µL sample was collected from the receiver chamber, and 50 µL of blank plasma was added, so that the final volume of each sample was 100 µL.

Methanol containing an internal standard was added to all samples to precipitate proteins. After centrifugation, the supernatant was collected, and the concentrations of the test compounds and Warfarin in the samples were determined by LC-MS/MS using relative quantification.

Pharmacokinetic Parameter Results: RPL-554, Compound 1-2, Compound 10-2, Compound 67, and Compound 69 all showed a high plasma protein binding ratio in human plasma. See Table 10.

**Table 10**

| Compound | Protein Binding Ratio (%) | | Average |
|---|---|---|---|
| | Sample 1 | Sample 2 | |
| RPL-554 | 91.8 | 92.9 | 92.4 |
| Compound **10-2** | 96.3 | 96.9 | 96.6 |
| Compound **1-2** | 93.9 | 90.6 | 92.3 |
| Compound **67** | 98.5 | 98.4 | 98.4 |
| Compound **69** | 98.0 | 97.7 | 97.9 |

### IX. In Vitro Human PK Study-CYP450 DDI

Test Objective: To evaluate the inhibitory effects of RPL-554, Compound 1-2, Compound 10-2, Compound 67, and Compound 69 on CYP3A4, CYP2C9, and CYP2D6.

Test Compounds: RPL-554, and Compounds 1-2, 10-2, 67, and 69.

CYP enzymes: CYP3A4, CYP2C9, and CYP2D6.

Incubation conditions: test compound incubation time, 5 min for CYP3A4, and 10 min for CYP2C9 and CYP2D6; test compound final concentration, 10 µM; microsomal protein concentration, 0.5 mg/mL; NADPH concentration, 1.0 mM; temperature, 37.0 °C.

### Experimental Procedure:

Working solutions of the substrates and inhibitors for CYP2C9, CYP2D6, and CYP3A4 were prepared separately.

The NADPH solution was prepared and prewarmed at 37 °C in a shaking water bath before use.

Liver microsomes were thawed and diluted with a buffer.

The corresponding solutions were added to each well of an incubation plate and preincubated for 15 minutes. Group: Test group = Test compound at different concentrations + liver microsomes in a solution. Negative control group (NC) = microsomes + a buffer. Positive control group (PC) = microsomes + selective inhibitor for the corresponding CYP isoform.

After preincubation, a reactions was initiated by adding the corresponding solution (CYP3A4: 5 min; CYP2C9 and CYP2D6: 10 min). Test group = substrate for each CYP isoform + coenzyme. Negative control group (NC) = substrate for each CYP isoform + coenzyme. Positive control group (PC) = substrate for each CYP isoform + coenzyme.

All samples were incubated at 37 °C. At the termination time point, each reaction was stopped by adding pre-chilled methanol and the time was recorded.

After thorough mixing, all samples were centrifuged at 4,000 rpm for 10 min. The supernatants were collected and analyzed by LC-MS/MS.

Pharmacokinetic Parameter Results: At 10 µM, RPL-554, Compound 1-2, Compound 10-2, Compound 67, and Compound 69 showed a certain degree of inhibition against CYP2C9, but showed weak inhibition against CYP2D6. At 10 µM, RPL-554 and Compound 10-2 showed no inhibition against CYP3A4. In contrast, Compound 1-2, Compound 67, and Compound 69 inhibited CYP3A4 to varying degrees. See Table 11.

**Table 11**

| Relative Activity (% of NC) | | | | |
|---|---|---|---|---|
| Test Compound | CYP2C9 | CYP2D6 | CYP3A4 (substrate: testosterone) | CYP3A4 (substrate: midazolam) |
| RPL-554 | 66.5 | 94.1 | 97.3 | 76.5 |
| Compound **10-2** | 69.9 | 106 | 107 | 95.2 |
| Compound **1-2** | 67.3 | 89.8 | 66.2 | 78.9 |
| Compound **67** | 16.8 | 82.0 | 31.7 | 17.2 |
| Compound **69** | 26.0 | 91.6 | 28.4 | 26.5 |

### X. In Vitro Human Pharmacokinetic Study-Liver Microsomal Incubation Assay

Test Objective: To evaluate the *in vitro* metabolic stability of RPL-554, Compound 1-2, Compound 10-2, Compound 67, and Compound 69.

Test Compounds: RPL-554, and Compounds 1-2, 10-2, 67, and 69.

Test System: Human liver microsomes.

Incubation conditions: test compound incubation time, 0, 30, 60, and 120 min; test compound final concentration, 0.5 µM; microsomal protein concentration, 0.5 mg/mL; NADPH and UDPGA concentrations, 1.0 mM; temperature, 37.0 °C.

### Experimental Procedure:

A mixed working solution of testosterone and 7-hydroxycoumarin was prepared.

A mixed solution of NADPH and UDPGA was prepared and prewarmed at 37 °C in a shaking water bath before use.

Liver microsomes were thawed and diluted with a buffer.

The prepared test compound solution and liver microsome suspension were added to each well of a incubation plate.

The experiment was divided into three groups, and the corresponding components were added to each group. Test group=test compound + microsomes + coenzyme. Test compound negative control group (NC)=test compound + microsomes + buffer. Positive control group=phase I and phase II substrates + microsomes + coenzyme.

All samples were incubated at 37 °C. At each designated termination time point, each reaction was stopped by adding pre-chilled methanol containing an internal standard, and the time was recorded.

After thorough mixing, all samples were centrifuged at 4,000 rpm for 10 min. The supernatants were collected and analyzed by LC-MS/MS.

Pharmacokinetic Parameter Results: The metabolic profiles of RPL-554, Compound 1-2, Compound 10-2, Compound 67, and Compound 69 are summarized in Table 12.

**Table 12**

| Test Compound | Species | T120 - Remaining (% of 0 min) | T1/2 (min) | CLint (mL/min/kg) | T120 NC - Remaining (% of 0 min- NC) | Metabolic Type (Fast/Moderate/ Slow) |
|---|---|---|---|---|---|---|
| RPL-554 | Human | 5.10 | <30 (15.1) | >57.9 (115) | 86.3 | Fast |
| Compound **1-2** | Human | 27.5 | 64.2 | 27.1 | 86.1 | Moderate |
| Compound **10-2** | Human | 4.53 | <30 (11.8) | >57.9 (147) | 79.3 | Fast |
| Compound **67** | Human | 8.76 | <30 (9.7) | >57.9 (179) | 56.6 | Fast |
| Compound **69** | Human | 10.9 | <30 (12.3) | >57.9 (142) | 91.0 | Fast |

### XI. In Vitro Human PK Study - Caco 2 Cell Permeability Assay

Test Objective: To evaluate the Caco2 cell permeability to RPL-554, Compound 1-2, Compound 10-2, Compound 67, and Compound 69.

Test compounds: RPL-554, and Compounds 1-2, 10-2, 67, and 69.

Cell Line: Human colorectal adenocarcinoma cell line.

Incubation conditions: Test system, Caco-2 cells; incubation time, 2 h; test compound final concentration, 1 µM; temperature, 37.0 °C.

### Experimental Procedure:

Quality control before the permeability assay: Before the assay, the transepithelial electrical resistance of the cells was measured using a resistance meter, and the apparent transepithelial resistance of the cell monolayer was calculated.

Permeability assay: Before the assay, the culture medium was aspirated from the culture plate, and the cells were washed three times with HBSS buffer prewarmed at 37°C (Apical side and Basal side); After the buffer in the plate was aspirated, 800 µL of HBSS buffer prewarmed at 37 °C was added to the B side, and 500 µL of propranolol solution (PC), nadolol solution (SC), or a test compound solution prewarmed at 37 °C was added to A side. A 100 µL solution was then taken from the A side as the 0 h apical sample and stored at -20 °C until analysis. The culture plate was incubated at 37°C for 120 min. At the end of incubation, a 100 µL solution was collected from each sample at both A side and B side as the 120 min sample and stored at -20 °C until analysis. All samples were mixed with internal-standard-containing methanol at a ratio of 1:4 and analyzed by LC-MS/MS. After incubation, 100 µL was taken from the basal side and transferred to a black 96-well plate. An LFY standard curve was prepared, and fluorescence was measured using a fluorescence microplate reader at an excitation wavelength of 485 nm.

Note: The A side contained 10 µM Lucifer Yellow. All test compound buffers contained 0.1% BSA.

Pharmacokinetic Parameter Results: At the test concentration of 1 µM, the Papp (A-B) values of RPL-554, Compound 1-2, Compound 10-2, Compound 67, and Compound 69 were 0.643×10⁻⁶ cm/s, 0.412×10⁻⁶ cm/s, 0.0477×10⁻⁶ cm/s, 0.422×10⁻⁶ cm/s, and 0.594×10⁻⁶ cm/s, respectively. According to the permeability classification criteria, RPL-554, Compound 1-2, Compound 10-2, Compound 67, and Compound 69 were classified as low-permeability compounds. The results are shown in Table 13.

**Table 13**

| Test Compound | Pₐₚₚ/ 10⁻⁶ cm/s | Recovery (%) |
|---|---|---|
| RPL-554 | 0.643 | 116 |
| Compound **1-2** | 0.412 | 97.0 |
| Compound **10-2** | 0.0477 | 87.7 |
| Compound **67** | 0.422 | 75.9 |
| Compound **69** | 0.594 | 117 |

### XII. Effect of the Compounds on Relaxation of Isolated Trachea

Preparation of K-H Solution: The modified K-H solution (Krebs-Henseleit solution) contained the following components (mmol/L): NaCl 133, KCl 4.7, MgSO₄ 0.61, NaH₂PO₄ 1.35, CaCl₂ 2.52, NaHCO₃ 16.3, and glucose 7.8. Except for NaHCO₃, CaCl₂, and glucose, which were freshly added before the experiment, the other components were prepared as concentrated stock solutions and stored at room temperature. On the day of the experiment, appropriate volumes of the stock solutions were taken and diluted with ultrapure water to required concentrations. The prepared K-H solution was adjusted to pH 7.2-7.4 with hydrochloric acid, and fully pre-saturated with mixed gas of 95% O₂ and 5% CO₂ before use.

Experimental Animals: Male guinea pigs each weighing 300-350 g, as well as the feed, were provided by Beijing Vital River Laboratory Animal Technology Co., Ltd. All animal experiments were performed in accordance with the National Institutes of Health Guide for the Care and Use of Laboratory Animals. The animals were housed at the New Drug Evaluation Center of Hebei Yiling Pharmaceutical Research Institute Co., Ltd. under a 12 h light/dark cycle, at 20-26 °C and 40-70% relative humidity, with five animals per cage. Corncob bedding for the experimental animals was provided by Jiangsu Xietong Pharmaceutical Bioengineering Co., Ltd. and used after autoclaving. The animals were fed an SPF-grade breeding diet and provided with bottled purified water ad libitum, and the water was changed once daily. After one week of adaption, the subsequent experimental procedures were performed.

During the experiment, the animals were weighed and their body weights were recorded. The animals were stunned by a single blow to the head with a wooden rod, after which the chest was rapidly opened. The intact trachea, extending from the thyroid cartilage to the tracheal bifurcation near the lung parenchyman, was excised and immediately immersed in the modified K-H solution aerated with mixed gas of 95% O₂ and 5% CO₂.

The connective tissue surrounding the trachea was removed. The trachea was cut transversely at intervals of two cartilage rings. Every four cartilage rings were ligated to form one tracheal chain segment. Two tungsten wire loops were passed in parallel through the lumen of each tracheal preparation, and the specimen was placed in a bath containing 10 mL of the modified K-H solution. One side of each tracheal preparation was fixed to the lower end of a stainless-steel support through a tungsten wire loop, and the other side was connected to a tension transducer through a tungsten wire loop and a silk thread. Tracheal tension signals were recorded by a data acquisition system through the tension transducer. The bath was maintained at 37°C and continuously supplied with oxygen. The initial load was adjusted to 2 g. The K-H solution was replaced every 15 min, and the specimen was equilibrated for 1 h, during which the solution was replaced four times. The experiment was initiated after the experimental conditions had stabilized. After a stable baseline curve was recorded, acetylcholine chloride (ACh) was added to give a final concentration of 1 × 10⁻⁵ mol/L. When the contraction was observed to have reached its maximum level, the compounds were added sequentially at final concentrations of 0.03, 0.1, 0.3, 1, 3, 10, 30, 100, and 300 µmol/L, with a 5-min interval between concentrations, and the value at the minimal relaxation was recorded. The tracheal relaxation function was expressed as the tracheal relaxation rate, which was calculated as the percentage of the relaxation amplitude induced by the compound based on the ACh-induced precontraction amplitude. The EC₅₀ value of each compound was calculated.

The results are shown in Figure 6 and Table 14. Compared with the positive control, Compound 1-2, Compound 10-2, Compound 12-1, and Compound 19 all effectively relieved ACh-induced contraction of isolated tracheal smooth muscle from guinea pig.

**Table 14**

| Compound | EC₅₀ (µmol/L) |
|---|---|
| RPL-554 | 6.211 |
| Compound **1-2** | 0.6258 |
| Compound **10-2** | 0.1667 |
| Compound **12-1** | 1.435 |
| Compound **19** | 0.6657 |

### XIII. hERG Experimental Evaluation

The experimental materials are listed in Table 15.

**Table 15**

| Reagents | Brand | Cat. No. |
|---|---|---|
| DMEM (high glucose) medium | Gibco | C11995500BT |
| Fetal bovine serum (FBS) | Gibco | 16000-044 |
| G418 | MedChem Express | HY-17561 |

A human embryonic kidney cell line (HEK293) stably expressing the hERG channel was cultured in DMEM medium in a cell culture incubator at 37°C under 5% CO₂. The culture medium was supplemented with 10% fetal bovine serum (FBS) and G418 at a final concentration of 400 µg/mL. Prior to the assay, cells were digested with 0.25% trypsin when they reached approximately 75% confluence, as confirmed under an inverted microscope, to obtain a single-cell suspension. The cell suspension was pipetted dropwise onto cell coverslips, and the coverslips were then incubated in a cell culture incubator. After cell attachment, the cells on the coverslips were used for patch-clamp recording. Terfenadine was used as the positive control.

Voltage-clamp parameters were set using Clampex 10.6 software. In the whole-cell configuration, the cells were held at -80 mV for 125 ms and then subjected to a depolarizing voltage step to +50 mV for 5000 ms to activate the hERG channels. The membrane potential was subsequently repolarized to -50 mV for 5000 ms to elicit the characteristic hERG tail current, which was recorded. The tail current was used for data acquisition, measurement, and analysis. The experimental results are shown in Table 16.

**Table 16**

| Agent | Inhibition rate (%) | | | |
|---|---|---|---|---|
| | n | 1 µM | n | 10 µM |
| RPL-554 | 3 | 17.2±6.5 | 4 | 67.2±3.4 |
| Compound **1-2** | 3 | 6.9±1.0 | 3 | 35.4±2.0 |
| Compound **10-2** | 3 | 23.0±4.9 | 3 | 62.5±15.4 |
| Compound **12-1** | 3 | 11.5±4.6 | 5 | 27.9±5.6 |
| Compound **19** | 3 | 7.7±3.6 | 3 | 50.5±2.4 |
| Terfenadine | | | 3 | 74.5±1.5 |

Conclusion: RPL-554, Compound 1-2, and Compound 19 at 10 µM inhibited hERG current, whereas the other agents showed no effect on hERG current at either 1 µM or 10 µM.

### XIV. Study on Mutagenicity of the Compounds in Salmonella typhimurium

The bacterial toxicity and potential mutagenicity of Compound **1-2,** Compound **10-2,** Compound **12-1,** and Compound **19** to *Salmonella typhimurium* were evaluated separately to determine the optimal dose ranges for subsequent mutagenesis assays in *Salmonella typhimurium.*

The assay was performed using five histidine auxotrophic *Salmonella typhimurium* strains, namely TA97a, TA98, TA100, TA102, and TA1535, supplied by JOINN Laboratories (Suzhou) Co., Ltd. Each bacterial strain system was treated with the test compound at doses of 5000, 1500, 500, 150, and 50 µg/plate in the absence or presence of metabolic activation. A vehicle control group, DMSO, was also included. For each test point, duplicate plates were set both in the presence of metabolic activation (+S9, with S9 mix) and in the absence of metabolic activation (-S9, without S9 mix). Strains TA97a, TA98, TA100, and TA1535 were incubated at 37°C for approximately 48 h, and strain TA102 was incubated at 37°C for approximately 72 h. Revertant colonies were counted for each plate, and the background lawn was examined microscopically. Additionally, turbidity and precipitation were also assessed at the time of addition of the control or test compound and at the end of incubation.
(1) Compound **1-2:** In this study, bacterial toxicity was observed in strains TA97a and TA100 with 5000 µg/plate of the test compounds both in the presence and absence of metabolic activation. For strains TA98, TA102, and TA1535, toxicity could not be evaluated with 5000 µg/plate of the test compound because precipitation was observed on the plates. No obvious bacterial toxicity was observed under the other test conditions. Compound 1-2 showed no mutagenic activity toward the histidine auxotrophic *Salmonella typhimurium* TA97a, TA98, TA100, TA102, and TA1535, either in the absence or presence of metabolic activation.
(2) Compound **10-2:** In this study, bacterial toxicity was observed in strain TA100 with 1500 µg/plate of the test compound both in the presence and absence of metabolic activation. For strains TA97a, TA98, TA100, TA102, and TA1535 with 5000 µg/plate of the test compound, for strains TA97a, TA98, TA102, and TA1535 with 1500 µg/plate of the test compound, and for strain TA102 with 500 µg/plate of the test compound, colonies could not be counted due to precipitation on the plates, and toxicity could not be evaluated. No obvious bacterial toxicity was observed under the other test conditions. Compound 10-2 showed no mutagenic activity toward the histidine auxotrophic *Salmonella typhimurium* TA97a, TA98, TA100, TA102, and TA1535, either in the absence or presence of metabolic activation.
(3) Compound **12-1:** In this study, for strains TA97a, TA98, TA100, TA102, and TA1535, the background bacterial lawn could not be observed on the plates with 1500-5000 µg/plate of the test compound due to precipitation. For strains TA97a, TA98, and TA102 both in the presence and absence of metabolic activation, and for strain TA100 in the presence of metabolic activation, colonies could not be counted due to precipitation, and toxicity could not be evaluated. No obvious bacterial toxicity was observed under the other test conditions. Compound **12-1** showed no mutagenic activity toward the histidine auxotrophic *Salmonella typhimurium* TA97a, TA98, TA100, TA102, and TA1535, either in the absence or presence of metabolic activation.
(4) Compound **19:** In this study, for strains TA97a, TA98, TA100, TA102, and TA1535, the background bacterial lawn could not be observed on the plates with 1500-5000 µg/plate of the test compound due to precipitation, and toxicity could not be evaluated, either in the absence or presence of metabolic activation. No obvious bacterial toxicity was observed under the other test conditions. Compound **19** showed no mutagenic activity toward the histidine auxotrophic *Salmonella typhimurium* TA97a, TA98, TA100, TA102, and TA1535, either in the absence or presence of metabolic activation.

The foregoing are only particular embodiments of the present disclosure. It should be noted that, for a person of ordinary skill in the art, various improvements and modifications can be made without departing from the principle of the present disclosure, and these improvements and modifications are also within the scope of protection of the present disclosure.

## Claims

1. A compound of Formula I, or a pharmaceutically acceptable form thereof, wherein the pharmaceutically acceptable form is selected from a pharmaceutically acceptable salt or co-crystal, a stereoisomer, a tautomer, a deuterate, a solvate, a chelate, a non-covalent complex, or a prodrug, 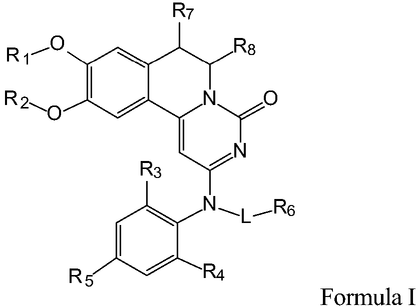 wherein:
R₁ and R₂ are each independently selected from H, a C₁₋₆ linear alkyl, a C₃₋₆ branched alkyl, or a C₃₋₆ cycloalkyl; each of the linear alkyl, branched alkyl, and cycloalkyl is optionally further substituted with 0 to 4 substituents selected from D, F, Cl, Br, I, OH, =O, NH₂, CN, COOH, a C₁₋₄ alkyl, or a C₁₋₄ alkoxyl;
R₃, R₄, and R₅ are each independently selected from H, halogen, CN, a C₁₋₆ alkoxy, a C₁₋₆ linear alkyl, a C₃₋₆ branched alkyl, or a C₃₋₆ cycloalkyl; each of the alkoxy, linear alkyl, branched alkyl, and cycloalkyl is optionally further substituted with 0 to 4 substituents selected from D, F, Cl, Br, I, OH, =O, NH₂, CN, COOH, a C₁₋₄ alkyl, or a C₁₋₄ alkoxy;
L is selected from 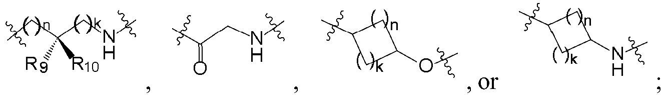 n is 0, 1, or 2; k is 0, 1, 2, or 3; the H in L is optionally further substituted with 0 to 4 substituents selected from F, Cl, Br, I, OH, =O, NH₂, CN, COOH, a C₁₋₄ alkyl, or a C₁₋₄ alkoxy; wherein R₉ and R₁₀ are each independently selected from H, a C₁₋₆ linear alkyl, a C₃₋₆ branched alkyl, a C₃₋₆ cycloalkyl, a C₃₋₆ heterocycloalkyl, a C₆₋₁₀ aryl, a C₅₋₁₀ heteroaryl, or COOCH₃, provided that R₉ and R₁₀ are not both H; the heteroaryl contains 1 to 3 heteroatoms selected from N, O, or S;
R₆ is selected from 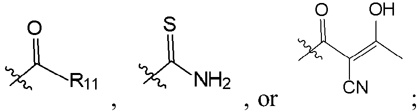 wherein R₁₁ is selected from amino, a C₁₋₆ alkoxy, a C₃₋₁₂ cycloalkyl, a C₆₋₁₀ aryl, or a C₅₋₁₀ heterocyclyl; wherein the heterocyclyl contains 1 to 3 heteroatoms selected from N, O, or S; each of the amino, alkoxy, cycloalkyl, aryl, and heterocyclyl is optionally substituted with 0 to 4 substituents selected from H, F, Cl, Br, I, OH, =O, NH₂, CN, COOH, CF₃, a C₁₋₄ alkyl, a C₁₋₄ alkoxy, or a C₃₋₆ cycloalkyl;
optionally, R₆ and L together form 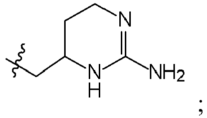
R₇ and R₈ are each independently selected from H, =O, halogen, NH₂, CN, a C₁₋₆ linear alkyl, a C₃₋₆ branched alkyl, a C₃₋₆ cycloalkyl, or

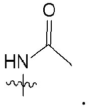

2. The compound or pharmaceutically acceptable form thereof according to claim 1, wherein
R₁ and R₂ are each independently selected from CH₃, CHF₂, CD₃ or a C₃₋₆ cycloalkyl;
preferably, R₁ and R₂ are each CD₃;
preferably, one of R₁ and R₂ is CH₃, and the other is CHF₂.

3. The compound or pharmaceutically acceptable form thereof according to claim 1 or 2, wherein
R₃, R₄, and R₅ are each independently selected from CH₃, i-Pr, OMe, CD₃, or halogen;
preferably, R₅ is CH₃ which is optionally further substituted with 0 to 3 deuterium; and R₃ and R₄ are the same and are selected from CH₃, i-Pr, OMe, or halogen.

4. The compound or pharmaceutically acceptable form thereof according to any one of claims 1 to 3, wherein L is selected from:
L is 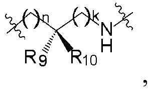 wherein n is 0 or 1, preferably 0; preferably, k is 0, 1, or 2; preferably, one of R₉ and R₁₀ is H, and the other is selected from H, a C₁₋₆ linear alkyl, a C₃₋₆ branched alkyl, a C₃₋₆ cycloalkyl, a C₃₋₆ heterocycloalkyl, a C₆₋₁₀ aryl, a C₅₋₁₀ heteroaryl, or COOCH₃; wherein the heteroaryl contains 1 or 2 N as heteroatom; and the H in L is optionally further substituted by 0 to 4 substituents selected from F, Cl, Br, I, OH, =O, NH₂, CN, COOH, a C₁₋₄ alkyl, or a C₁₋₄ alkoxy;
L is 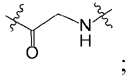
L is 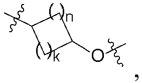 n is 0 or 1, and k is 1;
L is 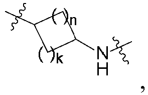 n is 0 or 1, and k is 1;
L is 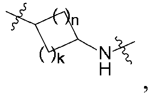 n is 2, and k is 2;
L is 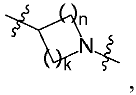 n is 1, and k is 1 or 2; or
L is 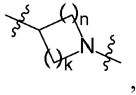 n is 2, and k is 2.

5. The compound or pharmaceutically acceptable form thereof according to any one of claims 1 to 4, wherein R₆ is selected from:
R₆ is 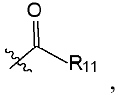 wherein R₁₁ is amino which is optionally further substituted with 0, 1, or 2 substituents selected from F, Cl, Br, I, OH, =O, NH₂, CN, COOH, CF₃, a C₁₋₄ alkyl, a C₁₋₄ alkoxy, or a C₃₋₆ cycloalkyl; or
R₆ is 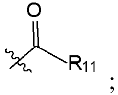 wherein R₁₁ is a C₆₋₁₀ aryl or a C₅₋₁₀ heterocyclyl; wherein the heterocyclyl contains 1, 2, or 3 N as heteroatoms; each of the aryl and heterocyclyl is optionally further substituted with 0, 1, 2, or 3 substituents selected from H, F, Cl, Br, I, OH, =O, NH₂, CN, COOH, CF₃, a C₁₋₄ alkyl, a C₁₋₄ alkoxy, or a C₃₋₆ cycloalkyl.

6. The compound or pharmaceutically acceptable form thereof according to any one of claims 1 to 5, wherein R₇ is H or CH₃, and R₈ is H or F.

7. The compound or pharmaceutically acceptable form thereof according to claim 1, wherein
R₁ and R₂ are each independently selected from CH₃ or CD₃;
R₃, R₄, and R₅ are each CH₃;
L is 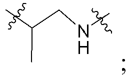
R₆ is 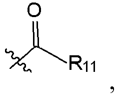 wherein R₁₁ is amino, methoxy, 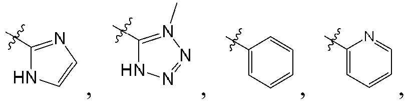 or 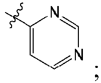 wherein the amino is optionally further substituted with 0 or 1 methyl or cyclopropyl; and the 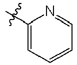 is optionally further substituted with 0 or 1 methyl or NH₂; and
R₇ and R₈ are each H.

8. The compound or pharmaceutically acceptable form thereof according to claim 1, wherein
R₁ and R₂ are each CH₃;
R₃, R₄, and R₅ are each CH₃;
L is 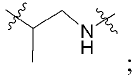
R₆ is 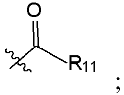 wherein R₁₁ is amino, 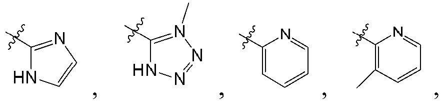 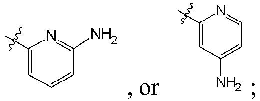
R₇ and R₈ are each H.

9. The compound or pharmaceutically acceptable form thereof according to claim 1, wherein the compound or pharmaceutically acceptable form thereof is one or more selected from the compounds set forth in Table 1.

10. An intermediate compound of Formula II: 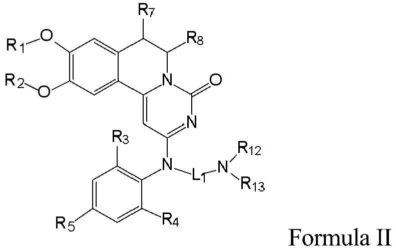
wherein R₁, R₂, R₃, R₄, R₅, R₇, and R₅ are as defined in any one of claims 1 to 9;
L₁ is selected from 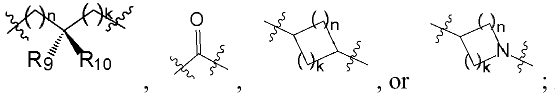 R₉, R₁₀, n, and k are as defined in any one of claims 1 to 9; and R₁₂ and R₁₃ are each independently H, Boc, Cbz, SEM, Fmoc, Alloc, Pht, OTs, PMB, Bn, or Trt;
preferably, the intermediate compound is 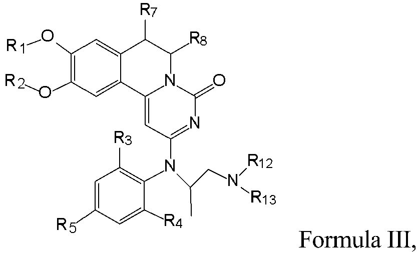
wherein one of R₁₂ and R₁₃ is H, and the other is Boc, Cbz, SEM, Fmoc, Alloc, Pht, OTs, PMB, Bn, or Trt.

11. A method for preparing the compound or pharmaceutically acceptable form thereof according to any one of claims 1 to 9, comprising:
subjecting the terminal groups R₁₂ and R₁₃ of the intermediate compound of Formula II according to claim 10 to a modification reaction, thereby obtaining the compound of Formula I.

12. The method according to claim 11, the method further comprises a step of preparing the intermediate compound according to claim 10.

13. A pharmaceutical composition, comprising: the compound or pharmaceutically acceptable form thereof according to any one of claims 1 to 9; and a pharmaceutically acceptable carrier, excipient, and/or one or more additional therapeutic agents.

14. Use of the compound or pharmaceutically acceptable form thereof according to any one of claims 1 to 9, or the pharmaceutical composition according to claim 13, in the manufacture of a preparation for inhibiting a phosphodiesterase.

15. Use of the compound or pharmaceutically acceptable form thereof according to any one of claims 1 to 9, or the pharmaceutical composition according to claim 13, in the manufacture of a medicament for treating a phosphodiesterase-related disease;
preferably, wherein the phosphodiesterase-related disease comprises a respiratory disease, such as asthma.
